# DEMANDE DE BREVET EUROPEEN

(11) **EP 1 097 921 A1**
(43) Date de publication de la demande: **09.05.2001**
(21) Numéro de dépôt: 01102661.4
(22) Date de dépôt: 11.04.1996
(51) Int. Cl.: C07C 243/22, C07C 255/66, C07C 309/45, C07C 309/47, C07C 311/21

(54) **Phenylhydrazines**

(30) Priorité: 11.04.1995 FR 9504350
(62) Demande divisionnaire de: 96913568.0
(71) Demandeur: SANOFI-SYNTHELABO, 75013 Paris (FR)
(72) Inventeur: Labeeuw, Bernard, 34080 Montpellier (FR); Gully, Danielle, 31600 Saubens (FR); Jeanjean, Francis, 34270 Valflaunes (FR); Molimard, Jean-Charles, 34980 St Gely Du Fesc (FR); Boigegrain, Robert, 34820 Assas (FR)
(74) Mandataire: Gillard, Marie-Louise

(57) **Abrégé**

L'invention a pour objet les phénylhydrazines de formule : 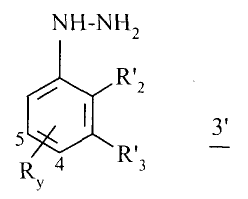 dans laquelle :
- R'₂ et R'₃ représentent chacun indépendamment un hydrogène, un (C₁-C₆)alkyle, un (C₃-C₈)cycloalkyle, un (C₃-C₈)cycloalkylméthyle ;
- ou R'₂ et R'₃ ensemble constituent un groupe triméthylène , tétraméthylène ou pentaméthylène ,
- R_{y} est en position 4 ou en position 5 et représente un groupe choisi parmi : cyano, carboxy, (C₁-C₄)alcoxycarbonyle, benzyloxycarbonyle, sulfo, (C₁-C₄)alkyl sulfonylamino, (C₁-C₄)alkylphénylsulfonylamino, carbamoyle, (C₁-C₄)alkyl carboxamido ;
- à la condition que R'₂ et R'₃ ne représente pas simultanément l'hydrogène et à la condition que R'₂ soit autre que méthyle lorsque R_{y} est le groupe sulfo ;
   et ses sels.
   Ces composés sont utiles notamment pour l'obtention de 1-phénylpyrazole-3-carboxamides substitués ayant une grande affinité pour les récepteurs humains de la neurotensine.

## Description

La présente invention concerne des nouveaux 1-phénylpyrazole-3-carboxamides substitués ayant une grande affinité pour les récepteurs humains de la neurotensine, un procédé pour leur préparation et des compositions pharmaceutiques les contenant en tant que principes actifs.

Les premiers médicaments potentiels de synthèse, non peptidiques, capables de se lier aux récepteurs de la neurotensine ont été décrits dans EP-0477049. Il s'agit d'amides de l'acide pyrazole-3-carboxylique, diversement substitués avec des acides aminés, qui déplacent la neurotensine iodée de son récepteur, à des doses inférieures à la micromole, sur des membranes de cerveau de cobaye. Cette série a conduit à la mise au point d'un composé, l'acide 2-[(1-(7-chloro-4-quinolyl)-5-(2,6-diméthoxyphényl)pyrazol-3-yl)carbonylamino]-adamantane-2-carboxylique, SR 48692, doté d'une activité antagoniste de la neurotensine puissante et sélective (D. Gully et al., Proc. Natl. Acad. Sci. USA, 1993, 90, 65-69).

La caractéristique de la série de produits décrite dans EP-0477049 est la présence, en position 1 du cycle pyrazole, notamment d'un groupe phényle, naphtyle, 4-quinolyle, substitués ou non substitués. Plus particulièrement, le SR 48692 possède, dans la position 1 du pyrazole, un groupe 7-chloro-4-quinolyle. Les produits décrits dans ce document ayant un groupe 1-naphtyle ou 4-chloro-1-naphtyle dans la position 1 du cycle pyrazole ont une affinité extrêmement élevée pour le récepteur de la neurotensine du cobaye car leur IC₅₀ est de l'ordre de 1 à 10 nanomoles, alors que leur affinité pour le récepteur humain est inférieure car leur IC₅₀ est de 10 à 100 nmoles.

Il a été maintenant trouvé qu'en substituant le groupe phényle des composés 1-phénylpyrazole-3-carboxamides avec des groupes particuliers, on augmente l'affinité pour les récepteurs de la neurotensine et plus particulièrement, on augmente l'affinité pour les récepteurs humains de la neurotensine.

De plus, les composés de la présente invention montrent *in vitro* un spectre d'activité plus large que les composés décrits dans EP 477049 en tant qu'antagonistes des récepteurs de la neurotensine.

Ainsi, la présente invention concerne, selon l'un de ses aspects, des nouveaux 1-phénylpyrazole-3-carboxamides substitués de formule : 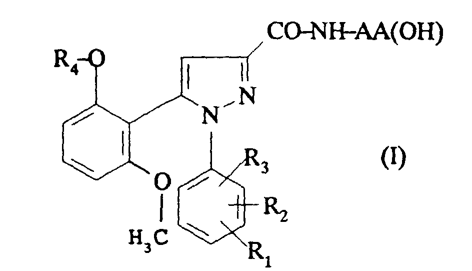 dans laquelle :
- R₁ représente un groupe choisi parmi :
   - T-CN;
   - C(NH₂) = NOH ;
   - C(= NOH)NH(CH₂)ᵣNR₅R₆ ;
   - T-C(NR₁₂R₁₃)= NR₁₄ ;
   - C(NH₂) = NO(CH₂)ᵣNR₅R₆ ;
   - T-CONRₐR_{b} ;
   - T-CONR₇R_{c} ;
   - Y-CO₂R₇ ;
   - OR_{d} ;
   - T-NR₅R₆, à la condition que R₅ et R₆ ne représentent pas simultanément l'hydrogène lorsque T représente une liaison directe ;
   - T-N(R₇)CORₑ ;
   - SO₂NRₐR_{b} ;
   - T-N(R₇)SO₂R'₇ ;
   - T-NR₂₇R₂₈;
- NRₐR_{b} représente un groupe choisi parmi :

   -NR₅R₆ ; -NR₉(CH₂)ₛCR₇R₈(CH₂)ₜNR₅R₆ ;

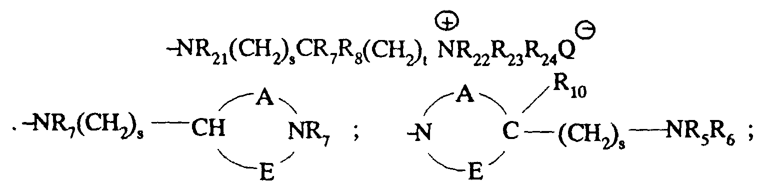

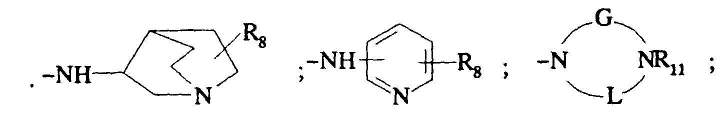

   -NR₇(CH₂)_{q}CN ; -NR₇(CH₂)_{q}C(NR₁₂R₁₃) = NR₁₄ ;

   -NR₇(CH₂)_{q}CONH₂ ; -NR₇(CH₂)_{q}CO₂R₇ ;

   -NR₂₁(CH₂)ₛCR₇R₈(CH₂)ₜNR₂₅R₂₆ ;
- R_{c} représente un groupe choisi parmi :

   -X-OR₇ ; -CHR₂₀CO₂R₇ ; -(CH₂)₄CH(NH₂)CO₂R₇ ;
- R_{d} représente un groupe choisi parmi :

   -X-NR₅R₆ ; -Y-CONR₅R₆ ; -Y-CO₂R₇ ; -Y-SO₂NR₅R₆ ; 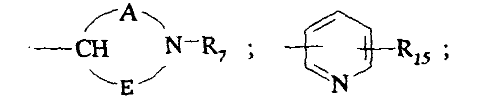
- Rₑ représente un groupe choisi parmi : 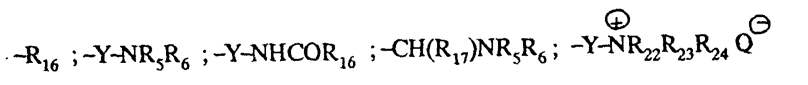 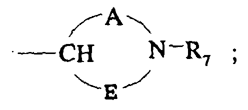 -(CH₂)_{q}CN ;-(CH₂)_{q}C(NR₁₂R₁₃) = NR₁₄ ;-NR₁₈R₁₉;
- R₂ et R₃ représentent chacun indépendamment l'hydrogène, un (C₁-C₆)alkyle, un (C₃-C₈)cycloalkylméthyle, un (C₃-C₈)cycloalkyle, un halogène, un nitro, un trifluorométhyle, un groupe -OR₄, un groupe -NR₅R₆, un pyrrol-1-yle, un cyano, un carbamoyle ;
- ou R₂ et R₃ ensemble constituent un groupe triméthylène, tétraméthylène ou pentaméthylène ;
- R₄ représente l'hydrogène ; un (C₁-C₆)alkyle ; un (C₃-C₄)alcényle ; un (C₃-C₈) cycloalkyle ; un (C₃-C₈)cycloalkylméthyle; un (C₁-C₄)alcoxy (C₁-C₄)alkylène ; un benzyle ;
- R₅ et R₆ représentent chacun indépendamment un hydrogène, un (C₁-C₆)alkyle; un (C₃-C₈)alcényle ; un (C₃-C₈)cycloalkylméthyle; un benzyle ; ou R₅ et R₆ ensemble avec l'atome d'azote auquel ils sont liés représentent un hétérocycle choisi parmi : la pyrrolidine, la pipéridine, la morpholine, la thiomorpholine, la pipérazine substituée en position 4 par R₉, l'aziridine, l'azétidine ou la perhydroazépine ;
- R'₅ et R'₆ représentent chacun indépendamment un hydrogène ou un (C₁-C₆)alkyle; ou bien R'₅ et R'₆ ensemble avec l'atome d'azote auquel ils sont liés représentent un hétérocycle choisi parmi : la pyrrolidine, la pipéridine, la morpholine, la thiomorpholine ou la pipérazine non substituée ou substituée en position 4 par un (C₁-C₆)alkyle ;
- R'₇ représente un (C₁-C₄)alkyle ; un phényle non substitué ou substitué une ou plusieurs fois par un (C₁-C₄)alkyle ; un groupe -X-NR₅R₆ ;
- R₇ représente un hydrogène, un (C₁-C₄)alkyle ou un benzyle ;
- R₈ représente un hydrogène, un (C₁-C₄)alkyle, un hydroxy, ou R₇ et R₈ ensemble avec l'atome de carbone auquel ils sont liés constituent un (C₃-C₅)cycloalcane ;
- R₉ représente l'hydrogène, un (C₁-C₄)alkyle, un benzyle, un groupe -X-OH ou un groupe -X-NR'₅R'₆, un (C₃-C₈)alcényle ;
- R₁₀ représente un hydrogène, un (C₁-C₄)alkyle, un benzyle, un carbamoyle, un cyano ;
- R₁₁ représente un hydrogène, un (C₁-C₄)alkyle, un groupe-X-OH, un groupe -X-NR'₅R'₆;
- R₁₂ et R₁₃ représentent chacun indépendamment un hydrogène ou un (C₁-C₄)alkyle ;
- R₁₄ représente l'hydrogène, R₁₄ peut de plus représenter un (C₁-C₄)alkyle lorsque R₁₂ représente l'hydrogène et R₁₃ représente un (C₁-C₄)alkyle;
- ou R₁₃ et R₁₄ ensemble représentent un groupe Z ;
- R₁₅ représente l'hydrogène, un (C₁-C₄)alkyle, un groupe -(CH₂)ₛNR₅R₆;
- R₁₆ représente l'hydrogène, un (C₁-C₈)alkyle, un (C₃-C₈)cycloalkyle, un phényle, un pipérid-2-yle , un pipérid-3-yle, un pipérid-4-yle ;
- R₁₇ représente un (C₁-C₆)alkyle, un phényle, un benzyle, un hydroxy -(C₁-C₄)alkyle, un amino(C₁-C₄)alkyle;
- R₁₈ et R₁₉ représentent chacun indépendamment un hydrogène, un (C₁-C₄)alkyle; R₁₈ peut de plus représenter un groupe -(CH₂)_{q}-NR₅R₆;
- ou R₁₈ et R₁₉ ensemble avec l'atome d'azote auquel ils sont liés représentent un hétérocycle choisi parmi : la pyrrolidine, la pipéridine, la morpholine, la thiomorpholine, la pipérazine substituée en position 4 par R₉ ;
- R₂₀ représente l'hydrogène, un (C₁-C₄)alkyle, un benzyle, un hydroxy phénylméthyle, de préférence un 4-hydroxy phénylméthyle, un hydroxy(C₁-C₄)alkyle, un mercapto(C₁-C₄)alkyle; un groupe -(CH₂)₃-NH-C(= NH)NH₂, un groupe -(CH₂)₄NH₂, un groupe -CH₂-Im dans lequel Im représente un imidazol-4-yl ;
- R₂₁ représente un (C₁-C₄)alkyle, un allyle ou un benzyle ;
- R₂₂ et R₂₃ représentent chacun indépendamment un (C₁-C₆)alkyle ; ou bien R₂₂ et R₂₃ ensemble avec l'atome d'azote auquel ils sont liés représentent un hétérocycle choisi parmi : la pyrrolidine, la pipéridine, la morpholine ou la perhydroazépine.
- R₂₄ représente un (C₁-C₄)alkyle, un benzyle, un allyle, un hydroxy(C₁-C₄)alkyle, un (C₁-C₄)alcoxy(C₁-C₄)alkyle ;
- Q^{⊖} représente un anion ;
- R₂₅ représente l'hydrogène ou un (C₁-C₆)alkyle;
- R₂₆ représente un (C₁-C₄)alcoxycarbonyle, un benzyloxycarbonyle ; un (C₁-C₄)alkylcarbonyle ;
- R₂₇ représente un hydrogène ; un (C₁-C₄)alkyle ; un (C₁-C₄)alkylcarbonyle; un groupe -CO-(CH₂)ᵣ-OH ; un groupe -SO₂R'₇ ;
- R₂₈ représente un groupe -X-NR₅R₆ ;
- s = 0 à 3 ;
- t = 0 à 3, à la condition que (s + t) dans un même groupe soit supérieur ou égal à 1 ;
- r = 2 à 5 ;
- q = 1 à 5 ;
- T représente une liaison directe ou (C₁-C₇)alkylène;
- X représente un (C₂-C₇)alkylène ;
- Y représente un (C₁-C₇)alkylène;
- Z représente un (C₂-C₆)alkylène;
- les radicaux divalents A et E ensemble avec l'atome de carbone et l'atome d'azote auxquels ils sont liés constituent un hétérocycle saturé ayant 4 à 7 chaînons et pouvant en outre être substitué par un ou plusieurs (C₁-C₄)alkyles;
- les radicaux divalents G et L ensemble avec les atomes d'azote auxquels ils sont liés constituent un cycle pipérazine ou imidazolidine ou imidazoline, lesdits cycles étant éventuellement substitués sur les atomes de carbone par un ou plusieurs (C₁-C₄)alkyles;
- le groupe -NH-AA(OH) représente le résidu d'un acide aminé : 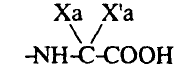
où Xₐ est l'hydrogène et X'ₐ est l'hydrogène, un (C₁-C₅)alkyle ou un radical carbocyclique non aromatique en C₃-C₁₅ ; ou bien Xₐ et X'ₐ, ensemble avec l'atome de carbone auquel ils sont liés, forment un carbocycle non aromatique en C₃-C₁₅; leurs sels, leurs sels d'ammonium quaternaire formés sur les amines tertiaires, acycliques ou cycliques et leurs solvates.

Lorsqu'un composé selon l'invention comprend un ou plusieurs atomes de carbone asymétriques chacun des isomères optiques fait partie de l'invention, ainsi que la forme racémique.

Lorsque un composé selon l'invention présente plusieurs formes tautomères, chacune d'elle fait partie de l'invention. C'est le cas notamment lorsque le substituant R₁ comporte un groupement amidine substitué -C(NR₁₂R₁₃) = NR₁₄.

Lorsque le groupe -NH(AA)OH représente le résidu d'un aminoacide cycloaliphatique, les groupes amino ou aminométhyle peuvent être en position endo ou en position exo par rapport au système cyclique ; dans les deux cas, les composés de formule (I) font partie de l'invention.

Selon la présente invention par alkyle ou alkylène, on entend un alkyle ou un alkylène droit ou ramifié, qualifié par le nombre d'atomes de carbone qu'il comporte ; par halogène on entend un atome de chlore, de brome, de fluor ou d'iode.

Les radicaux carbocycliques non aromatiques en C₃-C₁₅ comprennent les radicaux mono ou polycycliques, condensés ou pontés, saturés ou insaturés, éventuellement terpéniques. Ces radicaux sont éventuellement mono- ou polysubstitués par un alkyle en C₁-C₄.

Les radicaux monocycliques incluent les cycloalkyles par exemple cyclopropyle, cyclopentyle, cyclohexyle, cycloheptyle, cyclooctyle, cyclododécyle.

Dans le résidu de l'acide aminé ci-dessus lorsque Xₐ et X'ₐ ensemble avec l'atome de carbone auquel ils sont liés forment un carbocycle non aromatique en C₃-C₁₅, ledit carbocycle est tel que défini pour les radicaux correspondants ci-dessus.

Parmi les carbocycles non aromatiques polycycliques, l'adamantane, le bicyclo[3.3.1]nonane et le norbornane sont les préférés. Le radical correspondant à l'adamantane peut être le 1-adamantyle lorsque Xₐ est l'hydrogène ou le 2-adamantylidène lorsque Xₐ et X'ₐ ensemble avec l'atome de carbone auquel ils sont liés forment un carbocycle.

Parmi les carbocycles non aromatiques monocycliques, le cyclopentane et le cyclohexane sont particulièrement préférés.

Les sels des composés de l'invention peuvent être les sels internes ou bien des sels avec des métaux alcalins, de préférence le sodium ou le potassium, des métaux alcalino-terreux, de préférence le calcium et avec des bases organiques comme la diéthylamine, la trométhamine, la méglumine (N-méthyl-D-glucamine), la lysine, l'arginine, l'histidine, la choline ou la diéthanolamine ou des bases organiques optiquement pures telles que l'α-méthylbenzylamine.

Les sels des composés de formule (I) selon la présente invention comprennent également ceux avec des acides minéraux ou organiques qui permettent une séparation ou une cristallisation convenable des composés de formule I, tels que l'acide picrique, l'acide oxalique ou un acide optiquement actif, par exemple un acide mandélique ou un acide camphosulfonique, et de préférence ceux qui forment des sels pharmaceutiquement acceptables tels que le chlorhydrate, l'acétate, l'hydrogénosulfate, le dihydrogénophosphate, le méthanesulfonate, le maléate, le fumarate, le 2-naphtalènesulfonate, l'iséthionate, le benzènesulfonate, le paratoluènesulfonate, le tartrate, le citrate, l'édisylate.

Les sels d'ammonium quaternaires sur les amines tertiaires acycliques ou cycliques sont formés par substitution de l'amine avec un (C₁-C₄)alkyle, un benzyle, un allyle, un hydroxy(C₁-C₄)alkyle, un (C₁-C₄)alcoxy(C₁-C₄)alkylène; l'anion étant préférentiellement un anion pharmaceutiquement acceptable.

Avantageusement, l'invention concerne des composés de formule (Ip) : 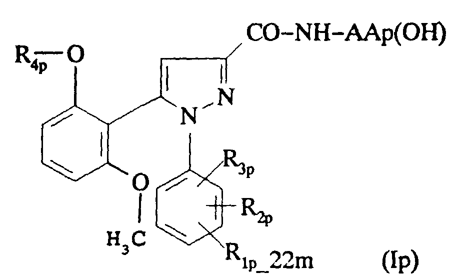 dans laquelle :
- R₁ₚ représente un groupe choisi parmi:
   - T-CN;
   - C(NH₂) = NOH ;
   - C(=NOH)NH(CH₂)ᵣNR₅R₆ ;
   - T-C(NR₁₂R₁₃)= NR₁₄ ;
   - C(NH₂) = NO(CH₂)ᵣNR₅R₆ ;
   - T-CONRₐR_{b} ;
   - T-CONR₇R_{c} ;
   - Y-CO₂R₇ ;
   - OR_{d};
   - T-NR₅R₆, à la condition que R₅ et R₆ ne représentent pas simultanément l'hydrogène lorsque T représente une liaison directe ;
   - T-N(R₇)CORₑ ;
   - SO₂NRₐR_{b} ;
   - T-N(R₇)SO₂R'₇ ;
- NRₐR_{b} représente un groupe choisi parmi :

   -NR₅R₆ ; -NR₉(CH₂)ₛCR₇R₈(CH₂)ₜNR₅R₆ ;

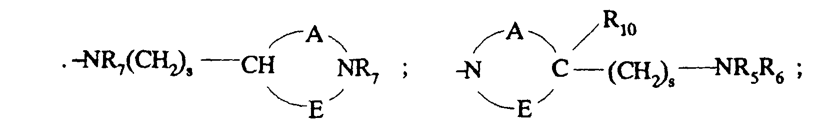

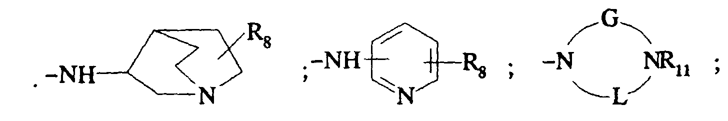

   -NR₇(CH₂)_{q}CN ; -NR₇(CH₂)_{q}C(NR₁₂R₁₃) = NR₁₄ ;
- R_{c} représente un groupe choisi parmi :

   -X-OR₇ ; -CHR₂₀CO₂R₇ ; -(CH₂)₄CH(NH₂)CO₂R₇ ;
- R_{d} représente un groupe choisi parmi :

   -X-NR₅R₆ ; -Y-CONR₅R₆ ; -Y-CO₂R₇ ; -Y-SO₂NR₅R₆ ; 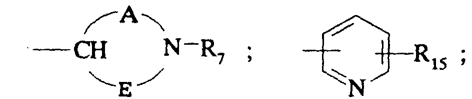
- Rₑ représente un groupe choisi parmi:

   -R₁₆ ; -Y-NR₅R₆ ; -Y-NHCOR₁₆ ; -CH(R₁₇)NR₅R₆ ;

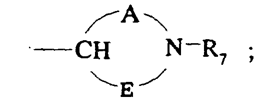

   -(CH₂)_{q}CN ;-(CH₂)_{q}C(NR₁₂R₁₃) = NR₁₄ ;-NR₁₈R₁₉;
- R₂ₚ et R₃ₚ représentent chacun indépendamment l'hydrogène, un (C₁-C₆)alkyle, un (C₃-C₈)cycloalkylméthyle, un (C₃-C₈)cycloalkyle, un halogène, un nitro, un trifluorométhyle, un groupe -OR₄, un groupe -NR₅R₆, un pyrrol-1-yle, un cyano, un carbamoyle ;
- ou R₂ₚ et R₃ₚ ensemble constituent un groupe triméthylène, tétraméthylène ou pentaméthylène ;
- R₄ₚ représente l'hydrogène ; un (C₁-C₆)alkyle ; un (C₃-C₄)alcényle ; un (C₃-C₈) cycloalkyle ; un (C₃-C₈)cycloalkylméthyle; un (C₁-C₄)alcoxy (C₁-C₄)alkyle ; un benzyle ;
- R₅ et R₆ représentent chacun indépendamment un hydrogène, un (C₁-C₆)alkyle ; ou R₅ et R₆ ensemble avec l'atome d'azote auquel ils sont liés représentent un hétérocycle choisi parmi : la pyrrolidine, la pipéridine, la morpholine, la thiomorpholine, la pipérazine substituée en position 4 par R₉ ;
- R'₇ représente un (C₁-C₄)alkyle;
- R₇ représente un hydrogène, un (C₁-C₄)alkyle ou un benzyle ;
- R₈ représente un hydrogène, un (C₁-C₄)alkyle, un hydroxy, ou R₇ et R₈ ensemble avec l'atome de carbone auquel ils sont liés constituent un (C₃-C₅)cycloalcane ;
- R₉ représente l'hydrogène, un méthyle, un groupe -X-OH ou un groupe -X-NR₅R₆ ;
- R₁₀ représente un hydrogène, un (C₁-C₄)alkyle, un benzyle, un carbamoyle, un cyano;
- R₁₁ représente un hydrogène, un (C₁-C₄)alkyle, un groupe-X-OH, un groupe -X-NR₅R₆ ;
- R₁₂ et R₁₃ représentent chacun indépendamment un hydrogène ou un (C₁-C₄)alkyle ;
- R₁₄ représente l'hydrogène, R₁₄ peut de plus représenter un (C₁-C₄)alkyle lorsque R₁₂ représente l'hydrogène et R₁₃ représente un (C₁-C₄)alkyle;
- ou R₁₃ et R₁₄ ensemble représentent un groupe Z ;
- R₁₅ représente l'hydrogène, un (C₁-C₄)alkyle, un groupe -(CH₂)ₛNR₅R₆ ;
- R₁₆ représente l'hydrogène, un (C₁-C₈)alkyle, un (C₃-C₈)cycloalkyle, un phényle, un pipérid-2-yle , un pipérid-3-yle, un pipérid-4-yle ;
- R₁₇ représente un (C₁-C₆)alkyle, un phényle, un benzyle, un hydroxy -(C₁-C₄)alkyle, un amino(C₁-C₄)alkyle;
- R₁₈ et R₁₉ représentent chacun indépendamment un hydrogène, un (C₁-C₄)alkyle; R₁₈ peut de plus représenter un groupe -(CH₂)_{q}-NR₅R₆ ;
- ou R₁₈ et R₁₉ ensemble avec l'atome d'azote auquel ils sont liés représentent un hétérocycle choisi parmi : la pyrrolidine, la pipéridine, la morpholine, la thiomorpholine, la pipérazine substituée en position 4 par R₉ ;
- R₂₀ représente l'hydrogène, un (C₁-C₄)alkyle, un benzyle, un hydroxy phénylméthyle, un hydroxy(C₁-C₄)alkyle, un mercapto(C₁-C₄)alkyle; un groupe -(CH₂)₃-NH-C(= NH)NH₂, un groupe -(CH₂)₄NH₂, un groupe -CH₂-Im dans lequel Im représente un imidazol-4-yl ;
- s = 0 à 3 ;
- t = 0 à 3, à la condition que (s + t) soit supérieur ou égal à 1 ;
- r = 2 à 5 ;
- q = 1 à 5 ;
- T représente une liaison directe ou (C₁-C₇)alkylène;
- X représente un (C₂-C₇)alkylène ;
- Y représente un (C₁-C₇)alkylène;
- Z représente un (C₂-C₆)alkylène ;
- les radicaux divalents A et E ensemble avec l'atome de carbone et l'atome d'azote auxquels ils sont liés constituent un hétérocycle saturé ayant 5 à 7 chaînons et pouvant en outre être substitué par un ou plusieurs (C₁-C₄)alkyles;
- les radicaux divalents G et L ensemble avec les atomes d'azote auxquels ils sont liés constituent un cycle pipérazine ou imidazolidine ou imidazoline, lesdits cycles étant éventuellement substitués sur les atomes de carbone par un ou plusieurs (C₁-C₄)alkyles ;
- le groupe -NH-AAₚ(OH) représente le résidu d'un acide aminé : 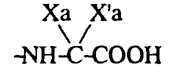
où Xₐ est l'hydrogène et X'ₐ est l'hydrogène, un (C₁-C₅)alkyle ou un radical carbocyclique non aromatique en C₃-C₁₅ ; ou bien Xₐ et X'ₐ, ensemble avec l'atome de carbone auquel ils sont liés, forment un carbocycle non aromatique en C₃-C₁₅; et leurs sels.

Des composés préférés selon l'invention répondent à la formule : 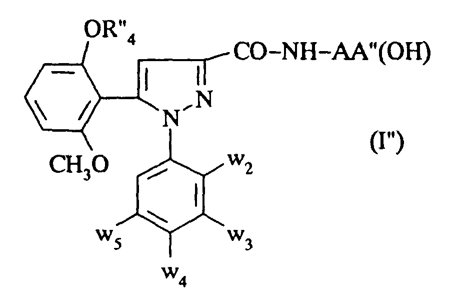 dans laquelle :
- R"₄ représente l'hydrogène, un méthyle ou un cyclopropylméthyle ;
- AA"(OH) représente un groupe 2-carboxyadamantan-2-yle, α-carboxycyclohexylméthyle ou 9-carboxybicyclo[3.3.1]nonan-9-yle ;
parmi les substituants w₂, w₃, w₄ et w₅, au moins un est l'hydrogène et au moins un autre est autre que l'hydrogène, de telle sorte que :
soit
(i)
   . w₅ est hydrogène ;
   . w₃ est hydrogène ou méthyle ;
   . w₂ est (C₁-C₄)alkyle, (C₃-C₆)cycloalkyle, (C₁-C₄)alcoxy, chlore ou trifluorométhyle, ou w₂ et w₃ ensemble forment un groupe 1,4-butylène ;
   . w₄ est choisi soit parmi les groupes suivants :
(i1) dialkylaminoalkylaminocarbonyle 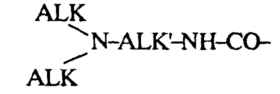
(i2) dialkylaminoalkyl(N-méthyl)aminocarbonyle 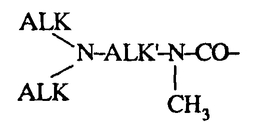
(i3) dialkylaminoalkyl(N-éthyl)aminocarbonyle 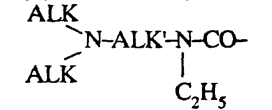
(i4) cyanoalkyl(N-méthyl)aminocarbonvle 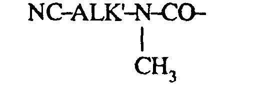
(i5) aminoalkylaminocarbonyle

   H₂N-ALK'-NH-CO-
(i6) aminoalkyl(N-méthyl)aminocarbonyle 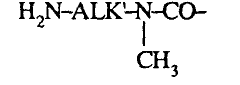
(i7) (N'-méthyl)-(N'-alcoxycarbonyl)aminoalkyl(N-méthyl)carbonyle 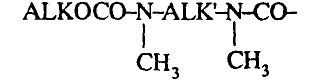
(i8) amidinoalkylaminocarbonyle 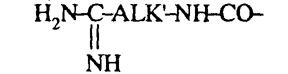
(i9) pyrrolidinoalkylaminocarbonyle 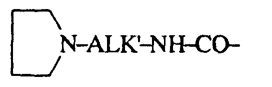
(i10) morpholinoalkylaminocarbonyle 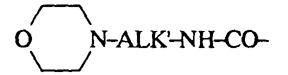
(i11) alkylaminoalkyl(N-méthyl)aminocarbonyle 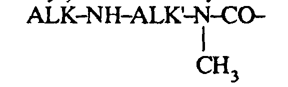
(i12) 2-(1H)-imidazolinylalkylaminocarbonyle 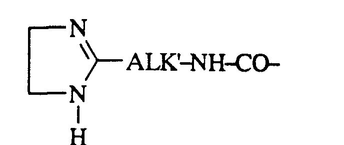
(i13) bis(dialkylaminoalkyl)aminocarbonyle

   (ALK₂N-ALK')₂N-CO-
(i14) aminocarbonylylkyl(N-méthyl)aminocarbonyle 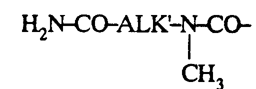
(i15) carboxyalkyl(N-méthyl)aminocarbonyle 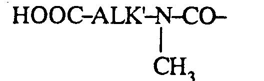
(i16) un groupe de structure 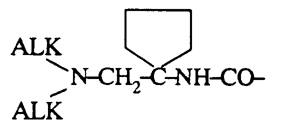
(i17) 2-pyridylaminocarbonyle 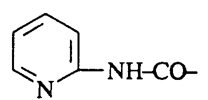
(i18) 1-benzylpipérid-4-ylaminocarbonyle 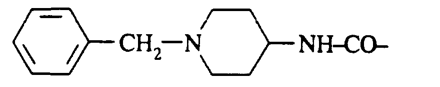
(i19) quinuclidin-3-ylaminocarbonyle 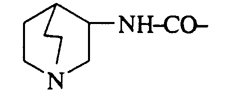
(i20) pipérid-4-ylaminocarbonyle 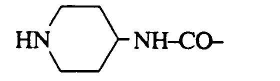
(i21) 2,2,6,6-tétraméthylpipérid-4-ylaminocarbonyle 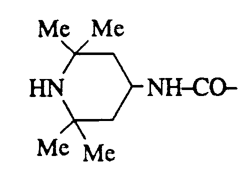
(i22) aminocarbonyle

   H₂N-CO-
(i23) 4-alkylpipérazinocarbonyle 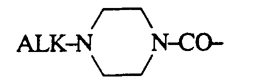
(i24) 4-dialkylaminopipéridinocarbonyle 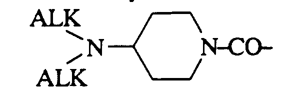
(i25) 3-dialkylaminopyrrolidinocarbonyle 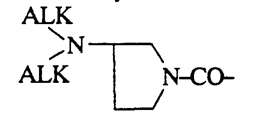
(i26) dialkylaminoalkyl(N-méthyl)aminosulfonyle 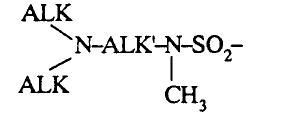
(i27) dialkylaminoalkyl(N-benzyl)aminosulfonyle 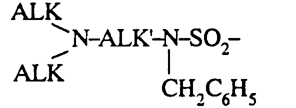
(i28) 1-alkylpyrrolidin-2-ylméthylaminocarbonyle 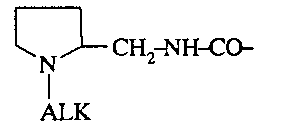
(i29) allylaminocarbonyle

   CH₂=CH-CH₂-NH-CO-
(i30) dialkylaminoalkyl(N-acétyl)amino 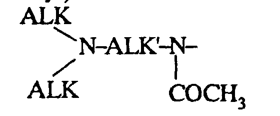
(i31) dialkylaminoalkylamino 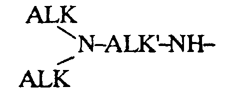
(i32) dialkylaminoalkylcarboxamido 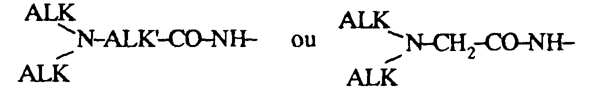 soit w₄ est choisi parmi les groupes suivants :
(i33) pipéridinoalkylcarboxamido 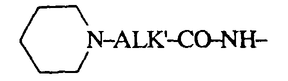
(i34) glycinamido

   H₂N-CH₂-CO-NH-
(i35) tosylamido 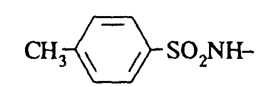
(i36) aminoalkylsulfonamido

   H₂N-ALK'-SO₂NH- ou H₂N-CH₂-SO₂NH-
(i37) sel de trialkylammonioalkyl(N-méthyl)aminocarbonyle 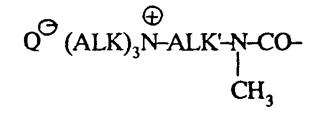
ALK étant (C₁-C₄)alkyle et ALK' étant (C₂-C₅)alkylène ;
soit
(ii)
   w₂ et w₅ sont l'hydrogène
   w₃ est chlore
   w₄ est cyano ou aminocarbonyle
   soit
(iii) w₂ et w₅ sont l'hydrogène, w₃ est isopropyle et w₄ est dialkvlaminoalkvlaminocarbonvle 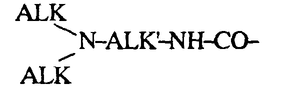 ALK et ALK' étant tels que définis ci-dessus ;
   soit
(iv) w₂ et w₅ sont l'hydrogène, w₃ est dialkylaminoalkyl(N-méthyl)aminocarbonyle 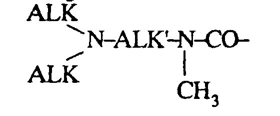 et w₄ est chloro ;
   ALK et ALK' étant tels que définis ci-dessus ;
   soit
(v)
   w₃ et w₄ sont l'hydrogène
   w₂ est chloro, (C₁-C₄)alcoxy ou (C₁-C₄)alkyle
   w₅ est
(v1) dialkylaminoalkyl(N-méthyl)aminocarbonyle 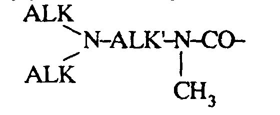
(v2) dialkylaminoalkylcarbonylamino 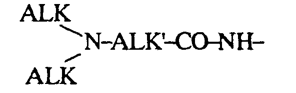 ALK et ALK' étant tels que définis ci-dessus ;
leurs sels internes et leurs sels pharamaceutiquement acceptables, leurs sels d'ammonium quaternaire et leurs solvates.

Un groupe de composés préférés selon l'invention est constitué par les composés de formule : 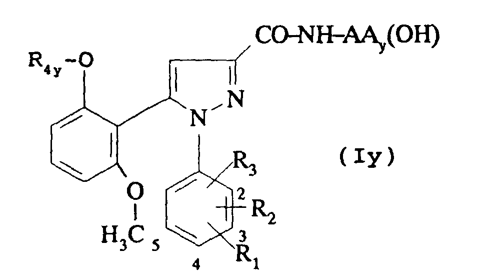 dans laquelle :
- R₁, R₂, R₃ sont tels que définis ci-dessus pour (I) ;
- R₄y représente l'hydrogène, un groupe (C₁-C₄)alkyle, un allyle ou un cyclopropylméthyle ; et
- le groupe -NH-AA_{y}-(OH) représente soit un résidu choisi parmi le résidu de l'acide 2-aminoadamantane-2- carboxylique, de l'acide (S) α-aminocyclohexaneacétique et de l'acide 9-aminobicyclo[3.3.1]nonane-9-carboxylique, soit le résidu de l'acide 2-aminonorbomane-2-carboxylique,
leurs sels et leurs sels d'ammonium quaternaire formés sur les amines tertiaires acycliques ou cycliques et leurs solvates.
Parmi les composés de formule (Iy) tels que définis ci-dessus, on préfère ceux dans lesquels :
- R₁, tel que défini pour (I), est en position 4 ou 5 ;
- R₂ est en position 2 et représente un groupe choisi parmi : l'hydrogène , un (C₁-C₆)alkyle , un (C₃-C₈)cycloalkyle , un (C₃-C₈)cycloalkylméthyle, un (C₁-C₆)alcoxy, un (C₃-C₈)cycloalkyloxy, un chlore, un trifluorométhyle ;
- R₃ est en position 3 et représente l'hydrogène, un (C₁-C₆)alkyle, un (C₃-C₈)cycloalkyle, un (C₃-C₈)cycloalkylméthyle ;
- ou R₂ et R₃ ensemble constituent un triméthylène, un tétraméthylène ou un pentaméthylène ;
leurs sels et leurs sels d'ammonium quaternaire formés sur les amines tertiaires acycliques ou cycliques et leurs solvates.

En particulier, on préfère les composés de formule (Iy'): 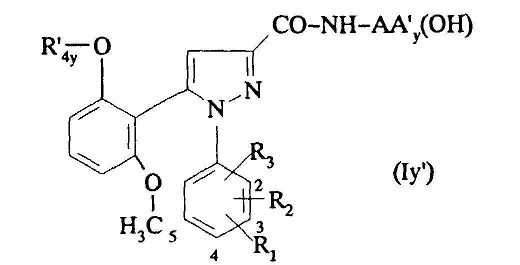 dans laquelle :
- R₁, R₂, R₃ représentent respectivement R₁ₚ, R₂ₚ et R₃ₚ tels que définis ci-dessus pour (Ip) ;
- R'_{4y} représente un groupe (C₁-C₄)alkyle ou cyclopropylmethyle ; et
- le groupe -NH-AA'y-(OH) représente le résidu de l'acide 2-aminoadamantane-2-carboxylique ou de l'acide (S) α-aminocyclohexaneacétique ou de l'acide 2-aminonorbornane-2-carboxylique.

Parmi les composés de formule (Iy') tels que définis ci-dessus, on préfère ceux dans lesquels :
- R₁ représente R₁ₚ tel que défini pour (I) et est en position 4 ou 5 ;
- R₂ est en position 2 et représente un groupe choisi parmi : l'hydrogène, un (C₁-C₆)alkyle, un (C₃-C₈)cycloalkyle, un (C₁-C₆)alcoxy, un chlore, un trifluorométhyle ;
- R₃ est en position 3 et représente l'hydrogène ou un (C₁-C₆)alkyle;
- ou R₂ et R₃ ensemble constituent un triméthylène, un tétraméthylène ou un pentaméthylène ;
et leurs sels.

De façon toute particulière, on préfère les composés de formule : 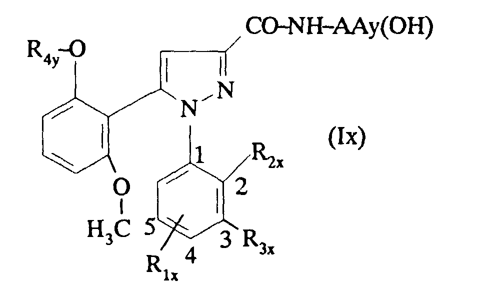 dans laquelle :
- R_{4y} et NH-AA_{y}(OH) sont tels que définis précédemment pour (Iy) ;
- R₁ₓ est en position 4 ou 5 et représente un groupe choisi parmi -T-CONRₐR_{b} ,
- SO₂NRₐR_{b}, -T-NR₅R₆, -N(R₇)CORₑ, -OR_{d}, -N(R₇)SO₂R'₇, -T-NR₂₇R₂₈ ; les groupes -T-, Rₐ, R_{b}, R_{d}, Rₑ, R₅, R₆, R₇, R'₇, R₂₇ et R₂₈ étant tels que définis ci-dessus pour (I) ;
- R₂ₓ et R₃ₓ représentent chacun indépendamment un hydrogène ; un (C₁-C₆)alkyle; un (C₃-C₈) cycloalkyle ; un (C₃-C₈)cycloalkylméthyle ;
   à la condition que R₂ₓ et R₃ₓ ne représentent pas simultanément l'hydrogène,
- ou R₂ₓ et R₃ₓ ensemble constituent un groupe tétraméthylène ;
   leurs sels et leurs sels d'ammonium quaternaire formés sur les amines tertiaires acycliques ou cycliques et leurs solvates.

Parmi les composés de formule (Ix) on préfère ceux de formule (Ix') : 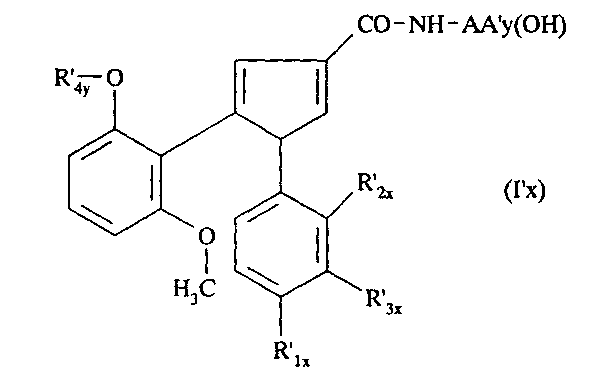 dans laquelle :
- R'_{4y} représente un groupe (C₁-C₄)alkyle ou cyclopropylmethyle et NH-AA'y(OH) est tel que défini précédemment pour (Iy') ;
- R'₁ₓ représente un groupe choixi parmi -T-CONRₐR_{b}, -SO₂NRₐR_{b}, -Y-NR₅R₆, -N(R₇)CORₑ, -OR_{d} ; les groupes -T-, NRₐR_{b}, R_{d}, Rₑ, R₅, R₆ et R₇ étant tels que définis ci-dessus pour (Ip) ;
- R'₂ₓ représente un (C₁-C₆)alkyle, un (C₃-C₈)cycloalkyle ;
- R'₃ₓ représente l'hydrogène ou un (C₁-C₆)alkyle;
- ou R'₂ₓ et R'₃ₓ ensemble constituent un groupe tétraméthylène ; et leurs sels.

Parmi les composés de formule (ly), un groupe préféré est constitué par les composés de formule : 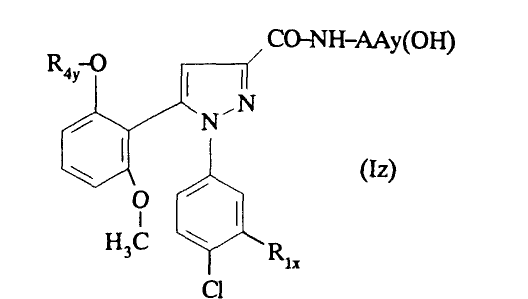 dans laquelle R_{4y}, R₁ₓ et NH-AA_{y}(OH) sont tels que définis ci-dessus, et de préférence représentent R'_{4y}, R₁ₚ et NH-AA'_{y}(OH) tels que définis pour (Iy') ; leurs sels, leurs sels d'ammonium quaternaire formés sur les amines tertiaires acycliques ou cycliques et leurs solvates.
Plus particulièrement on choisit les composés de formule : 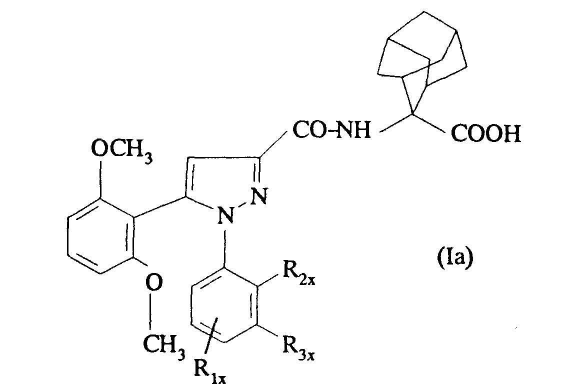 dans laquelle :
- R₁ₓ, R₂ₓ, R₃ₓ sont tels que définis ci-dessus pour (Ix) ; de préférence R'₁ₓ, R'₂ₓ et R'₃ₓ tels que définis pour (I'x), R'₁ₓ étant de préférence en position para,
leurs sels, leurs sels d'ammonium quaternaire formés sur les amines tertiaires acycliques ou cycliques et leurs solvates.

De façon préférentielle, l'invention se rapporte à l'acide 2-[5-(2,6-diméthoxyphényl)-1-[4-[N-méthyl-N-(3-diméthylaminopropyl)carbamoyl]-2-isopropylphényl]pyrazol-3-ylcarbonylamino]adamantane-2-carboxylique à son sel interne, à ses sels, de préférence pharmaceutiquement acceptables et à ses solvates.

Selon un autre de ses aspects, la présente invention concerne un procédé pour la préparation des 1-phénylpyrazole-3-carboxamides substitués de formule (I) et de leurs sels, caractérisé en ce que :
1) l'on traite un dérivé fonctionnel d'un acide 1-phénylpyrazole-3-carboxylique de formule : 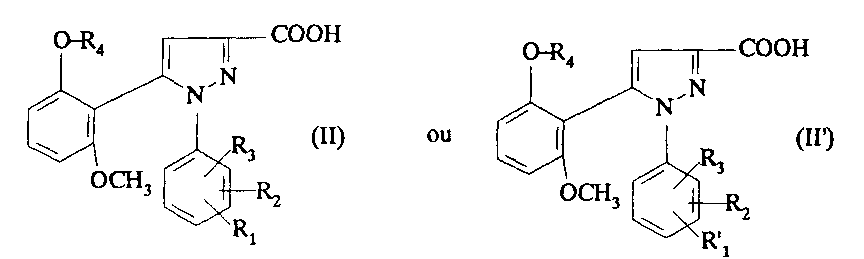 dans laquelle R₁, R₂, R₃, R₄ ont les significations données ci-dessus pour le composé de formule (I) et R'₁ représente un précurseur de R₁ choisi parmi les groupes nitro, amino, phtalimido, halogéno, hydroxy, sulfo, hydroxy (C₁-C₇) alkylène, cyano, carboxy, (C₁-C₄)alcoxycarbonyle, benzyloxycarbonyle, avec un acide aminé, éventuellement protégé par les groupements protecteurs habituels en synthèse peptidique, de formule :

   H-HN-AA(OH) (III)

   dans laquelle -NH-AA(OH) est tel que défini ci-dessus pour le composé de formule (I);
2) le cas échéant, on soumet le dérivé fonctionnel d'acide ainsi obtenu de formule: 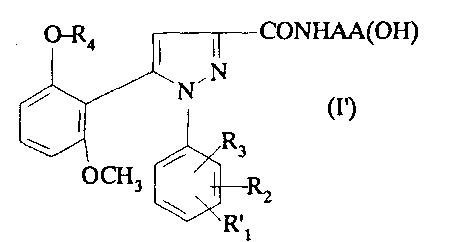 à un traitement ultérieur approprié pour transformer le substituant R'₁, précurseur de R₁, en le substituant R₁ ;
3) éventuellement, on déprotège le composé ainsi obtenu à l'étape 1) ou à l'étape 2) pour conduire à l'acide libre de formule (I) correspondant ;
4) le cas échéant, on prépare un sel du composé (I) ainsi obtenu ou son sel d'ammonium quaternaire.

Comme dérivé fonctionnel de l'acide 1-phénylpyrazole-3-carboxylique substitué de formule (II) ou (II'), on peut utiliser le chlorure d'acide, l'anhydride, un anhydride mixte, un ester alkylique en C₁-C₄, un ester activé, par exemple l'ester de p-nitrophényle, ou l'acide libre opportunément activé, par exemple, avec le N,N-dicyclohexylcarbodiimide ou avec l'hexafluorophosphate de benzotriazol-1-yloxytris-(diméthylamino)-phosphonium (BOP).

Les acides aminés de formule (III) peuvent être utilisés soit tels quels, soit après protection préalable du groupe carboxyle par des groupements protecteurs habituels en synthèse peptidique comme décrit par exemple dans Protective Groups in Organic Chemistry, Ed. J.F.W. McOmie, Plenum Press, 1973, page 183, ou dans Protective Groups in Organic Synthesis, II Ed. J.F.W. Greene et P.G.M. Wuts, John Wiley & Sons, 1991, page 224.

Pour cette protection, le groupe carboxylique de l'acide aminé (III) peut être tout simplement estérifié, par exemple sous forme d'ester méthylique, benzylique ou tert-butylique, le groupe estérifiant étant ensuite éliminé par hydrolyse acide ou basique ou par hydrogénolyse. La protection par estérification ne peut être utilisée que lorsque le groupe R₁ ou R'₁ ne contient pas, lui aussi, soit un groupe ester qui doit être conservé, comme au cas où, par exemple, R₁ représenterait un groupe O-Y-COOR₇ ou -Y-COOR₇ ou -T-CONR₇CHR₂₀COOR₇ ou -T-CONR₇(CH₂)₄CH(NH₂)CO₂R₇ avec R₇ = alkyle, soit, de toute façon, un groupe susceptible d'être affecté pendant le déblocage du groupe ester. La protection du groupe carboxylique de l'acide aminé (III) peut également être effectuée par silylation, par exemple avec le bis(triméthylsilyl)acétamide, ladite protection pouvant être effectuée *in situ*. L'ester silylique du composé (I) est ensuite facilement éliminé pendant l'isolement du produit final par simple acidification, hydrolyse ou échange avec un alcool.

Ainsi dans l'étape 1) du procédé on peut faire réagir le chlorure d'un acide 1-phénylpyrazole-3-carboxylique, obtenu par réaction du chlorure de thionyle sur un acide de formule (II) ou (II'), avec un acide aminé de formule (III), dans un solvant tel que l'acétonitrile, le THF, le DMF ou le DCM, sous une atmosphère inerte, à la température ambiante, pendant un temps compris entre quelques heures et quelques jours, en présence d'une base telle que la pyridine, la soude ou la triéthylamine.

Une variante de l'étape 1) consiste à préparer le chlorure d'acide ou l'anhydride mixte d'un acide 1-phénylpyrazole-3-carboxylique par réaction du chloroformiate d'isobutyle ou d'éthyle avec un acide de formule (II) ou (II'), en présence d'une base telle que la triéthylamine, et à le faire réagir avec un dérivé de N,O-bis-triméthylsilyle d'un acide aminé de formule (III), obtenu par réaction du bis(triméthylsilyl)acétamide ou de la 1,3-bis(triméthylsilyl)urée ou du bis(trifluorométhylsilyl)acétamide avec un acide aminé de formule (III), dans des solvants tels que l'acétonitrile, le DCM, sous atmosphère inerte, pendant un temps compris entre 1 heure et quelques jours, à une température comprise entre la température ambiante et la température de reflux du solvant.

Une autre variante au mode opératoire de l'étape 1) consiste à faire réagir l'anhydride mixte d'un acide 1-phénylpyrazole-3-carboxylique de formule (II) ou (II') avec un acide aminé de formule (III), dans un solvant tel que le DCM, sous une atmosphère inerte, à la température ambiante, pendant un temps compris entre 1 jour et quelques jours, en présence d'une base telle que la triéthylamine.

Lorsque le composé de formule (I) présente une fonction basique et est obtenu sous forme de base libre, la salification est effectuée par traitement avec l'acide choisi, dans un solvant organique ou aqueux. Par traitement de la base libre dissoute par exemple dans un alcool, tel que l'isopropanol, avec une solution de l'acide choisi, dans le même solvant, on obtient le sel correspondant qui est isolé selon les techniques classiques. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le 2-naphtalènesulfonate.

Lorsque le composé de formule (I) présente une fonction basique et est isolé sous forme d'un de ses sels, par exemple le chlorhydrate ou l'oxalate, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Lorsque le produit de formule (I) est obtenu sous forme acide, il peut être transformé en un sel métallique, notamment alcalin, tel que le sel de sodium, ou alcalino-terreux, tel que le sel de calcium, selon les procédés classiques.

Les composés de formule (I) ou (I') peuvent subir une deshydratation, en présence d'un anhydride, par exemple l'anhydride acétique, pour former un dérivé d'oxazolone de formule : 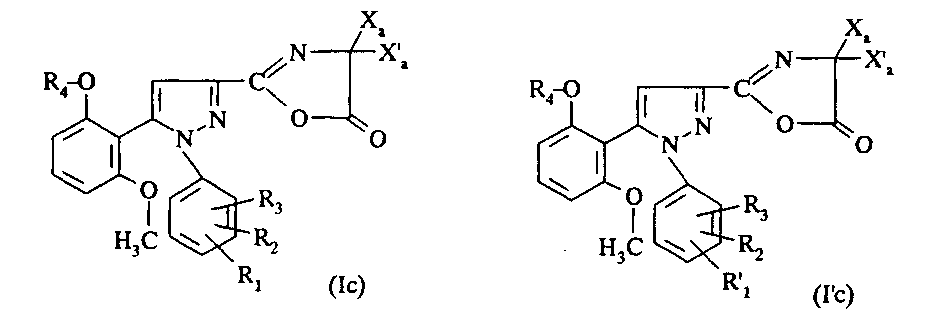 dans laquelle R₁, R'₁, R₂, R₃, R₄, Xₐ, et X'ₐ ont les significations données ci-dessus pour (I) et R'₁ représente un précurseur de R₁ tel que défini ci-dessus.

Ces composés sont nouveaux et constituent un aspect ultérieur de l'invention.

Parmi les composés de formule (Ic) et (I'c) on préfére ceux pour lesquels Xₐ et X'ₐ ensemble avec l'atome de carbone auquel ils sont liés constituent un cycle adamantane, un bicyclo[3.3.1]nonane ou bien Xₐ est l'hydrogène et X'ₐ représente un cyclohexane.

A partir d'un composé de formule (I'c) ou (Ic), on prépare à nouveau un composé de formule (I) ou (I') par hydrolyse en milieu acide ou en milieu basique, par exemple en présence d'un sel alcalin tel que le *tert*-butylate de potassium.

La préparation intermédiaire d'un composé de formule (Ic) peut être utile pour permettre la purification d'un composé de formule (I). Par ailleurs, la préparation intermédiaire d'un composé de formule (I'c) peut être utile pour permettre la transformation d'un substituant R'₁ en un autre substituant R'₁ ou R₁, la fonction acide du groupe NHAA(OH) étant protégée dans le groupe oxazolone.

Les acides 1-phénylpyrazole-3-carboxyliques substitués de formule : 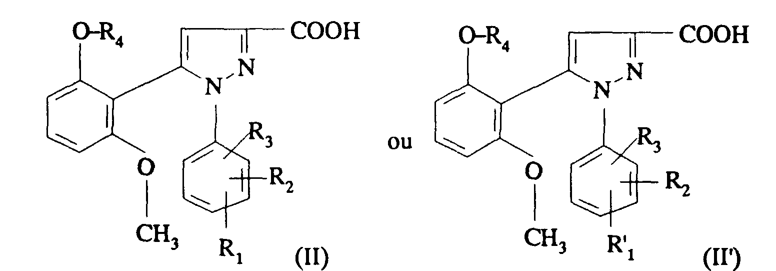 dans laquelle R₁, R₂, R₃, R₄ ont les définitions données ci-dessus pour les composés (I) et R'₁ représente un précurseur de R₁ choisi parmi les groupes halogéno, nitro, amino, phtalimido, hydroxy, hydroxy (C₁-C₇)alkylène, sulfo, cyano, carboxy, (C₁-C₄)alcoxycarbonyle, benzyloxycarbonyle ainsi que leurs dérivés fonctionnels de la fonction acide sont des intermédiaires clés dans la préparation des composés de formule (I) . Lorsque R'₁ est autre que carboxy ou halogéno, les composés de formule (II) et (II') sont nouveaux et ils constituent un aspect ultérieur de la présente invention.

Les acides de formule (II) et (II'), les chlorures des acides de formules (II) et (II'), les esters alkyliques en C₁-C₄ des acides de formules (II) et (II'), qui peuvent être également des précurseurs desdits acides (notamment les esters méthylique, éthylique et *tert*-butylique) et l'anhydride mixte des acides de formule (II) et (II') avec le chloroformiate d'isobutyle ou d'éthyle sont des produits intermédiaires particulièrement préférés.

Le procédé de préparation des composés (II) ou (II') via les esters (IIa) ou (II'a) est représenté par le schéma suivant :

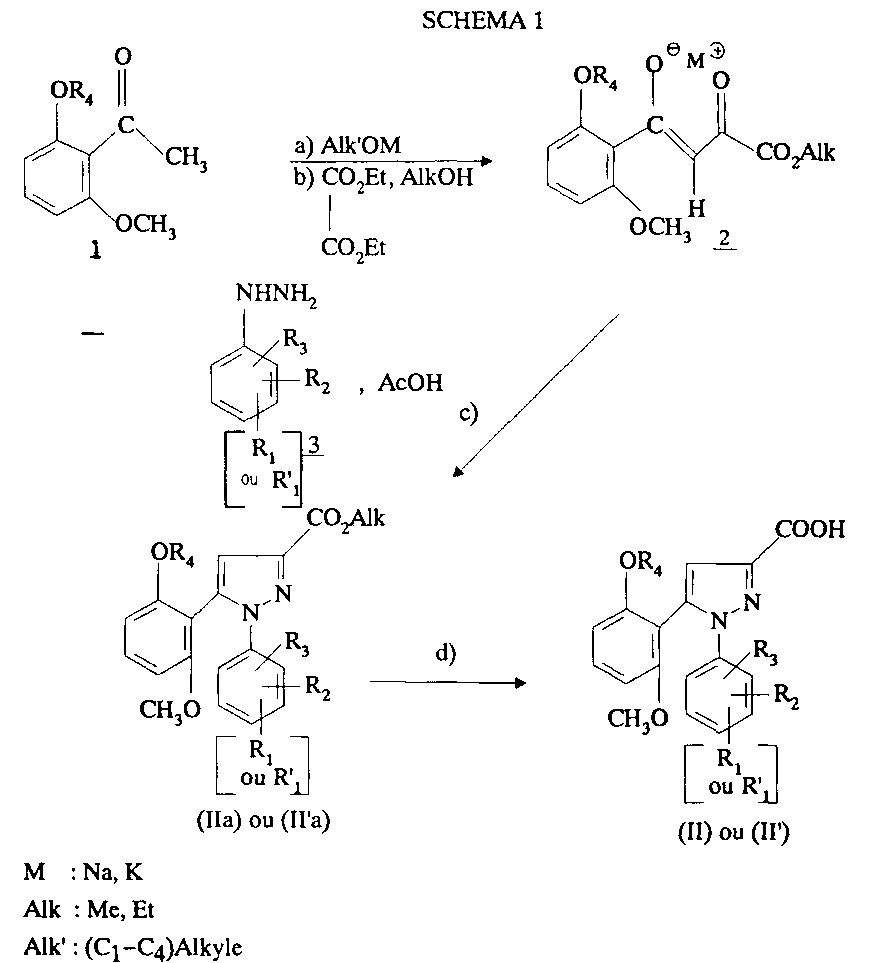

Dans la première étape a) on fait réagir une base forte, telle qu'un alcoolate métallique sur une cétone de formule 1 dans laquelle R₄ est tel que défini précédemment, puis on fait agir (étape b) une quantité équimolaire d'oxalate d'éthyle dans un alcanol comme par exemple le méthanol ou l'éthanol, selon L. Claisen, Ber., 1909, 42, 59. Après précipitation dans un éther, tel que l'éther éthylique ou l'éther isopropylique, les énolates 2 sont séparés par filtration. On peut également préparer un énolate de lithium selon W.V. Murray et al., J. Heterocyclic Chem., 1989, 26, 1389.

L'énolate métallique 2 ainsi préparé et le dérivé de phénylhydrazine 3 ou un sel de celui-ci sont alors chauffés au reflux de l'acide acétique (étape c)) pour obtenir les esters IIa ou II'a.

Par saponification des esters IIa ou II'a, par action d'un agent alcalin comme par exemple l'hydroxyde de potassium, l'hydroxyde de sodium ou l'hydroxyde de lithium puis acidification, on obtient les acides II ou II' (étape d).

Parmi les composés de formule 3 certains sont nouveaux et constituent un objet ultérieur de la présente invention.

Ainsi les composés de formule : 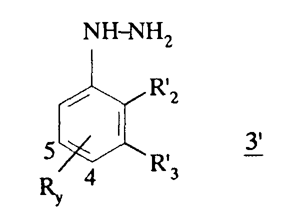 dans laquelle :
- R'₂ et R'₃ représentent chacun indépendamment un hydrogène, un (C₁-C₆)alkyle, un (C₃-C₈)cycloalkyle, un (C₃-C₈)cycloalkylméthyle ;
- ou R'₂ et R'₃ ensemble constituent un groupe triméthylène, tétraméthylène ou pentaméthylène ;
- R_{y} est en position 4 ou en position 5 et représente un groupe choisi parmi : cyano, carboxy, (C₁-C₄)alcoxycarbonyle, benzyloxycarbonyle, sulfo, (C₁-C₄)alkyl sulfonylamino, (C₁-C₄)alkylphénylsulfonylamino, carbamoyle, (C₁-C₄)alkyl carboxamido ;
à la condition que R'₂ et R'₃ ne représentent pas simultanément l'hydrogène et à la condition que R'₂ soit autre que méthyle lorsque R_{y} est le groupe sulfo ;
et leurs sels, sont nouveaux et constituent un objet ultérieur de la présente invention.

Les dérivés de la phénylhydrazine (3) peuvent être préparés selon Houben-Weyl, 1967, X-2, 169. Par exemple on peut réaliser la diazotation de la phénylamine correspondante en présence du nitrite de sodium, puis la réduction du sel de diazonium par exemple par action du chlorure stanneux. Lorsque le phényle comporte un substituant électro attracteur, tel que cyano ou nitro, on peut également substituer un dérivé de fluorophényle par l'hydrate d'hydrazine pour obtenir le dérivé d'hydrazinophényle correspondant.Les phénylamines substituées sont connues ou préparées par des méthodes connues. Par exemple, les acides aminosulfoniques sont préparés selon Houben-Weyl, Methoden der Organischen Chemie. Verlag, 1955, vol. IX, 450.

Les dérivés de la phénylhydrazine substitués par un groupement R₁ = YCO₂R₇ sont préparés à partir de dérivés de l'aniline ou du nitrophényle correspondants.

La transformation d'un composé de formule I' ou respectivement de formule II' ou de formule II'a dans lequel le groupe phényle est substitué par R'₁ en un composé de formule I ou respectivement de formule II ou de formule IIa dans lequel le groupe phényle est substitué par R₁ est effectuée par des méthodes classiques bien connues de l'homme de l'art.

Les composés de formule IIa ou II'a dans lesquels R₁ ou R'₁ représente un groupe carboxy ou carboxy(C₁-C₇)alkylène permettent de préparer des composés de formule IIa dans lesquels R₁ représente un groupe -TCONRₐR_{b} avec une amine HNRₐR_{b} par réaction du chlorure d'acide, préparé intermédiairement ou de tout autre dérivé activé de l'acide tel que les anhydrides mixtes, les esters activés ou les dérivés obtenus avec le 1,3-dicyclohexylcarbodiimide.

De la même façon les composés de formule I' dans lesquels R'₁ représente un groupe -TCOOH permettent de préparer les composés de formule I dans lesquels R₁ représente un groupe -TCONRₐR_{b}, la fonction acide carboxylique du résidu d'acide aminé NHAA(OH) doit alors être protégée de façon intermédiaire, par exemple par un groupe ester tel que *tert*-butylester ou par formation du dérivé oxazolone de formule (Ic).

A partir d'un composé de formule I ou respectivement Ic, ou respectivement d'un ester de formule IIa, ou respectivement d'un sel alcalin d'un acide de formule II dans lesquels le substituant R₁ est un groupe TCONH(CH₂)ₛCR₇R₈(CH₂)ₜNR₅R₆, on peut préparer un composé de formule I ou respectivement de formule Ic ou respectivement de formule IIa, ou respectivement un sel alcalin d'acide de formule II dans lesquels le substituant R₁ est un groupe TCONR₉(CH₂)ₛCR₇R₈(CH₂)ₜNR₅R₆ par action d'une iodure de formule R₉ I.

Les composés de formule IIa, ou respectivement II, ou respectivement Ic dans lesquels R₁ représente un groupe carboxy (C₁-C₇)alkylène permettent de préparer les composés de formule IIa, ou respectivement II, ou respectivement I dans lesquels R₁ représente un groupe -TCONR₇R_{c} par réaction du chlorure d'acide, préparé intermédiairement avec un composé HNR₇R_{c} c'est-à-dire avec un composé de formule HNR₇XOR₇ ou un composé de formule HNR₇CHR₂₀CO₂R₇ ou un composé de formule HNR₇(CH₂)₄CH(NHPro)CO₂R₇ dans laquelle Pro représente un groupe protecteur de la fonction amine utilisé classiquement en chimie peptidique, par exemple *tert*-butoxycarbonyle ou benzyloxycarbonyle.

Par réaction des composés de formule I dans lesquels R₁ représente un groupe -CH₂CONH₂, avec du péroxyde de sodium, on obtient les composés de formule I dans lesquels R₁ représente un groupe carboxyméthyle. Par réduction des composés de formule I dans lesquels R₁ représente un groupe -CH₂CN, par exemple par hydrogénation en présence d'un catalyseur tel que le nickel de Raney® , on obtient les composés de formule I dans lesquels R₁ représente un groupe -CH₂CH₂NH₂. Ces derniers composés permettent de préparer des composés de formule I dans lesquels R₁ représente un groupe -CH₂CH₂NR₅R₆, un groupe -CH₂CH₂N(R₇)CORₑ ou un groupe -CH₂CH₂N(R₇)SO₂R'₇ par des méthodes connues de l'homme de l'art.

De même les composés de formule (I) dans lesquels R₁ représente un groupe -T'-CN, T' étant une liaison directe ou un (C₁-C₆)alkylène permettent de préparer des composés de formule I dans lesquels R₁ représente un groupe T'-CH₂NH₂, puis des composés de formule I dans lesquels R₁ est un groupe -T'-CH₂NR₅R₆, un groupe -T'-CH₂N(R₇)CORₑ ou un groupe -T'-CH₂N(R₇)SO₂R'₇.

La réduction catalytique peut être réalisée selon Catalytic Hydrogenation, R.L. Augustine.- Marcel Dekker, 1967, 96-97 ; elle peut s'appliquer aux composés de formule I dans lesquels R₂ et R₃ sont autres qu'un nitro ou un cyano et R₄ est autre qu'un (C₃-C₄)alcényle. La substitution du groupe amino peut être effectuée par différents procédés décrits par exemple dans Catalytic Hydrogénation,, R.L. Augustine, M. Dekker, 1965, 102-113 et Catalytic Hydrogenation over Platinum Metals, P.N. Rylander. - Academic Press, 1967, 291. Ainsi, par exemple l'addition d'un aldéhyde de formule R_{V}CHO sur un groupe amino conduit à un groupe imine qui par hydrogénation catalytique se transforme en amine secondaire -NHCH₂R_{V} dans laquelle R_{V} représente un hydrogène ou un (C₁-C₃)alkyle. L'addition d'une cétone de formule RCOR' conduit à une amine -NHCHRR' dans laquelle -CHRR' représente un groupe R₅, et R₆ est l'hydrogène. L'addition d'un halogénure de (C₁-C₄)alkyle permet également la préparation d'un groupe amino substitué par un ou deux (C₁-C₄)alkyles. L'addition d'un dihalogénure approprié permet de préparer des composés dans lesquels R₅ et R₆ ensemble avec l'atome d'azote auquel ils sont liés constituent un hétérocycle choisi parmi la pyrrolidine, la pipéridine, la morpholine, la thiomorpholine ou la pipérazine substituée en 4 par R₉, l'aziridine, l'azétidine ou la perhydroazépine.

Les composés de formule IIa dans lesquels R₁ représente un groupe T-NHSO₂R'₇ permettent de préparer des composés de formule II'a dans laquelle R₁ représente un groupe T-N(XNR₅R₆)SO₂R'₇ par action d'un halogènure de formule HaIXNR₅R₆. En milieu acide, on peut ensuite préparer des composés de formule II dans lesquels R₁ est un groupe TNHXNR₅R₆ qui représente TNHR₂₈. Pour obtenir les composés de formule I dans laquelle R₁ est un groupe TNR₂₇R₂₈, la substitution de l'azote est effectuée par des méthodes connues soit sur un composé de formule II, soit sur un composé de formule I.

Les composés de formule II'a, ou respectivement les composés de formule II' ou les composés de formule I', dans lesquels R'₁ représente un groupe nitro peuvent être transformés en composés de formule II'a, ou respectivement de formule II' ou de formule I', dans lesquels R'₁ est un groupe amino ; puis par des méthodes connues, on prépare les composés de formule IIa ou respectivement de formule II ou de formule I, dans lesquels R₁ représente un groupe -N(R₇)CORₑ ou NR₅R₆.

Les composés de formule II'a, ou respectivement, de formule II', ou de formule I', dans lesquels R'₁ est un groupe amino permettent également de préparer des composés de formule II'a ou respectivement de formule II' ou de formule I', dans lesquels R'₁ représente un groupe hydroxyle ; puis, par des méthodes connues, on prépare les composés de formule IIa ou respectivement de formule II ou de formule I dans lesquels R₁ représente un groupe -OR_{d}.

A partir des composés de formule II'a dans laquelle R'₁ est un groupe -Y-OH, on peut préparer des composés de formule II'a dans laquelle R'₁ est un groupe -Y-Cl par action de l'acide chlorhydrique ou du chlorure de thionyle ; par action d'un dérivé d'acide sulfonique sur ces mêmes composés, portant le substituant -Y-OH on peut préparer des composés de formules II'a dans lesquels R'₁ est le groupe -Y-OSO₂W, W représentant un méthyle, un trifluorométhyle ou un tolyle. L'action d'une amine HNR₅R₆, sur des composés de formule II'a substitués par un groupe -Y-Cl ou un groupe -Y-O-SO₂W permet de préparer des composés de formule IIa dans laquelle R₁ représente un groupe -Y-NR₅R₆.

Lorsque R₂ représente un nitro ou un cyano et R₃ représente l'hydrogène, l'action d'un composé R_{d}OH, en milieu basique, sur un composé de formule II'a, ou respectivement de formule II', ou respectivement de formule I', dans laquelle R'₁ est un halogène en position ortho ou para par rapport à R₂, permet de préparer des composés de formule IIa, ou respectivement de formule II ou respectivement de formule I, dans laquelle R₁ est un groupe -OR_{d}.

Lorsque R₂ et R₃ sont différents d'un atome d'halogène, on peut également préparer un composé de formule IIa, ou respectivement de formule II, ou respectivement de formule I, dans laquelle R₁ est un cyano à partir d'un composé de formule II'a, ou respectivement de formule II',ou respectivement I', dans laquelle R'₁ est un halogène par action d'un dérivé cyané, par exemple le cyanure cuivreux.

Les composés de formule I dans lesquels R₁ est un groupe cyano permettent de préparer, par réaction avec du péroxyde d'hydrogène en présence d'une base telle que la soude, les composés de formule I dans lesquels R₁ est un groupe carbamoyle. De la même façon on obtient les composés de formule II dans lesquels R₁ est un groupe carbamoyle à partir des composés de formule IIa dans lesquels R₁ est un groupe cyano.

Les composés de formule I dans lesquels R₁ est un groupe cyano permettent également de préparer, par réaction avec l'hydroxylamine éventuellement O-substituée par -(CH₂)_{q}NR₅R₆ en présence d'une base telle que le carbonate de potassium, les composés de formule I dans lesquels R₁ représente un groupe -C(NH₂) = NOH ou un groupe -C(NH₂) = NO(CH₂)ᵣNR₅R₆.

A partir des composés de formule IIa, ou respectivement de formule II, ou respectivement de formule I dans laquelle R₁ représente un groupe C(NH₂) = NOH, on prépare les composés de formule IIa, ou respectivement de formule II, ou respectivement de formule I dans laquelle R₁ représente C(=NOH)NH(CH₂)ᵣNR₅R₆ selon Chem. Ber., 1970, 103, 2330-2335.

Par réduction des composés de formule IIa ou respectivement de formule II ou de formule I dans lesquelles R₁ représente un groupe cyano, par exemple par hydrogénation en présence d'un catalyseur tel que l'oxyde de platine, puis réaction avec un chlorure d'acide ou un anhydride approprié ou respectivement avec un chlorure de sulfonyle, on obtient les composés de formule IIa ou respectivement de formule II ou de formule I dans lesquels R₁ représente un groupe -CH₂NHCOR₁₆ ou respectivement -CH₂NHSO₂R'₇. De même on obtient les composés de formule IIa ou respectivement de formule II ou de formule I dans lesquels R₁ représente un groupe-CH₂N(R₇)COR₁₆ ou un groupe -CH₂N(R₇)SO₂R'₇ avec R₇ autre que l'hydrogène, en effectuant une réaction d'alkylation sur l'amide obtenue intermédiairement.

Lorsque l'hydrogénation d'un composé de formule II'a ou respectivement de formule II', ou respectivement de formule I' dans lesquelles R'₁ représente un groupe cyano, est effectuée en présence d'une amine NHR₅R₆, on obtient un composé de formule I ou respectivement de formule II, ou respectivement de formule IIa dans lesquelles R₁ représente un groupe CH₂NR₅R₆.

La réaction des composés de formule I dans lesquels R₁ représente un groupe TCN, -T-CON(R₇)(CH₂)_{q}CN ou un groupe -T-N(R₇)CO(CH₂)_{q}CN ou un groupe -SO₂N(R₇)(CH₂)_{q}CN avec de l'acide chlorhydrique en solution alcoolique AlkOH, permet d'obtenir intermédiairement l'imidate correspondant de formule :
-T-C(=NH)OAlk, -T-CONR₇(CH₂)_{q}C(=NH)OAlk, -T-N(R₇)CO(CH₂)_{q}C (=NH)-OAlk, ou -SO₂N(R₇)(CH₂)_{q}C(=NH)OAlk dans laquelle Alk est un (C₁-C₄) alkyle.

Si l'on fait réagir l'imidate avec une quantité équimolaire d'amine HNR₁₂R₁₃ on obtient les composés de formule I dans lesquels R₁ représente :
un groupe -TC(NR₁₂R₁₃)=NR₁₄
un groupe -TCON(R₇)(CH₂)_{q}C(NR₁₂R₁₃) = NR₁₄ ou
un groupe -TN(R₇)CO(CH₂)_{q}C(NR₁₂R₁₃)=N-R₁₄ ou
un groupe -SO₂N(R₇)CH₂)_{q}C(NR₁₂R₁₃)=NR₁₄ , dans lesquels R₁₄ = H

Si l'on fait réagir l'imidate avec un excès d'amine NH₂R₁₃, dans lequel R₁₃ est différent de l'hydrogène, on obtient les composés de formule I dans lesquels R₁ représente :
un groupe -TC(NHR₁₃)=NR₁₃
un groupe -TCON(R₇)(CH₂)_{q}C(NHR₁₃)=N-R₁₃ ou
un groupe -TN(R₇)CO(CH₂)_{q}C(NHR₁₃)=NR₁₃ ou
un groupe -SO₂N(R₇)(CH₂)_{q}C(NHR₁₃)=NR₁₃.

Si l'on fait réagir l'imidate avec une diamine de formule H₂N-Z-NHR₁₂, on obtient les composés de formule I dans lesquels R₁ représente :
un groupe -TC(NR₁₂R₁₃)=NR₁₄
un groupe -TCON(R₇)(CH₂)_{q}C(NR₁₂R₁₃)=N-R₁₄ ou
un groupe -TN(R₇)CO(CH₂)_{q}C(NR₁₂R₁₃)=NR₁₄ ou
un groupe -SO₂N(R₇)(CH₂)_{q}C(NR₁₂R₁₃)=NR₁₄ dans lesquels R₁₃ et R₁₄ ensemble constituent un groupe alkylène en C₂-C₆ et R₁₂ représente un hydrogène ou un (C₁-C₄)alkyle.

On peut également préparer un composé de formule (I) dans laquelle R₁ contient un radical amidino éventuellement substitué selon les méthodes décrites dans The chemistry of amidines and imidates, Saul Patai, 1975, John Wiley and Sons.

A partir des composés de formule I, ou respectivement de formule II ou IIa dans laquelle R₁ représente un groupe -TCONR₇(CH₂)_{q}CN, on prépare par des réactions connues, des composés de formule I, ou respectivement II ou IIa dans laquelle R₁ représente un groupe -TCONR₇(CH₂)_{q}CONH₂ ou un groupe -TCONR₇-(CH₂)_{q}CO₂R₇.

Lorsque R'₁ = SO₃H on prépare un composé II'a dans lequel R'₁ = SO₂Cl puis on le transforme en un autre composé IIa dans lequel R₁ est un groupe aminosulfonyle, éventuellement substitué, par action d'une amine appropriée HNRₐR_{b}.

Les composés de formule I comprenant un groupe ammonium quaternaire sont obtenus à partir des composés aminés correspondants par action d'un composé de formule QR₂₄ dans laquelle Q est un anion, par exemple un iodure.

Les acides aminés de formule III incluent par exemple la glycine, l'alanine, la leucine, la norleucine, l'isoleucine, la valine, la 1-adamantylglycine, la 2-adamantylglycine, la cyclopropylglycine, la cyclopentylglycine, la cyclohexylglycine, la cycloheptylglycine, l'acide 1-aminocyclopropanecarboxylique, l'acide 1-aminocyclobutanecarboxylique, l'acide 1-aminocyclopentanecarboxylique, l'acide 1-aminocyclohexanecarboxylique, l'acide 1-aminocycloheptanecarboxylique, l'acide 1-amino-4-méthylcyclohexane carboxylique, l'acide 2-aminoadamantane-2-carbo xylique, l'acide 2-aminobicyclo [3.2.1]octane-2-carboxylique, l'acide 9-aminobicyclo [3.3.1]nonane-9-carboxylique, l'acide 2-aminobicyclo[2.2.1]heptane-2-carboxylique ou 2-aminonorbornane-2-carboxylique.

Les acides aminés de formule III sont des produits commerciaux ou peuvent être très facilement préparés selon les méthodes classiques. Notamment les aminoacides (III) non commerciaux sont préparés selon la synthèse de Strecker, Ann, 1850, 75, 27 ou selon la synthèse de H.T. Bucherer et al., J. Pract. Chem., 1934, 141, 5, suivie d'une hydrolyse pour conduire aux aminoacides ; par exemple, l'acide 2-aminoadamantane-2-carboxylique et l'acide 9-aminobicyclo[3.3.1]nonane-9-carboxylique sont préparés selon H.T. Nagasawa et al., J. Med. Chem., 1973, 16 (7), 823.

Les acides α-amino-1-adamantylacétique et α-amino-2-adamantylacétique sont préparés selon B. Gaspert et al., Croatica Chemica Acta, 1976, 48 (2), 169-178.

L'acide 2-aminonorbomane-2-carboxylique est préparé selon H.S. Tager et al., J. Am. Chem. Soc., 1972, 94, 968.

Les acides α-aminocycloalkyl carboxyliques sont préparés selon J.W. Tsang et al., J. Med. Chem., 1984, 27, 1663.

Les cyclopentylglycines R et S sont préparées selon la demande de brevet européen EP 477049.

Les cyclohexylglycines R et S sont préparées selon Rudman et al., J. Am. Chem. Soc., 1952, 74, 551.

On peut également préparer les cyclohexylglycines R et S par hydrogénation catalytique des phénylglycines R et S.

On peut aussi préparer les acides α-aminocycloalkylcarboxyliques de configuration R ou S par hydrolyse enzymatique, stéréospécifique, des dérivés N-acétylés racémiques correspondants, selon J. Hill et al., J. Org. Chem., 1965, 1321.

Les composés de formule (I) et leurs sels possèdent une très grande affinité pour les récepteurs humains de la neurotensine, dans les tests décrits par D. Gully et al. dans Proc. Natl. Acad. Sci. USA, 1993, 90, 65-69.

Les composés de formule I et leurs sels ont été étudiés *in vivo*. En opérant selon la technique décrite par M. Poncelet *et al*. dans Naunyn Schmiedberg's Arch. Pharmacol., 1994, 60, 349-357, on observe qu'un composé selon l'invention, administré par voie orale, antagonise les rotations contralatérales induites par injection intrastriatale unilatérale de neurotensine chez la souris.

Par ailleurs, en opérant selon la technique décrite par D. Nisato *et al*. dans Life Sciences, 1994, 54, 7, 95-100, on constate qu'un composé selon l'invention, administré par voie intraveineuse, inhibe l'augmentation de la pression artérielle induite par injection intra-veineuse de neurotensine chez le cobaye.

Les composés décrits dans le brevet EP 0477 049 présentent dans ces tests une activité inférieure à celle des composés selon la présente invention.

Les composés de la présente invention sont peu toxiques ; notamment, leur toxicité aiguë est compatible avec leur utilisation comme médicament. Pour une telle utilisation, on administre aux mammifères une quantité efficace d'un composé de formule I, ou d'un de ses sels pharmaceutiquement acceptables, pour le traitement des pathologies neurotensine-dépendantes. Ainsi les composés de la présente invention peuvent être utilisés pour le traitement de désordres neuropsychiatriques en particulier ceux qui sont liés à un dysfonctionnement des systèmes dopaminergiques, par exemple les psychoses, plus particulièrement la schizophrénie et les maladies du mouvement comme la maladie de Parkinson, (D.R. Handrich et al., Brain Research, 1982, 231, 216-221 et C.B. Nemeroff, Biological Psychiatry, 1980, 15 (2), 283-302). Ils peuvent être utiles pour diagnostiquer et/ou traiter les maladies cancéreuses, par exemple les méningiomes humains non accessibles chirurgicalement (P. Mailleux, Peptides, 1990, 11, 1245-1253), les cancers de la prostate (I. Sehgal *et al*., Proc. Nat. Acad. Sci., 1994, 91, 4673-4677), les cancers du poumon à petites cellules (T. Sethi *et al*., Cancer Res., 1991, 51, 3621-3623). Ils peuvent être utilisés dans le traitement de désordres gastrointestinaux moteurs, sécrétoires, ulcéreux et/ou tumoraux (Revue de A. Shulkes dans "Gut Peptides : Biochemistry and Physiology, Ed. J. Waish and G.J. Dockray, 1994"). Ainsi les composés I selon l'invention peuvent être utiles dans le traitement d'affections telles que : syndrome du colon irritable, diarrhée, colites, ulcères, tumeurs du tractus gastro-intestinal, dyspepsie, pancréatite, oesophagite. Ils peuvent également présenter un intérêt en tant que modulateurs de la prise de nourriture (Beck, B. Metabolism., 1995, 44, 972-975). Les composés selon l'invention peuvent être indiqués comme diurétiques ainsi que dans le cas de troubles cardiovasculaires, et également dans le cas de pathologies associées à une libération d'histamine telles que les processus inflammatoires (D.E. Cochrane *et al*., Faseb J., 1994, 8, 7, 1195). Ces composés peuvent être également intéressants pour soigner certains désordres occasionnés par le stress, tels que migraines, prurit neurogénique et cystite intersticielle (Theoharides T.C. et al., Endocrynol., 1995, 136, 5745-5750). Les composés de la présente invention peuvent également présenter un intérêt dans l'analgésie en agissant sur les effets de la morphine (M.O. Urban, J. Pharm. Exp. Ther., 1993, 265, 2, 580-586).

Ainsi, la présente invention a pour objet, selon un autre de ses aspects, des compositions pharmaceutiques contenant, comme principes actifs, les composés de formule I ou leurs sels éventuels pharmaceutiquement acceptables.

Dans les compositions pharmaceutiques de la présente invention pour l'administration orale, sublinguale, sous-cutanée, intramusculaire, intraveineuse, transdermique ou rectale, les principes actifs peuvent être administrés, sous formes unitaires d'administration, en mélange ou avec des supports pharmaceutiques classiques aux animaux et aux êtres humains. Les formes unitaires d'administration appropriées comprennent les formes par voie orale, telles que les comprimés, les gélules, les poudres, les granules et les solutions ou suspensions orales, les formes d'administration par voie inhalée, les formes d'administration sublinguale et buccale, les formes d'administration sous-cutanée, transcutanée, intramusculaire ou intraveineuse et les formes d'administration rectale.

Afin d'obtenir l'effet désiré, la dose de principe actif peut varier entre 0,5 et 1000 mg par jour, de préférence entre 2 et 500 mg.

Chaque dose unitaire peut contenir de 0,5 à 250 mg de principe actif, de préférence de 1 à 125 mg, en combinaison avec un support pharmaceutique. Cette dose unitaire peut être administrée 1 à 4 fois par jour.

Lorsqu'on prépare une composition solide sous forme de comprimés, on mélange le principe actif avec un véhicule pharmaceutique, tel que la gélatine, l'amidon, le lactose, le stéarate de magnésium, le talc, la gomme arabique, ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, tels que la polyvinylpyrrolidone et similaires, de même qu'avec des édulcorants ou des correcteurs du goût.

Pour une administation rectale on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Pour améliorer la solubilité des produits de l'invention, les composés de formule I ou leurs sels pharmaceutiquement acceptables peuvent être également présentés sous forme de complexes avec des cyclodextrines.

Dans la description et dans les exemples, les abréviations suivantes sont utilisées.
MeOH : méthanol
EtOH : éthanol
Ether : éther éthylique
Ether iso : éther isopropylique
Ether chlorhydrique : solution saturée d'acide chlorhydrique dans l'éther
Ethanol chlorydrique : solution saturée d'acide chlorhydrique dans l'éthanol
ACOEt : acétate d'éthyle
MeCN : acétonitrile
DCM : dichlorométhane
DMF : diméthylformamide
DMSO : diméthylsulfoxyde
THF : tétrahydrofurane
HCl : acide chlorhydrique
H₂SO₄ : acide sulfurique
AcOH : acide acétique
TFA : acide trifluoroacétique
H₂SO₄ : acide sulfurique
NaOH : soude
KOH : potasse
LiOH : hydroxyde de lithium
NH₄OH : ammoniaque
Na₂SO₄ : sulfate de sodium
NaHCO₃ : hydrogénocarbonate de sodium
NaHSO₃ : hydrogénosulfite de sodium
Na₂CO₃ : carbonate de sodium
K₂CO₃ : carbonate de potassium
P₂O₅ : anhydride phosphorique
NBS : N-bromosuccinimide
POCl₃ : oxychlorure de phosphore
NaNO₂ : nitrite de sodium
SOCl₂ : chlorure de thionyle
SnCl₂ : chlorure stanneux
CuCN : cyanure cuivreux
Me, MeO : méthyle, méthoxy
Et : éthyle
iPr : isopropyle
iBu : isobutyle
n-Bu : n-butyle
t-Bu : *tert*-butyle
Bz : benzyle
F : point de fusion
TA : température ambiante
Silice H : gel de silice 60 H commercialisé par MERCK (DARMSTADT)
RMN : résonance magnétique nucléaire

Sauf indication contraire, les spectres de RMN sont enregistrés à 200 MHz dans le DMSO-d₆. Les déplacements chimiques δ sont exprimés en partie par million (p.p.m.) par rapport au tétraméthylsilane comme référence interne.
s : singulet
s.e : singulet élargi
s.d : singulet dédoublé
d : doublet
d.d : doublet de doublet
t : triplet
qd : quadruplet
qt : quintuplet
sp : septuplet
m : massif
mt : multiplet

### Préparation 1.1

Sel de sodium du 4-(2,6-diméthoxyphényl)-4-oxydo-2-oxobut-3-énoate de méthyle : Composé A.

On ajoute lentement une solution de 100 g de 2,6-diméthoxyacétophénone et 7,5 ml d'oxalate d'éthyle dans 520 ml de MeOH anhydre à une solution de méthylate de sodium préparée à partir de 12,7 g de sodium et 285 ml de MeOH anhydre. On chauffe à reflux pendant 7 heures et laisse une nuit à TA. On verse le mélange réactionnel sur 2 litres d'éther isopropylique et laisse 15 minutes sous agitation. On obtient le produit attendu par filtration, lavage à l'éther isopropylique et séchage sous vide, m = 120 g, F = 178°C.

Sel de potassium du 4-(2,6-diméthoxyphényl)-4-oxydo-2-oxobut-3-énoate d'éthyle : composé A₁.

A une solution de 18 g de 2,6-diméthoxyacétophénone dans 54 ml d'éthanol agitée et chauffée à 50°C on ajoute en 6 minutes une solution de 13,4 g de tertiobutylate de potassium 95 % dans 72 ml d'éthanol. On chauffe à reflux, ajoute 16,3 ml d'oxalate d'éthyle en 9 minutes et poursuit le reflux pendant 1 heure. On distille ensuite 40 ml d'éthanol et laisse refroidir sous agitation pendant 2 heures et demie. On filtre, lave le précipité avec 40 ml d'éthanol et sèche sous vide à 60°C pendant 17 heures pour obtenir 31 g de produit attendu.

RMN : 1,2 : t : 3H ; 3,6 : s : 6H ; 4 : mt : 2H ; 5,5 : s : 1H ; 6,55 : d : 2H ; 7,1 : t : 1H.

### Préparation 1.2

Sel de sodium du 4-[2-(cyclopropylméthyloxy)-6-méthoxyphényl]-4-oxydo-2-oxobut-3-énoate d'éthyle.

A) 2-(Cyclopropylméthyloxy)-6-méthoxyacétophénone.

A une solution de 26 g de 2-hydroxy-6-méthoxyacétophénone dans 400 ml de propan-2-ol on ajoute, à TA, 32,7 ml d'une solution à 50 % d'hydroxyde de césium dans l'eau et laisse 15 minutes sous agitation à TA. On concentre sous vide, reprend le résidu avec du propan-2-ol, concentre sous vide, puis ajoute du toluène et concentre sous vide. On dissout le résidu dans 200 ml de DMF, ajoute 25,3 g de bromure de cyclopropylméthyle et chauffe à 80°C pendant 2 heures 30 minutes. On concentre sous vide, reprend le résidu à l'eau, extrait à l'AcOEt, lave la phase organique par une solution saturée de NaCl, sèche sur Na₂SO₄ et évapore sous vide le solvant. On obtient 32,7 g du produit attendu.

B) Sel de sodium du 4-[2-(cyclopropylméthyloxy)-6-méthoxyphényl]-4-oxydo-2-oxobut-3-énoate d'éthyle.

On ajoute lentement une solution de 32,6 g du composé obtenu à l'étape précédente et 20,1 ml d'oxalate de diéthyle dans 100 ml d'EtOH à une solution d'éthylate de sodium préparée à partir de 3,4 g de sodium et 60 ml d'EtOH. On chauffe une nuit à 60°C, laisse refroidir à TA et concentre sous vide. On reprend le résidu au pentane, essore le précipité formé, le lave au pentane et le sèche sous vide. On obtient 41,2 g du produit attendu.

### PREPARATIONS DES HYDRAZINES 3

### Préparation 2.1

Chlorhydrate de l'acide 3-isopropyl-4-hydrazinobenzoïque.

A) 2-Isopropyl-acétanilide.

Ce composé est décrit dans Bull. Soc. Chim., France, 1949, 144.

On refroidit dans la glace un mélange contenant 300 ml de toluène et 31 ml de 2-isopropylaniline et on ajoute lentement 22 ml d'anhydride acétique. Après 40 minutes sous agitation à TA, on évapore le milieu réactionnel puis on reprend à l'éther de pétrole. On essore le précipité formé. On obtient 35,9 g du produit attendu après cristallisation dans l'éther de pétrole, F = 81°C.

B) 4-Bromo-2-isopropylacétanilide.

Ce composé est décrit dans J. Med. Chem., 1974, 17(2), 221.

A un mélange contenant 34,8 g du composé obtenu à l'étape précédente dans 250 ml d'acide acétique, on ajoute lentement quelques gouttes d'une solution de 10,1 ml de brome dans 180 ml d'acide acétique puis on chauffe à 50°C ; après refroidissement on ajoute à nouveau quelques gouttes de la solution et on chauffe à 50°C et ce jusqu'à la fin de l'addition. Le milieu réactionnel est chauffé progressivement jusqu'au reflux puis on le laisse revenir à TA pendant une nuit. Le précipité formé est filtré puis ajouté à une solution diluée de NaHSO₃. On filtre à nouveau, rince à l'eau puis sèche sur P₂O₅. On obtient 27,4 g du produit attendu, F = 134°C.

Le composé de l'étape B) peut également être préparé selon le mode opératoire décrit ci-dessous.

B') 4-Bromo-2-isopropylacétanilide.

On prépare un mélange contenant 117,6 g de 2-isopropylacétanilide dans 330 ml de DMF et on ajoute en 25 minutes 117,6 g de NBS dans 330 ml de DMF. On laisse sous agitation à TA pendant 5 heures puis on verse sur 1,5 litre d'eau en refroidissant le milieu réactionnel par de la glace. Le précipité formé est filtré, rincé à l'eau puis séché à 50°C sous vide. Le filtrat est extrait au DCM (2 fois) lavé à l'eau puis séché sur Na₂SO₄ pour obtenir une seconde fraction du produit attendu. En réunissant les différentes fractions purifiées on obtient 158 g du produit attendu, F = 134°C.

C) 4-Cyano-2-isopropylacétanilide.

On agite à reflux pendant 10 heures un mélange contenant 26,48 g du produit obtenu à l'étape précédente, 60 ml de DMF, 1 ml d'eau et 10,25 g de cyanure cuivreux. Après refroidissement, on verse sur une solution de 50 g de cyanure de sodium dans 150 ml d'eau, à 40°C. On filtre le précipité formé et on le rince plusieurs fois à l'eau. On obtient 16,7 g du produit attendu, F = 134°C.

D) Chlorhydrate de 4-cyano-2-isopropylaniline.

On mélange 16,13 g du composé obtenu à l'étape précédente, 65 ml d'éthanol 100 et 40 ml d'HCl 1N et on agite à reflux pendant 19 heures. Après une nuit à TA, on ajoute une solution de NaOH à 10 % jusqu'à obtenir pH = 10. Le milieu réactionnel est extrait 2 fois au DCM, on sèche la phase organique sur Na₂SO₄ et concentre sous vide. Le résidu est dissous dans l'éther et on ajoute de l'éther chlorhydrique. On filtre le précipité formé et rince à l'éther. On obtient 15,32 g du produit attendu qui cristallise dans le mélange Et₂O/HCl, F = 188°C.

E) Chlorhydrate de l'acide 4-amino-3-isopropylbenzoïque.

Ce composé est décrit dans J. Med. Chem., 1974, 17(2), 221.

On chauffe à reflux pendant 12 heures un mélange contenant 1 g du composé obtenu à l'étape précédente, 2,86 g de potasse broyée, 6 ml d'eau et 0,5 ml de diméthoxyéthane. Après refroidissement, on ajoute HCl concentré jusqu'à obtenir pH = 1, puis on extrait 2 fois au DCM ; on sèche la phase organique sur Na₂SO₄ et concentre. On obtient 0,96 g du produit attendu, F = 128°C.

F) Chlorhydrate de l'acide 3-isopropyl-4-hydrazinobenzoïque.

On refroidit à -5°C un mélange contenant 0,96 g du produit obtenu à l'étape précédente, 22 ml d'HCl concentré et 20 ml d'acide acétique , on ajoute 0,36 g de NaNO₂ dans 4 ml d'eau puis on laisse sous agitation à 0°C pendant 1 heure 15 minutes. On refroidit à -10°C et on ajoute 3,73 g de dihydrate de chlorure stanneux dans 4 ml d'HCl concentré. On laisse remonter la température jusqu'à 18°C puis on filtre le précipité formé et le rince par 1 ml d'HCl dilué. On obtient 0,96 g du produit attendu après séchage sur P₂O₅.

### Préparation 2.1 bis

Le chlorhydrate de l'acide 3-isopropyl-4-hydrazinobenzoïque peut également être préparé selon le mode opératoire décrit ci-après.

A) 4-Bromo-2-isopropylacétanilide.

On ajoute en 10 minutes 200 ml d'anhydride acétique sur 300 ml de 2-isopropylaniline en maintenant la température inférieure à 60°C. Après 45 minutes d'agitation à TA, on ajoute une solution d'un équivalent de NBS dans 720 ml de DMF. Après 2 heures d'agitation on verse sur 5,7 1 de mélange eau/AcOEt (2/1 ; v/v), décante, sépare la phase organique, sèche sur Na₂SO₄ et évapore sous vide. On concrétise le résidu dans l'éther isopropylique et filtre pour obtenir 367 g du produit attendu.

B) 4-Cyano-2-isopropylacétanilide.

On chauffe à reflux 10,25 g du produit de l'étape A et 1,2 équivalent de cyanure cuivreux dans 20 ml de DMF pendant 6 heures. On refroidit à 20°C, verse sur un mélange de 200 ml d'AcOEt et 200 ml d'ammoniaque à 20 %. On relave la phase organique avec 50 ml d'ammoniaque à 20 % puis 2 fois avec une solution saturée de NaCl. Après sèchage sur Na₂SO₄, on évapore sous vide, traite le résidu avec de l'éther isopropylique, filtre et sèche à 40° sous vide pour obtenir 6,15 g du produit attendu.

Le composé de l'étape B peut aussi être préparé selon le mode opératoire ci-dessous :

B') On fait barbotter de l'argon dans un mélange agité de 22,16 g de produit de l'étape A et 0,6 équivalent de cyanure de zinc dans 67 ml de DMF anhydre. On chauffe à 80°C et ajoute 2 g de Tétrakis(triphénylphosphine) palladium (0) à l'abri de la lumière. Après 3 heures d'agitation à 80°C on laisse refroidir à TA et ajoute 120 ml d'ammoniaque à 4 % et 200 ml d'AcOEt, relave les phases organiques rassemblées avec de l'ammonique à 4 %. On sèche sur Na₂SO₄, évapore sous vide, traite le résidu à l'éther isopropylique, filtre et sèche sous vide pour obtenir 15 g du nitrile attendu.

C) Chlorhydrate de l'acide 4-amino-3-isopropylbenzoïque.

On chauffe pendant 10 heures à reflux un mélange de 100 g du produit de l'étape B avec 500 ml d'HCl concentré et 500 ml d'AcOH. On concentre sous vide, filtre le précipité et sèche sous vide pour obtenir 103,8 g de produit attendu.

D) Chlorhydrate de l'acide 3-isopropyl-4-hydrazinobenzoïque.

A un mélange, refroidi à -5°C, de 59 g du produit de l'étape C avec 1050 ml d'AcOH et 1420 ml d'HCl concentré, on ajoute lentement une solution de 27,7 g de NaNO₂ dans 250 ml d'eau. Après 1 heure 20 minutes d'agitation à 0°C on refroidit à -10°C et ajoute une solution de 236 g de SnCl₂, 2H₂O dans 250 ml d'HCl concentré. On laisse remonter à TA, filtre le précipité, lave avec HCI concentré et sèche sous vide pour obtenir 56,36 g de produit attendu.

### Préparation 2.2

Chlorhydrate de l'acide 3-isopropyl-4-hydrazinobenzènesulfonique.

A) Acide 4-amino-3-isopropylbenzènesulfonique.

On ajoute 5,7 ml de H₂SO₄ à 10 ml d'eau et on chauffe à 80°C puis on ajoute 13,5 g de 2-isopropylaniline. L'eau est évaporée par chauffage sous vide puis on augmente la température progressivement en 1 heure et demie pour atteindre 260°C. Après 3 heures sous agitation et sous vide à 260°C, on laisse revenir à TA et à pression atmosphérique puis on chauffe 30 minutes en présence de 15 ml de NaOH et 100 ml d'eau pour dissoudre le milieu réactionnel. On filtre l'insoluble, refroidit à 5°C puis on acidifie à pH 1 par addition de H₂SO₄ concentré. On filtre le précipité formé, lave avec 5 ml d'eau froide et sèche pour obtenir 20 g du produit attendu
RMN : 1,1 : d : 6H ; 2,95 : mt : 1H ; 6,85 : d : 1H ; 7,45 : dd : 1H ; 7,6 : d : 1H.

B) Chlorhydrate de l'acide 3-isopropyl-4-hydrazinobenzènesulfonique.

A une solution de 10 g du produit préparé à l'étape précédente dans 10 ml de NaOH à 30 % et 20 ml d'eau, on ajoute 10 ml de glace et 3,2 g de NaNO₂. On verse lentement cette solution sur une solution de 30 ml d'HCl concentré dans 20 ml d'eau à une température comprise entre -5°C et -15°C. On laisse sous agitation pendant une heure à cette température puis on ajoute 26 g de dihydrate de chlorure stanneux dans 40 ml d'HCl concentré à une température comprise entre 0°C et -5°C. Après 2 heures et demie sous agitation à TA, on filtre puis on sèche le produit obtenu sous vide, en présence de P₂O₅. On obtient ainsi 9,7 g du produit attendu.
RMN : 1.2 : d : 6H ; 3,15 : mt : 1H ; 6,8 : d : 1H ; 7,45 : d : 1H ; 7,55 : s : 1H ; 7,9 : se : 1H ; 10 : se : 3H.

### Préparation 2.3

Chlorhydrate de l'acide 4-hydrazino-5,6,7,8-tétrahydronaphtalène-1-carboxylique.

A) Acide 4-amino-5,6,7,8-tétrahydronaphtalène-1-carboxylique.

L'acide 4-nitro-5,6,7,8-tétrahydronaphtalène-1-carboxylique est décrit dans Chem. Pharm. Bull., 1984, 32, 3968. On effectue l'hydrogénation de 1,48 g de ce dérivé nitré dans le méthanol en présence de nickel de Raney® . Après 4 heures sous agitation, on filtre le catalyseur, évapore à sec, reprend à l'éther et filtre pour obtenir 1 g du produit attendu. F = 180°C (déc.).

B) Chlorhydrate de l'acide 4-hydrazino-5,6,7,8-tétrahydronaphtalène-1-carboxylique.

A une solution de 0,73 g d'acide 4-amino-5,6,7,8-tétrahydronaphtalène-1-carboxylique dans 10 ml d'HCl concentré refroidie à -5°C on ajoute une solution de 0,26 g de NaNO₂ dans 1 ml d'eau. Après 1 heure et demie d'agitation à -5°C on ajoute à -5°C une solution de 3,4 g SnCl₂, 2H₂O dans 34 ml d'HCl concentré. On agite 1 heure à TA, filtre, lave par HCl concentré, sèche sous courant d'azote sec pour obtenir 0,67 g de l'hydrazine attendue.

### Préparation 2.4

Chlorhydrate de l'acide 4-hydrazino-5,6,7,8-tétrahydronaphtalène-1-sulfonique.

A) Acide 4-amino-5,6,7,8-tétrahydronaphtalène-1-sulfonique.

A une solution de 10 g de 5,6,7,8-tétrahydronaphtylamine dans 100 ml de 1,2-dichlorobenzène on ajoute une suspension chaude de 20 g d'acide sulfamique dans 40 ml de N-méthylpyrrolidone. On chauffe sous agitation 7 heures à 150°C. On filtre, lave au dichlorobenzène puis au toluène. Le précipité est remis en suspension dans 70 ml d'eau, neutralisé à pH 7 avec 5,5 ml de soude à 30 %. On filtre l'insoluble, extrait la phase aqueuse à l'éther. On ajuste à pH = 5 la phase aqueuse par addition d'HCl, à 5°C ; on filtre, lave à l'eau et sèche pour obtenir 7,5 g du produit attendu.

B) Chlorhydrate de l'acide 4-hydrazino-5,6,7,8-tétrahydronaphtalène-1-sulfonique.

A une solution de 3 g de l'acide obtenu à l'étape A dans 10 ml d'eau et 2 ml de soude à 30 % on ajoute 1 g de NaNO₂. On verse en 1 heure cette solution sur 10 ml d'HCl concentré refroidi à 5°C. Après agitation 3 heures à 5°C on ajoute lentement une solution de 7,5 g de SnCl₂, 2H₂O dans 15 ml d'HCl concentré en maintenant à 5°C. On laisse sous agitation 1 heure et demie à TA, filtre et sèche sous vide pour obtenir 2,86 g du produit attendu.
RMN (D₂O - NaOD) :1,6 : mt : 4H ; 2,25 : mt : 2H ; 2,9 : mt : 2H ; 6,75 : d : 1H; 7,6 : d : 1 H;

### Préparation 2.5

Chlorhydrate de 4-hydrazino-3-méthylbenzamide.

A) 4-Amino-3-méthylbenzamide.

Ce produit est préparé par hydrogénation catalytique de 3-méthyl-4-nitrobenzamide, F = 124°C.

B) Chlorhydrate de 4-hydrazino-3-méthylbenzamide.

On dissout 0,5 g du composé de l'étape A dans 10 ml HCl 1N et 5 ml d'HCl concentré. On refroidit à 0°C et on ajoute une solution de 230 mg de NaNO₂ dans 3 ml d'eau. Après 15 minutes à -10°C, on ajoute une solution de 1,5 g de SnCl₂, 2H₂O dans 5 ml d'HCl concentré. Après 1 heure, on filtre le précipité et sèche sous vide sur P₂O₅ pour obtenir 390 mg du produit attendu.

### Préparation 2.6

Chlorhydrate de l'acide 2,3-diméthyl-4-hydrazinobenzoïque.

A une solution de 4,5 g d'acide 4-amino-2,3-diméthylbenzoïque dans 135 ml d'HCl concentré refroidie à -5°C, on ajoute lentement une solution de 1,87 g de NaNO₂ dans 7 ml d'eau. Après 2 heures d'agitation à -5°C, on ajoute, à -10°C, une solution de 25 g SnCl₂, 2H₂O dans 250 ml d'HCl concentré, agite 30 minutes à -5°C puis 2 heures à TA. On filtre, lave le précipité avec 5 ml d'HCl concentré, sèche sous courant d'azote sec puis sous vide pour obtenir 5,5 g de produit attendu.
RMN : 2,1 : s : 3H ; 2,4 : s : 3H ; 6,8 : d : 2H ; 7,6 : d : 2H ; 8,2 : s : 1H ; 10 : s.e. : 2H.

### Préparation 2.7

Chlorhydrate de l'acide 4-hydrazino-3-méthoxybenzoïque.

A une solution de 5 g d'acide 4-amino-3-méthoxybenzoïque dans 50 ml d'HCl concentré refroidie à 0°C, on ajoute lentement une solution de 2,17 g de NaNO₂ dans 40 ml H₂O. Après 1 heure 15 minutes d'agitation à 0°C, on refroidit à -10°C, ajoute, en 30 mn, une solution de 23,6 g de SnCl₂, 2H₂O dans 20 ml d'HCl concentré et 20 ml d'eau. Après 1 heure et demie d'agitation à -10°C on filtre, lave le précipité avec 50 ml de pentane pour obtenir, après séchage, 6 g de produit attendu.
RMN : 3,8 : s : 3H ; 7 : d : 1H ; 7,4 : s : 1H ; 7,5 : d.d : 1H ; 8 : s.e : 1H ; 10,6 : s.e : 2H.

### Préparation 2.8

Chlorhydrate de 2-chloro-4-hydrazinobenzonitrile.

A -5°C on mélange, 5 g de 4-amino-2-chlorobenzonitrile et 40 ml d'HCl concentré dans 30 ml de THF ; on ajoute 2,26 g de NaNO₂ dans 30 ml d'eau et laisse 2 heures sous agitation puis on ajoute 30 g de SnCl₂, 2H₂O dans 30 ml d'HCl concentré et maintient l'agitation à -5°C pendant 30 minutes.

Après retour à TA, on filtre l'insoluble, ajoute du NaCl et agite à nouveau. Le produit attendu cristallise avec NaCl, il est repris dans l'éthanol, tandis que NaCl est filtré. Après évaporation des solvants on obtient 4,25 g du produit attendu.

### Préparation 2.9

Chlorhydrate de l'acide 3-cyclopropyl-4-hydrazinobenzoïque.

A) 4-Acétamido-3-cyclopropyibenzonitrile.

A une solution de 4,3 g de 4-bromo-2-cyclopropylacétanilide (préparé selon J. Am. Chem. Soc., 1968, 90 3404) dans 100 ml de DMF on ajoute 1,67 g de CuCN et chauffe 24 heures à reflux. On verse sur 30 ml d'eau, filtre, lave le précipité à l'eau puis on agite pendant 30 minutes, le précipité dans un mélange de 59 ml d'eau et 25 ml d'éthylènediamine. Après extraction par 100 ml AcOEt, séchage sur Na₂SO₄ et évaporation sous vide, on obtient 2,39 g du produit attendu.

RMN : 0,6 : m : 2H ; 0,9 : m : 2H ; 1,9 : m : 1H ; 2,1 : s : 3H ; 7,3 : d : 1H ; 7,5 : d.d : 1H ; 7,8 : d : 1H ; 9,5 : s.e : 1H.

B) Chlorhydrate du 4-amino-3-cyclopropylbenzonitrile.

On agite pendant 12 heures à reflux un mélange de 2,39 g du produit de l'étape A en solution dans 45 ml d'éthanol avec 36 ml d'eau et 5 ml d'HCl concentré. On évapore sous vide l'éthanol, filtre le précipité, le lave avec 1 ml d'eau et sèche sous vide pour obtenir 1,5 g du produit attendu.

RMN : 0,5 : m : 2H ; 0,9 : m : 2H ; 1,6 : m : 1H ; 6,7 : d : 1H ; 7,1-7,3 : mt : 2H ; 8 : s.e : 2H.

C) Chlorhydrate de l'acide 4-amino-3-cyclopropylbenzoïque.

On agite pendant 29 heures à reflux 1,3 g du produit de l'étape B dans 21 ml de KOH à 50 %. Après acidification à pH 1 avec HCl concentré, on filtre et sèche sous vide pour obtenir 0,96 g du produit attendu.

RMN : 0,5 : m : 2H ; 0,9 : m: 2H ; 1,7 : m : 1H ; 5,9 : s.e : 2H ; 6,6 : d : 1H ; 7,4 : s : 1H ; 7,5 : mt : 1H ; 12 : s.e : 1H.

D) Chlorhydrate de l'acide 3-cyclopropyl-4-hydrazinobenzoïque.

A une solution de 0,95 g du produit obtenu à l'étape C dans 22 ml d'HCl concentré et 21 ml d'AcOH refroidie à -5°C, on ajoute lentement une solution de 0,38 g NaNO₂ dans 4,5 ml d'eau et agite 1 h 15 à 0°C. On refroidit à -10°C et ajoute lentement une solution de 3,76 g de SnCl₂, 2H₂O dans 8 ml d'HCl concentré. Après 4 heures d'agitation à TA on filtre, lave avec 2 ml d'HCl concentré et sèche sous vide pour obtenir 1 g de produit attendu.

RMN : 0,6 : m : 2H ; 1 : m : 2H ; 1,9 : m : 1H ; 7,1 : d : 1H ; 7,6 : s : 1H ; 7,8 : d : 1 H ; 8,4 : s : 1H ; 10,7 : s.e : 2H.

### Préparation 2.10

Chlorhydrate de l'acide 5-hydrazino-2-chlorobenzoïque.

A une suspension de 5 g d'acide 5-amino-2-chlorobenzoïque dans 50 ml d'HCl concentré refroidie à -2°C on ajoute, en 30 minutes, une solution de 2,11 g de NaNO₂ dans 40 ml d'eau. On agite 2 heures à -3°C, refroidit la solution à -10°C et ajoute en 30 minutes une solution de 23 g de SnCl₂, 2H₂O dans 20 ml d'HCl concentré et 20 ml d'eau. On agite 1 heure et demie à 0°C, filtre et sèche le précipité pour obtenir 4 g de produit attendu.

RMN : 7,6 : s.e : 2H ; 7,7 : s.e : 1H ; 8,4 : s.e : 1H ; 11,0 : s.e : 3H.

### Préparation 2.11

Chlorhydrate de l'acide 3-hydrazino-4-méthylbenzoïque.

A une solution de 5 g d'acide 3-amino-4-méthylbenzoïque dans 120 ml d'HCl concentré et 40 ml d'AcOH, refroidie à -5°C, on ajoute, en 30 minutes, une solution de 2,74 g de NaNO₂ dans 28 ml d'eau et on laisse sous agitation 1 heure 20 minutes à 0°C. Après refroidissement à -10°C on ajoute lentement une solution de 27,6 g de SnCl₂, 2H₂O dans 28 ml d'HCl concentré. Après agitation 1 heure à TA, filtration, lavage du précipité avec 5 ml d'HCl 1N, séchage sur P₂O₅ sous vide on obtient 6,15 g du produit attendu.

En opérant selon les modes opératoires ci-dessus, à partir de dérivés de l'aniline correctement substitués, on prépare les hydrazines décrites dans le tableau 1 ci-dessous.

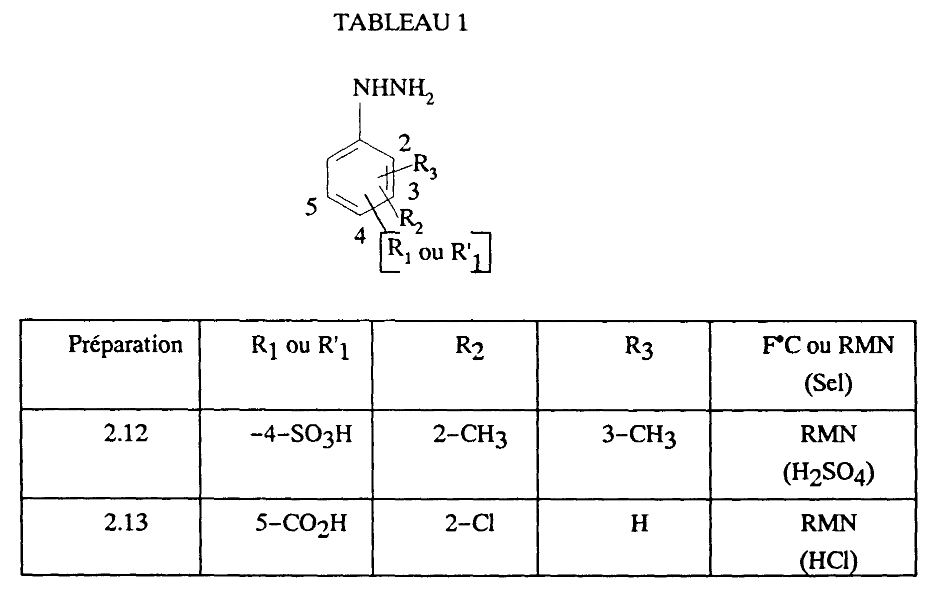

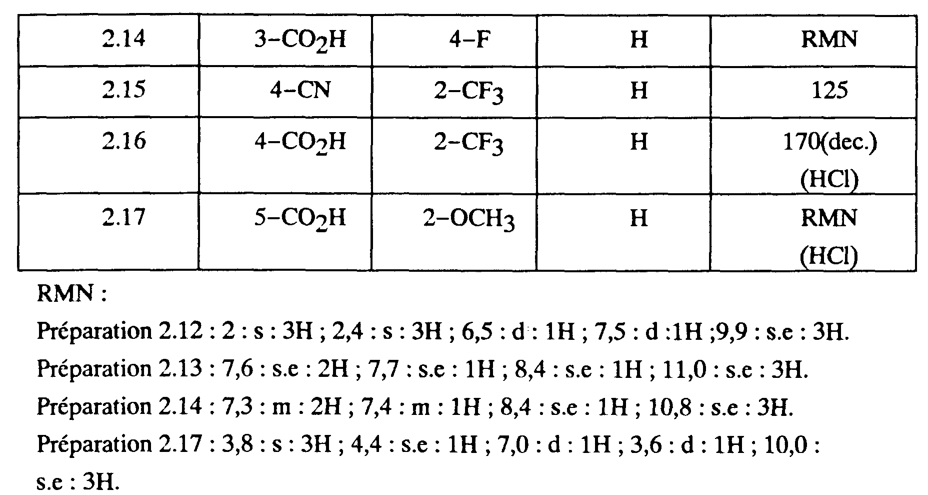

### Préparation 2.18

Oxalate de N-(4-hydrazino-3-isopropylphényl)-4-méthylbenzènesulfonamide.

A) N-(2-Bromo-5-isopropyl-4-nitrophényl)-4-méthylbenzènesulfonamide.

On refroidit à -30°C une solution de 23,47 g de N-(4-nitrophényl)-4-méthylbenzène sulfonamide dans 230 ml de THF, ajoute 100 ml d'une solution 2M de chlorure d'isopropylmagnésium dans l'éther et laisse 30 minutes sous agitation à -30°C. Puis on ajoute 10,3 ml de brome à -30°C, laisse 15 minutes sous agitation à cette température puis laisse remonter la température à 20°C. On ajoute ensuite 55 ml de triéthylamine et laisse 1 heure sous agitation à TA. On ajoute de l'eau, acidifie à pH = 3-4 par ajout d'une solution d'HCl 10 %, décante la phase organique, extrait la phase aqueuse à l'éther et sèche les phases organiques jointes sur Na₂SO₄. On agite la phase organique en présence de noir animal, filtre et concentre sous vide. On reprend le résidu dans l'EtOH et essore le produit cristallisé formé. On obtient 11,6 g du produit attendu, F = 132°C.
RMN : 1,0 : d : 6H ; 2,32 : s : 3H ; 3,12 : s : 1H ; 7,14 : s : 1H ; 7,36 : d : 2H ; 7,65 : d : 2H ; 8,08 : s : 1H ; 10,25 : se : 1H.

B) N-(4-amino-3-isopropylphényl)-4-méthylbenzènesulfonamide.

On hydrogène pendant 7 heures à TA et sous une pression de 1 bar un mélange de 11,5 g du composé obtenu à l'étape précédente et 1 g de palladium sur charbon à 5 % dans 200 ml de MeOH et 30 ml de DMF. On filtre le catalyseur et concentre sous vide le filtrat. On reprend le résidu à l'eau, neutralise à pH = 7 par ajout d'une solution à 10 % de NaOH, extrait à l'AcOEt, sèche la phase organique sur Na₂SO₄ et évapore sous vide le solvant. On obtient 8 g du produit attendu.
RMN : 1,01 : d : 6H ; 2,4 : s : 3H ; 2,89 : mt : 1H ; 4,91 : se : 2H ; 6,42-6,68 : m : 3H ; 7,35 : d : 2H ; 7,56 : d : 2H ; 9,38 : s : 1H.

C) Oxalate de N-(4-hydrazino-3-isopropylphényl)-4-méthylbenzènesulfonamide.

On agite à 0°C un mélange de 6,68 g du composé obtenu à l'étape précédente et 70 ml d'une solution concentrée d'HCl, ajoute une solution de 1,48 g de NaNO₂ dans 5 ml d'eau et laisse 1 heure sous agitation à 0°C. On ajoute ensuite une solution de 11,35 g de dithionite de sodium dans 60 ml d'eau et poursuit l'agitation 1 heure à 0°C. Puis on ajoute 120 g d'acétate de sodium en poudre, 300 ml d'eau et laisse 30 minutes sous agitation à 0°C. On extrait le mélange réactionnel à l'AcOEt, sèche la phase organique sur Na₂SO₄, filtre, ajoute au filtrat une solution de 1,98 g d'acide oxalique dans un minimum d'EtOH et concentre sous vide. On reprend le résidu dans l'éther isopropylique, laisse 12 heures sous agitation et essore le précipité formé. On obtient 4,84 g du produit attendu que l'on utilise tel quel.

### Préparation 2.19

Fluorhydrate de 1-Hydrazino-2-méthyl-4-nitrobenzène.

On chauffe à reflux pendant 2 heures un mélange de 4,6 g de 1-fluoro-2-méthyl-4-nitrobenzène et 3 ml d'hydrate d'hydrazine dans 45 ml de propan-2-ol. On ajoute 3 ml d'hydrate d'hydrazine, poursuit le reflux pendant 2 heures et laisse une nuit sous agitation à TA. On essore le précipité formé. On obtient 3,53 g du produit attendu, F = 182°C.

### Préparation 2.20

Bromhydrate de N-(4-hydrazino-3-isobutylphényl)-4-méthylbenzènesulfonamide.

A) N-(2-bromo-5-isobutyl-4-nitrophényl)-4-méthylbenzènesulfonamide.

On prépare ce composé selon le mode opératoire décrit à l'étape A de la Préparation 2.18 à partir de 10 g de N-(4-nitrophényl)-4-méthylbenzènesulfonamide dans 100 ml de THF et 42,6 ml d'une solution 2M de chlorure d'isobutylmagnésium dans l'éther puis 4,4 ml de brome et 23,4 ml de triéthylamine. On obtient 5,9 g du produit attendu, F = 170°C.
RMN : 0,8 : d : 6H ; 1,7 : mt : 1H ; 2,35 : s : 3H ; 2,6 : d : 2H ; 7,2 : s : 1H ; 7,3 : d : 2H ; 7,4 : d : 2H ; 8,2 : s : 1H ; 10,25 : s : 1H.

B) N-(4-Amino-3-isobutylphényl)-4-méthylbenzènesulfonamide.

On prépare ce composé selon le mode opératoire décrit à l'étape B de la Préparation 2.18 à partir de 4,7 g du composé obtenu à l'étape précédente. On obtient 2,8 g du produit attendu.
RMN : 0,8 : d : 6H ; 1,75 : mt : 1H ; 2,2 : d : 2H ; 2,4 : s : 3H ; 4,8 : s : 2H ; 6,45 : d : 1H ; 6.5 : d : 1H ; 6,65 : dd : 1H ; 7,35 : d : 2H ; 7,55 : d : 2H ; 9,4 : s : 1H.

C) Bromhydrate de N-(4-hydrazino-3-isobutylphényl)-4-méthylbenzène sulfonamide.

On prépare ce composé selon le mode opératoire décrit à l'étape C de la Préparation 2.18 à partir de 2,5 g du composé obtenu à l'étape précédente, 35 ml d'HCl concentré, 50 ml d'acide acétique et 0,53 g du NaNO₂ puis 4,78 g de dithionite de sodium dans 50 ml d'eau, 140 g d'acétate de sodium et 70 ml d'eau. Après 30 minutes d'agitation à 0°C, on ajoute de l'acide bromhydrique à 0°C, on essore le produit cristallisé et le sèche. On obtient 1,9 g du produit attendu.

RMN : 0,65 : d : 6H ; 1,6 : mt : 1H ; 2,05 : d : 2H ; 2,2 : s : 3H ; 6,05 : se : 1H; 6,4 : se : 1H ; 6,6-6,8 : m : 2H ; 7,2 : d : 2H ; 7,4 : d : 2H.

### Préparation 2.21

Oxalate de N-(4-hydrazino-3-cyclopentylphényl)-4-méthylbenzène sulfonamide.

A) N-(2-bromo-5-cyclopentyl-4-nitrophényl)-4-méthylbenzènesulfonamide.

On prépare ce composé selon le mode opératoire décrit à l'étape A de la Préparation 2.18 à partir de 15 g de N-(4-nitrophényl)-4-méthylbenzènesulfonamide dans 100 ml de THF et 64 ml d'une solution 2M de chlorure de cyclopentylmagnésium dans l'éther puis 6,8 ml de brome et 35 ml de triéthylamine. On obtient 6,1 g du produit attendu, F = 122°C.
RMN (DMSO + TFA) : 1,25 : mt : 2H ; 1,5-1,7 : m : 4H ; 1,95 : mt : 2H ; 2,36 : s : 3H ; 3,2 : qt : 1H ; 7,12 : s : 2H ; 7,4 : d : 2H ; 7.7 : d : 2H ; 8,08 : s : 1H.

B) N-(4-Amino-3-cyclopentylphényl)-4-méthylbenzènesulfonamide.

On prépare ce composé selon le mode opératoire décrit à l'étape B de la Préparation 2.18 à partir de 6 g du composé obtenu à l'étape précédente. On obtient 4,25 g du produit attendu, F = 128°C.
RMN (DMSO + TFA) : 1,25 : mt : 2H ; 1,5-1,75 : m : 4H ; 1,95 : mt : 2H ; 2,3 : s : 3H ; 3,0 : qt : 1H ; 7,0 : dd : 1H ; 7,12 : d : 1H ; 7,2 : d : 1H ; 7,34 : d : 2H ; 7,65 : 2H.

C) Oxalate de N-(4-hydrazino-3-cyclopentylphényl)-4-méthylbenzène sulfonamide.

On prépare ce composé selon le mode opératoire décrit à l'étape C de la Préparation 2.18 à partir de 3,35 g du composé obtenu à l'étape précédente, 20 ml d'H₂SO₄, 50 ml d'acide acétique, 10 ml d'eau et 0,69 g de NaNO₂ puis 5,5 g de dithionite de sodium dans 50 ml d'eau et 200 g d'acétate de sodium et 300 ml d'eau. On obtient 2,42 g du produit attendu.

### Préparation 2.22

Chlorhydrate de l'acide 4-hydrazino-2-isopropylbenzoïque.

A) 4-Iodo-3-isopropylacétanilide.

On prépare ce composé selon le procédé décrit dans Bull. Soc. Chim. Jap., 1989, 62, 1349.

A une solution de 5 g de 3-isopropylacétanilide dans 150 ml d'acide acétique, on ajoute à TA 5,7 g de chlorure de zinc et 10,8 g de benzyltriméthylammonium dichloroiodate et laisse 2 jours sous agitation. On concentre sous vide, reprend le résidu par 100 ml d'une solution à 5 % d'hydrogénosulfite de sodium, amène à pH = 5-6 par ajout d'une solution à 10 % de Na₂CO₃, extrait 4 fois par 200 ml de chloroforme et sèche la phase organique sur Na₂SO₄. Après filtration, on chromatographie le filtrat sur 100 g d'alumine en éluant au chloroforme. On obtient 5,2 g du produit attendu.
RMN : 1,1 : d : 6H ; 2,0 : s : 3H ; 3,06 : m : 1H ; 7,28 : dd : 1H ; 7,5 : d : 1H ; 7,72 : d : 1H ; 10,0 : se : 1H.

B) 4-Iodo-3-isopropylaniline.

On chauffe à reflux pendant 6 heures un mélange de 5,1 g du composé obtenu à l'étape précédente dans 40 ml d'EtOH 96 et 25 ml de NaOH concentrée. On concentre sous vide, extrait à l'éther, sèche la phase organique sur Na₂SO₄ et évapore sous vide le solvant. On obtient 5 g du produit attendu sous forme d'huile.
RMN : 1,16 : d : 6H ; 2,94 : m : 1H ; 5,2 : se : 2H ; 6,2 : dd : 1H ; 6,6 : d : 1H ; 7,4 : d : 1H.

C) Acide 4-amino-2-isopropylbenzoïque.

A une solution de 5 g du composé obtenu à l'étape précédente dans 60 ml de DMF, on ajoute 40 ml d'eau et 11 g de K₂CO₃ puis on dégaze la solution pendant 10 minutes par barbotage d'azote. On ajoute ensuite 0,5 g d'acétate de palladium (II) puis dégaze pendant 10 minutes par barbotage d'azote. On place le mélange réactionnel sous une pression de 1 bar de monoxyde de carbone pendant 10 heures et sous agitation. On filtre la solution, lave 4 fois par 20 ml d'eau et concentre sous vide le filtrat. On reprend le résidu par 50 ml d'eau et 10 ml d'une solution saturée de NaCl, lave la phase aqueuse à l'éther, acidifie à pH = 3,5-4 par ajout d'HCl concentré, extrait à l'AcOEt, lave la phase organique par une solution saturée de NaCl, sèche sur Na₂SO₄ et évapore sous vide le solvant. On obtient 2 g d'un produit brut que l'on reprend dans 20 ml d'une solution saturée d'HCl gaz dans le méthanol et chauffe à reflux pendant une nuit. On concentre sous vide, reprend le résidu dans 20 ml d'eau, alcalinise à pH = 8 par ajout de NaOH concentrée et extrait par 30 ml de DCM. On ajoute à la phase organique 0,8 ml d'anhydride acétique, NaHCO₃ et Na₂SO₄ et laisse sous agitation. Après filtration, on concentre sous vide le filtrat et chromatographie le résidu sur silice en éluant par le mélange DCM/Ether (50/50 ; v/v). On obtient 0,8 g d'ester méthylique de l'acide 4-acétamido-2-isopropylbenzoïque sous forme d'huile. On chauffe à reflux pendant une nuit un mélange de 0,8 g du produit obtenu et 3 g de KOH dans 10 ml d'eau et 2 ml de 1,2-diméthoxyéthane. Après refroidissement à TA, on lave le mélange réactionnel à l'éther, acidifie la phase aqueuse à pH = 3-4 par ajout d'HCl concentré, extrait à l'AcOEt, sèche la phase organique sur Na₂SO₄ et évapore sous vide le solvant. On obtient 0,6 g du produit attendu.
RMN : 1,17 : d : 6H ; 4,0 : sp ; 1H ; 5,7 : se : 2H ; 6,36 : dd : 1H ; 6,80 : d : 1H; 7,6 : d : 1H ; 11,80 ; se : 1H.

D) Chlorhydrate de l'acide 4-hydrazino-2-isopropylbenzoïque.

On refroidit à 0°C un mélange de 0,5 g du composé obtenu à l'étape précédente dans 7 ml d'HCl concentré, ajoute une solution de 0,23 g de NaNO₂ dans 4 ml d'eau et laisse 1 heure 30 minutes sous agitation à 0°C. On refroidit à -10°C et ajoute une solution de 2,6 g de SnCl₂, 2H₂O dans 5 ml d'HCl concentré et 3 ml d'eau et laisse 2 heures sous agitation à 0°C. On essore le précipité formé, le lave avec HCl concentré et le sèche à 50°C sous vide. On obtient 0,36 g du produit attendu.
RMN : 1,15 : d : 6H ; 3,98 : m : 1H ; 6,7 : dd : 1H ; 6,96 : d : 1H ; 7,8 : d : 1H ; 8,2 : se : 1H ; 13 : se : 4H.

### Préparation 2.23

Chlorhydrate de 1-hydrazino-4-nitro-5,6,7,8-tétrahydronaphtalène.

A) 1-Acétamido-5,6,7,8-tétrahydronaphtalène.

On laisse 1 heure sous agitation à TA un mélange de 24,39 g de 1-amino-5,6,7,8-tétrahydronaphtalène et 18,6 ml d'anhydride acétique dans 230 ml de DCM. On concentre sous vide, reprend le résidu à l'éther et essore le précipité formé. On obtient 26,8 g du produit attendu, F = 158°C.

B) 1-Amino-4-nitro-5,6,7,8-tétrahydronaphtalène.

On refroidit à 0°C un mélange de 13 g du composé obtenu à l'étape précédente dans 72 ml d'acide sulfurique concentré, ajoute un mélange de 4,55 ml d'acide nitrique (d = 1,4) et 22 ml d'acide sulfurique concentré et laisse 45 minutes sous agitation à 0°C. On verse le mélange réactionnel sur de la glace et essore le précipité formé. On reprend le précipité dans 145 ml d'EtOH, 30 ml d'HCl concentré et 30 ml d'eau puis chauffe à reflux pendant 1 heure 30 minutes. On évapore 70 ml du mélange réactionnel, ajoute à la solution restante 220 ml d'eau, amène à pH = 7 par ajout d'une solution concentrée d'ammoniaque, essore le précipité formé et le sèche. On reprend le précipité par 210 ml de nitrobenzène, refroidit à 0°C et fait barboter pendant 50 minutes un courant d'HCl gaz. On essore le précipité formé et le lave à l'éther. On reprend le précipité au MeOH, neutralise par ajout d'une solution concentrée d'ammoniaque, ajoute de l'eau et essore le précipité. On obtient 5,15 g du produit attendu après séchage, F = 114°C.

C) Chlorhydrate de 1-hydrazino-4-nitro-5,6,7,8-tétrahydronaphtalène.

On refroidit à 3°C un mélange de 3,8 g du composé obtenu à l'étape précédente dans 70 ml d'HCl concentré, ajoute une solution de 1,34 g de NaNO₂ dans 2 ml d'eau et laisse 2 heures sous agitation à 3°C. Puis on ajoute une solution de 18,2 g de SnCl₂, 2H₂O dans 90 ml d'HCl concentré et laisse 30 minutes sous agitation à 3°C puis laisse remonter la température à TA. On essore le précipité formé et le sèche. On obtient 6,3 g du produit attendu mélangé à des sels d'étain.
RMN (DMSO + TFA) : 1,7 : mt : 4H ; 2,55 : mt : 2H ; 2,85 : mt : 2H ; 6,88 : d : 1H ; 7,85 : d : 1H.

### Préparation 2.24

3-Diéthylamino-N-(4-isopropyl-3-hydrazinophényl)propionamide.

A) Oxalate de 3-diéthylamino-N-(4-isopropyl-3-nitrophényl)propionamide.

On traite 4 g de 4-isopropyl-3-nitroaniline (préparée selon J. Org. Chem., 1954, 19, 1067) par chauffage à reflux pendant 1 heure avec 8,14 ml de bis(triméthylsilyl)acétamide dans 20 ml d'acétonitrile, on ajoute le chlorure d'acide préparé à partir de 4 g de chlorhydrate de l'acide 3-N,N-diéthylaminopropanoïque dans le DCM, puis 8,9 ml de triéthylamine. Après 1 heure d'agitation à TA on évapore à sec, extrait au DCM, lave à l'eau, puis avec NaOH 5 %. Après séchage sur Na₂SO₄, on évapore sous vide, redissout l'huile obtenue dans le minimum d'éthanol, ajoute 1,2 g d'acide oxalique. Après 2 heures d'agitation, on filtre pour obtenir 4,5 g d'oxalate attendu, F = 165°C.

B) Oxalate de 3-diéthylamino-N-(4-isopropyl-3-aminophényl)propionamide.

On hydrogène pendant 5 heures une solution de 4,5 g de dérivé nitré obtenu à l'étape précédente dans 100 ml de MeOH et 10 ml de DMF avec du nickel de Raney® Après filtration du catalyseur, on concentre sous vide, précipite par addition d'éther isopropylique et laisse sous agitation pendant 1 heure à TA. Après filtration on obtient 3 g d'oxalate de l'aniline attendue, F = 127°C.

C) 3-Diéthylamino-N-(4-isopropyl-3-hydrazinophényl)propionamide.

A un mélange de 2,7 g d'amine obtenue à l'étape précédente et de 30 ml d'HCl concentré refroidi à 0°C, on ajoute 0,67 g de NaNO₂ dissous dans le minimum d'eau et laisse agiter pendant 1 heure à 0°C. On ajoute ensuite 5 g de dithionite de sodium dissous dans le minimum d'eau et agite encore 30 minutes à 0°C. On ajoute 50 g d'acétate de sodium en poudre et agite 30 minutes à 0°C. On ajoute 50 g d'eau et extrait quelques impuretés avec AcOEt. On sature la phase aqueuse avec NaCl, remonte le pH à 9 avec NH₄OH 20 %, extrait au DCM, sèche sur Na₂SO₄, et évapore sous vide. On laisse cristalliser le résidu dans le pentane pendant une nuit et filtre pour obtenir 3,26 g d'hydrazine attendue, F = 77-80°C.

### PREPARATIONS DES ESTERS IIa,.II'a.

### Préparation 3.1

Ester méthylique de l'acide 1-(4-carboxy-2-isopropylphényl)-5-(2,6-diméthoxyphényl)pyrazole-3-carboxylique.

(II'a : R'₁ = 4-CO₂H ; R₂ = 2-iPr ; R₃ = H ; R₄ = CH₃).

On porte à reflux pendant 5 heures un mélange contenant 0,96 g du produit obtenu à la Préparation 2.1, et 1,2 g du composé A obtenu à la Préparation 1.1, dans 15 ml d'acide acétique. Après 3 jours à TA, on verse sur un mélange eau et glace. Le précipité formé est filtré, rincé à l'eau puis séché sur P₂O₅. On obtient 1,34 g du produit attendu, F = 228-230°C.
RMN : 0,85 : d : 6H ; 2,55 : s.p : 1H ; 3,5 : s : 6H ; 3,75 : s : 3H ; 6,5 : d : 2H ; 6,75 : s : 1H ; 7,05-7,3 : m : 2H ; 7,7 : d : 1H ; 13,05 : s.e : 1H.

### Préparation 3.1 bis

Ester éthylique de l'acide 1-(4-carboxy-2-isopropylphényl)-5-(2,6-diméthoxy phényl)pyrazole-3-carboxylique.

Par réaction du produit de la Préparation 2.1 ou 2.1 bis avec le composé A1 selon le mode opératoire de la Préparation 3.1 on obtient, après recristallisation dans l'AcOEt, l'ester éthylique attendu, F = 231°C.

### Préparation 3.2

Ester méthylique de l'acide 1-[4-[N-méthyl-N-(3-N',N'-diméthylamino propyl)-carbamoyl]-2-isopropylphényl]-5-(2,6-diméthoxyphényl)pyrazole-3-carboxylique.

(IIa : R₁ = 4-CON(CH₃)(CH₂)₃NMe₂ ; R₂ = 2-iPr ; R₃ = H ; R₄ = CH₃).

A) Ester méthylique de l'acide 1-(4-chloroformyl-2-isopropylphényl)-5-(2,6-diméthoxyphényl)pyrazole-3-carboxylique.

On prépare un mélange contenant 26 g du produit obtenu à la préparation 3.1, et 170 ml de chlorure de thionyle. On laisse sous agitation à TA pendant 1 jour. On évapore sous vide, reprend le résidu par du DCM, évapore, l'opération est répétée 3 fois.

B) Ester méthylique de l'acide 1-[4-[N-méthyl-N-(3-N'-N'-diméthylaminopropyl)-carbamoyl]-2-isopropylphényl]-5-(2,6-diméthoxyphényl)pyrazole-3-carboxylique.

A 50 ml de DCM on ajoute 9,3 ml de triéthylamine et 9,9 ml de N,N,N'-triméthyl-1,3-propanediamine. On ajoute sous azote le produit obtenu à l'étape précédente dans 280 ml de DCM et on laisse sous agitation à TA pendant 3 heures et demie. Après lavage à l'eau (2 fois), on sèche sur MgSO₄ et évapore sous vide. Le résidu est repris par de l'éther. Après filtration de l'insoluble et évaporation, le résidu est chromatographié sur silice en éluant par DCM/MeOH/H₂O (95/5/0,5 ; v/v/v jusqu'à 88/12/0,8 ; v/v/v). On obtient 24,5 g du produit attendu.
RMN : 0,95 : d : 6H ; 1,7 : m : 2H ; 1,9-2,4 : m : 8H ; 2,95 : s.d : 3H ; 3,5 : m : 2H ; 3,7 : s : 6H ; 3,9 : s : 9H ; 6,7 : d : 2H ; 6,9 : s : 1H ; 7,1-7,5 : m : 4H.

### Préparation 3.2 bis

Ester éthylique de l'acide 1-[4-[N-méthyl-N-(3-N',N'-diméthylaminopropyl) carbamoyl]-2-isopropylphényl]-5-(2,6-diméthoxyphényl)pyrazole-3-carboxylique.

A) Ester éthylique de l'acide 1-(4-chloroformyl-2-isopropylphényl)-5-(2,6-diméthoxyphényl)pyrazole-3-carboxylique.

A 50 ml de SOCl₂ refroidi à 5° on ajoute 26 g du produit de la Préparation 3.1 ou 3.1 bis et agite pendant 5 heures à TA sous barbottage d'azote à sec. Après évaporation sous vide on reprend le résidu par du DCM et évapore sous vide ; l'opération est répétée 2 fois.

On peut aussi préparer le chlorure d'acide selon le mode opératoire A' ci-dessous :
A') A une solution de 5 g du produit de la Préparation 3.1 ou 3.1 bis dans 50 ml de DCM on ajoute 2,5 ml de SOCl₂ et chauffe à reflux pendant 3 heures, on évapore sous vide, reprend le résidu par du DCM et évapore sous vide ; l'opération est faite 2 fois.
B) Ester éthylique de l'acide 1-[4-[N-méthyl-N-(3-N'N'-diméthylaminopropyl) carbamoyl]-2-isopropylphényl]-5-(2,6-diméthoxyphényl) pyrazole-3-carboxylique.

A une solution de 9 ml de triéthylamine et 9,5 ml de N,N,N'-triméthyl-1,3-propanediamine dans 50 ml de DCM on ajoute, sous azote sec, une solution de chlorure d'acide obtenu à l'étape A dans 220 ml de DCM et agite pendant une nuit. On lave 2 fois à l'eau, extrait 2 fois au DCM les phases aqueuses. Les phases organiques rassemblées sont séchées sur MgSO₄ et évaporées sous vide. Le résidu est agité avec 300 ml d'éther, on filtre un insoluble, décolore le filtrat sur charbon animal et évapore pour obtenir 28,8 g du produit attendu sous forme d'huile.
RMN (DMSO + TFA) : 1 : d : 6H ; 1,3 : t : 3H ; 1,8-2,1 : m : 2H ; 2,65 : qt : 1H ; 2,7-3,05 : m : 9H ; 3,15 : mt : 2H ; 3,5 : mt : 2H ; 3,65 : s : 6H ; 4,35 : qd : 2H ; 6,6 : d : 2H ; 6,85 : s : 1H ; 7,2-7,4 : m : 4H.

Le produit de l'étape B peut aussi être obtenu selon le mode opératoire décrit ci-après :

B') A une solution de 1,32 g de N,N,N'-triméthyl-1,3-propanediamine dans 37,5 ml de soude 3N on ajoute une solution du chlorure d'acide obtenu à l'étape A' dans 37,5 ml de DCM. Après 1 heure d'agitation, on ajoute 25 ml de chloroforme et 25 ml d'eau, décante, on sépare la phase organique sèche sur Na₂SO₄ et évapore sous vide pour obtenir 6,1 g de produit attendu.

### Préparation 3.3

Ester méthylique de l'acide 1-[4-[N-(2-cyanoéthyl)carbamoyl]-2-isopropylphényl]-5-(2,6-diméthoxyphényl)pyrazole-3-carboxylique.

(IIa : R₁ = 4-CONHCH₂CH₂CN ; R₂ = 2-iPr ; R₃ = H ; R₄ = CH₃).

On mélange 0,935 g de 3-aminopropionitrile hémifumarate avec 3,7 ml de NaOH 1,3 N, extrait au DCM, sèche la phase organique sur Na₂SO₄ et évapore sous vide le solvant. On reprend le résidu dans 6 ml de DCM, ajoute 0,96 ml de triéthylamine puis, lentement, une solution de 2,45 g du composé obtenu à l'étape A de la Préparation 3.2 dans 30 ml de DCM. On laisse une nuit sous agitation à TA et sous atmosphère d'azote. On filtre un insoluble, lave le filtrat à l'eau par une solution saturée de NaHCO₃, à l'eau, sèche sur Na₂SO₄ et évapore sous vide le solvant. On obtient 2,24 g du produit attendu, F = 114-116°C (déc.).
RMN : 1 : d: 6H ; 2,7 : mt : 1H ; 2,8 : t : 2H ; 3,5 : q : 2H ; 3,65 : s : 6H ; 3,9 : s : 3H ; 6,7 : d : 2H ; 6,9 : s : 1H ; 7,3-7,4 : m : 2H ; 7,75 : d : 1H ; 7,9 : s : 1H ; 9 : t : 1H.

En faisant réagir l'amine primaire appropriée sur le composé obtenu à la Préparation 3.2 étape A et en opérant selon le mode opératoire décrit pour la préparation 3.2, étape B, on prépare les esters décrits dans le tableau 2 ci-dessous.

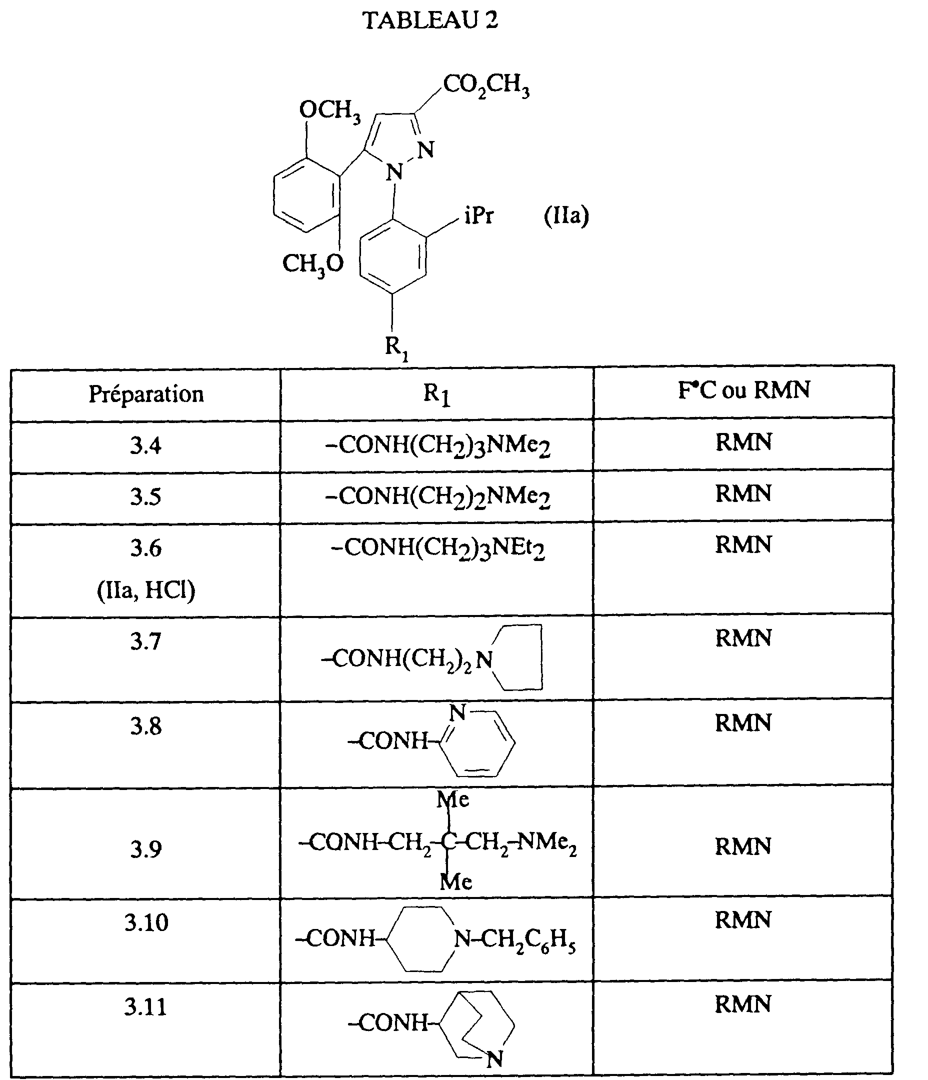

RMN :

Préparation 3.4 : 1 : d : 6H ; 1,65 : mt : 2H ; 2,1 : s : 6H ; 2,25 : t : 2H ; 2,6 : mt : 1H ; 3,25 : m : 2H ; 3,6 : s : 6H ; 3,85 : s : 3H ; 6,6 : d : 2H ; 6,85 : s : 1H ; 7,2-7,3 : m : 2H ; 7,6 : d.d : 1H ; 7,8 : d : 1H ; 8,6 : t : 1H.

Préparation 3.5 : (DMSO + TFA) 1 : d : 6H ; 2,6 : mt : 2H ; 2,8 : s : 6H ; 3,25 : mt : 2H ; 3,6 : m+s : 8H : 3,85 : s : 3H ; 6,6 : d : 2H ; 6,8 : s : 1H ; 7,2-7,4 : m : 2H ; 7,7: d : 1H ; 7,8 : d : 1H.

Préparation 3.6 :(DMSO + TFA) : 0,9 : d : 6H ; 1,15 : t : 6H ; 1,8-2 : m : 2H ; 2,6 : mt : 1H ; 3,1 : mt : 4H ; 3,3 : t : 2H ; 3,55 : m+s : 8H ; 3,8 : s : 3H ; 6,5 : d : 2H ; 6,8 : s : 1H ; 7,15-7,3 : m : 2H ; 7,6 : d.d : 1H ; 7,8 : s.e : 1H.

Préparation 3.7 : 1,05 : d : 6H ; 1,7 : mt : 4H ; 2,45-2,75 : m : 7H ; 3,4 : mt : 2H ; 3,7 : s : 6H ; 3,9 : s : 3H ; 6,65 : d : 2H ; 6,9 : s : 1H ; 7,3-7,4 : m : 2H ; 7,7-7,9 : m : 2H ; 8,6 : t : 1H.

Préparation 3.8 : 1,05 : d : 6H ; 2,7 : mt : 1H ; 3,7 : s : 6H ; 3,9 : s : 3H ; 6,7 : d : 2H ; 6,9 : s : 1H ; 7,2-7,4 : m : 3H ; 7,85-8,5 : m : 5H ; 11 : s : 1H.

Préparation 3.9 : 0,9 : s : 6H ; 1,05 : d : 6H ; 2.25 : s : 2H ; 2,3 : s : 6H ; 2,7 : mt : 1H ; 3,2 : d : 2H ; 3,7 : s : 6H : 3,9 : s : 3H ; 6,65 : d : 2H ; 6,9 : s.e : 1H ; 7,3-7,4 : m : 2H ; 7,6-7,8 : m : 2H ; 8,7 : t : 1H.

Préparation 3.10 : 1 : d : 6H ; 1,4-1,9 : m : 4H ; 2 : t : 2H ; 2,6 : mt : 1H ; 2,8 : d : 2H ; 3,5 : s : 2H ; 3,6 : s : 6H ; 3,6-3,85 : m : 1H ; 3,85 : s : 3H ; 6,6 : d : 2H ; 6,85 : s : 1H;7,1-7,4 : m : 5H ; 7,65 : d : 1H ; 7,8 : s : 1H ; 8,3 : d : 1H.

Préparation 3.11 (DMSO + TFA) : 1,05 : d : 6H ; 1,6-2,3 : m : 5H ; 2,7 : mt : 1H ; 3,2-3,4 : m : 5H ; 3,5-3,8 : m + s : 7H ; 3,9 : s : 3H ; 4,3-4,4 : m : 1H ; 6,65 : d : 2H ; 6,9 : s : 1H ; 7,3-7,4 : mt : 2H ; 7,8 : d : 1H ; 7,9 : s : 1H.

### Préparation 3.12

Ester méthylique de l'acide 5-(2,6-diméthoxyphényl)-1-(2-isopropyl-4-sulfophényl)pyrazole-3-carboxylique.

(II'a : R'₁ = 4-SO₃H ; R₂ = 2-iPr ; R₃ = H ; R₄ = CH₃)

On chauffe pendant 5 heures à reflux un mélange contenant 0,82 g d'acide 3-isopropyl-4-hydrazinobenzènesulfonique obtenu à la Préparation 2.2 et 1,26 g du composé A obtenu à la Préparation 1.1 dans 15 ml d'acide acétique. Après évaporation, on dissout le résidu dans du DCM, lave par HCl normal et décolore la solution sur du charbon activé. On sèche, évapore puis on agite à reflux dans l'éther isopropylique et on filtre à chaud. On obtient 1,28 g du produit attendu.
RMN : 1 : d : 6H ; 2,6 : mt : 1H ; 3,6 : s : 6H ; 3,8 : s : 3H ; 6,6 : d : 2H ; 6,8 : s : 1H ; 7,15 : d : 1H ; 7,3 : t : 1H ; 7,4 : d : 1H ; 7,5 : s : 1H.

### Préparation 3.13

Ester méthylique de l'acide 5-(2,6-diméthoxyphényl)-1-[2-isopropyl-4-(N-méthyl-N-(3-N',N'-diméthylaminopropyl)aminosulfonyl)phényl]pyrazole-3-carboxylique.

(IIa : R₁ = -4-SO₂NMe(CH₂)₃NMe₂; R₂ = 2-iPr ; R₃ = H ; R₄ = CH₃)

A) Ester méthylique de l'acide 5-(2,6-diméthoxyphényl)-1-(4-chlorosulfonyl-2-isopropylphényl)pyrazole-3-carboxylique.

On laisse sous agitation à TA pendant 24 heures 1,08 g du produit obtenu à la Préparation 3.12 et 4 ml de POCl₃ puis on prolonge l'agitation à 70°C pendant 24 heures supplémentaires. Le milieu réactionnel est évaporé 2 fois avec du toluène et l'on obtient 1,6 g du produit attendu.

B) Ester méthylique de l'acide 5-(2,6-diméthoxyphényl)-1-[2-isopropyl-4-(N-méthyl-N-(3-N',N'-diméthylaminopropyl)aminosulfonyl)phényl]pyrazole-3-carboxylique.

A une suspension de 1,6 g du produit obtenu à l'étape précédente dans 10 ml de toluène et 5 ml de DCM, on ajoute 1,8 ml de N,N,N'-triméthyl-1,3-propanediamine puis 2 ml de triéthylamine. On laisse sous agitation 3 heures à TA puis 1 heure et demie à 50°C. Après filtration et évaporation à sec, on extrait à l'éther puis par AcOEt. On lave à l'eau, sèche sur Na₂SO₄ et évapore sous vide. On obtient 1,13 g du produit attendu.

RMN : 0,85 : d : 6H ; 1,45 : mt : 2H ; 2 : s : 6H ; 2,6 : s+mt : 4H ; 2,85 : t : 2H ; 3,5 : s : 6H ; 3,8 : s : 3H ; 6,5 : d : 2H ; 6,8 : s : 1H ; 7,2 : t : 1H ; 7,4 : d : 1H ; 7,5-7,6 : m : 2H.

### Préparation 3.14

Ester méthylique de l'acide 5-(2,6-diméthoxyphényl)-1-(4-carboxy-5,6,7,8-tétrahydronapht-1-yl)pyrazole-3-carboxylique.

(II'a : R'₁ = 4-CO₂H ; R₂, R₃ = -(CH₂)₄- ; R₄ = CH₃)

On agite 2 heures à reflux un mélange de 0,67 d'hydrazine obtenue à la préparation 2.3 et 0,83 g de composé A dans 6 ml d'AcOH. On extrait par DCM, lave à l'eau la phase organique, sèche sur MgSO₄ et évapore sous vide. On chromatographie sur silice en éluant avec le mélange DCM/MeOH (100/2 ; v/v) pour obtenir 0,7 g de produit attendu.

RMN : 1,4-2 : m : 4H ; 2,3-3,1 : m : 4H ; 3,4-4 : m : 9H ; 6,6 : d : 2H ; 6,8-7,6 : m : 4H ; 12,95 : s.e : 1H.

### Préparation 3.15

Ester méthylique de l'acide 5-(2,6-diméthoxyphényl)-1-{4-[N-(3-(N',N'-diméthylaminopropyl)carbamoyl])-5,6,7,8-tétrahydronapht-1-yl}pyrazole-3-carboxylique.

(IIa : R₁ = 4-CONH(CH₂)₃NMe₂ ; R₂, R₃ = -(CH₂)₄- ; R₄ = CH₃).

On chauffe 1 heure et demie à 40°C une solution de 0,7 g de produit obtenu à la Préparation 3.14 dans 6 ml de SOCl₂ et 30 ml de DCM. On évapore sous vide puis on redissout le chlorure d'acide dans 5 ml de DCM, refroidit à 5°C, ajoute 0,225 ml de N,N diméthylpropylènediamine et 0,225 ml de triéthylamine. Après agitation 2 heures à TA, on évapore sous vide, redissout dans le DCM, lave à l'eau, sèche sur MgSO₄ et évapore sous vide pour obtenir 0,79 g de produit attendu.

### Préparation 3.16

Ester méthylique de l'acide 5-(2,6-diméthoxyphényl)-1-(4-sulfo-5,6,7,8-tétrahydronapht-1-yl)pyrazole-3-carboxylique.

(II'a : R'₁ = 4-SO₃H ; R₂, R₃ = -(CH₂)₄- ; R₄ = CH₃).

On chauffe 4 heures et demie à reflux un mélange de 0,5 g d'hydrazine obtenue à la préparation 2.4 et 0,62 g du composé A dans 4 ml d'AcOH. On évapore sous vide, redissout dans DCM, lave 2 fois avec HCI 1N, sèche sur MgSO₄ et évapore pour obtenir 1 g de produit attendu.

RMN : 1,7 : s.e : 4H ; 2,5 : m : 2H ; 3,2 : m : 2H ; 3,7 : s : 6H ; 3,95 : s : 3H ; 6,7 : d : 2H ; 6,8-6,9 : m : 2H ; 7,35 : t : 1H ; 7,55 : d : 1H.

### Préparation 3.17

Ester méthylique de l'acide 5-(2,6-diméthoxyphényl)-1-{4-[N-méthyl-N-(2-N',N'-diméthylaminoéthyl)aminosulfonyl]-5,6,7,8-tétrahydronapht-1-yl}pyrazole-3-carboxylique.

(IIa : R₁ = 4-SO₂NMe(CH₂)₂NMe₂ ; R₂, R₃ = (CH₂)₄ ; R₄ = CH₃).

On agite 5 heures à TA puis 3 heures et demie à 70°C un mélange contenant 1 g d'acide obtenu à la Préparation 3.16 et 3 ml de POCl₃. On évapore sous vide, puis ajoute du toluène et évapore sous vide (2 fois). La solution du chlorure de sulfonyle ainsi obtenu dans 10 ml DCM est ajoutée à une solution de 1,5 ml de N,N,N'-triméthyléthylènediamine et 1,5 ml de triéthylamine dans 10 ml DCM à 5°C. On laisse sous agitation 4 jours à 10°C, filtre et évapore sous vide. Après redissolution dans du DCM, lavage à l'eau, extraction de la phase aqueuse par DCM, on sèche sur MgSO₄ et évapore sous vide pour obtenir 1,07 g de sulfonamide attendu, F = 90°C.

RMN : 1,6-1,8 : m : 4H ; 2,1 : s : 6H ; 2,35 : t : 2H ; 2,4-2,6 : m : 2H ; 2,9 : s : 3H ; 3,1 : mt : 2H ; 3,2 : t : 2H ; 3,6 : s : 6H ; 3,9 : s : 3H ; 6,7 : d : 2H ; 6,9 : s : 1H ; 7,1 : d : 1H ; 7,4 : t : 1H ; 7.7 : d : 1H.

### Préparation 3.18

Ester méthylique de l'acide 5-(2,6-diméthoxyphényl)-1-(4-carbamoyl-2-méthylphényl)pyrazole-3-carboxylique.

(IIa : R₁ = 4-CONH₂ ; R₂ = 2-CH₃ ; R₃ = H ; R₄ = CH₃).

On chauffe à reflux une suspension de 0,39 g de l'hydrazine obtenue à la Préparation 2.5 et 450 mg du composé A dans 5 ml d'AcOH pendant 8 heures. On ajoute 100 ml d'eau au milieu réactionnel ; le précipité formé est filtré puis placé dans 10 ml d'éther isopropylique et chauffé à reflux pendant 30 minutes, et filtré. On obtient 300 mg du produit attendu, F = 219°C. Par filtration de la phase aqueuse 24 heures après, on obtient un second jet de 70 mg de produit attendu sous forme d'aiguilles.

RMN : 2,05 : s : 3H ; 3,5 : s : 6H ; 3,8 : s : 3H ; 6,6 : d : 2H ; 6,8 : s : 1H ; 7 : d : 1H ; 7,2 : t : 1H ; 7,4 : s.e : 1H ; 7,6 : d : 1H ; 7,7 : s : 1H ; 7,9 : s.e : 1H.

### Préparation 3.19

Ester méthylique de l'acide 5-(2,6-diméthoxyphényl)-1-(4-carboxy-2,3-diméthylphényl)pyrazole-3-carboxylique.

(II'a : R'₁ = 4-CO₂H ; R₂ = 2-CH₃ ; R₃ = 3-CH₃ ; R₄ = CH₃.

On chauffe sous agitation 3 heures à reflux un mélange de 4,5 g du produit de la Préparation 2.6 et 12 g de composé A dans 50 ml d'AcOH. On précipite en versant sur 300 ml d'eau glacée, filtre, lave avec 50 ml d'eau. Après agitation dans 50 ml d'éther, filtration, séchage sous vide sur P₂O₅ on obtient 5 g de produit attendu, F = 240°C.

### Préparation 3.20

Ester méthylique de l'acide 5-(2,6-diméthoxyphényl)-1-{2,3-diméthyl-4-[N-(2-N',N'-diméthylaminoéthyl)carbamoyl]phényl}pyrazole-3-carboxylique.

(IIa : R₁ = 4-CONH(CH₂)₂NMe₂ ; R₂ = 2-CH₃ ; R₃ = 3-CH₃ ; R₄ = CH₃).

Après chauffage 1 heure et demie à 40°C d'une solution de 2 g du produit de la Préparation 3.19 dans 10 ml de SOCl₂ et 40 ml de DCM, on évapore sous vide, redissout le chlorure d'acide dans 10 ml de DCM et verse sur une solution de 0,54 ml de N,N diméthylaminoéthylènediamine dans 10 ml de DCM. On ajoute 0,68 ml de triéthylamine et agite 2 heures à TA. Après évaporation sous vide, extraction avec 100 ml de DCM, lavage à l'eau, séchage sur MgSO₄ et évaporation sous vide, on obtient 1,8 g de produit attendu.

RMN : 1,9 : s : 3H ; 2,2 : s+s : 9H ; 2,4 : t : 2H ; 3,4 : m : 2H ; 3,6 : s : 6H ; 3,8 : s : 3H ; 6,6 : d : 2H ; 6,8 : s : 1H ; 6,9-7,1 : d.d : 2H ; 7,3 : t : 1H ; 8,2 : t : 1H.

### Préparation 3.21

Ester méthylique de l'acide 5-(2,6-diméthoxyphényl)-1-(4-carboxy-2-méthoxyphényl)pyrazole-3-carboxylique.

(II'a : R'₁ = 4-CO₂H ; R₂ = 2-OCH₃ ; R₃ = H ; R₄ = CH₃).

On laisse sous agitation 6 heures à reflux un mélange de 4,8 g du produit de la Préparation 2.7 et 5,6 g du composé A dans 60 ml d'AcOH. Après précipitation sur 300 ml d'eau glacée, agitation 30 minutes, filtration, lavage à l'eau puis au pentane et séchage, on obtient 6 g de produit attendu, F = 210°C.

### Préparation 3.22

Ester méthylique de l'acide 5-(2,6-diméthoxyphényl)-1-{4-[N-méthyl-N-(2-N',N'-diéthylaminoéthyl)carbamoyl]-2-méthoxyphényl}pyrazole-3-carboxylique.

(IIa : R₁ = 4-CONMe(CH₂)₂NEt₂ ; R₂ = 2-OCH₃ ; R₃ = H ; R₄ = CH₃).

On chauffe 3 heures et demie à reflux une solution de 2,5 g du produit de la Préparation 3.21 dans 30 ml de DCM et 5 ml de SOCl₂. Après évaporation sous vide suivie de 2 évaporations azéotropiques avec 20 ml deDCM, on redissout le chlorure d'acide formé dans 40 ml de DCM, ajoute sur une solution de 1,1 ml N,N-diéthyl, N'-méthyléthylènediamine et 1 ml de triéthylamine dans 40 ml de DCM et laisse sous agitation 15 heures à TA. Après évaporation sous vide, on chromatographie sur silice, en éluant avec un gradient de solvants allant du mélange DCM/MeOH (90/10 ; v/v) au mélange DCM/MeOH/H₂O (80/20/0,7 ; v/v/v) pour obtenir 1,73 g du produit attendu.

RMN : 0,7-1,1 : mt : 6H ; 2,2-3,7 : mt : 20H ; 3;85 : s : 3H ; 6,55 : d : 2H ; 6,8 : s : 1H ; 6,95 : mt : 2H ; 7,3 : mt : 2H.

### Préparation 3.23

Ester éthylique de l'acide 1-(4-carboxy-2-chlorophényl)-5-(2,6-diméthoxyphényl)pyrazole-3-carboxylique.

(II'a : R'₁ = 4-CO₂H ; R₂ = 2-Cl ; R₃ = H ; R₄ = CH₃)

L'acide 3-chloro-4-hydrazinobenzoïque est décrit dans le brevet US 3 959 309. On chauffe à reflux pendant 6 heures 5,6 g de cet acide et 8,75 g du composé A₁ dans 100 ml d'AcOH. On verse le mélange réactionnel sur 500 ml d'eau glacée, filtre le précipité formé puis on lave ce dernier par de l'eau, du pentane, puis de l'éther isopropylique. On sèche sous vide pour obtenir 2 g du composé attendu.

### Préparation 3.24

Ester éthylique de l'acide 5-(2,6-diméthoxyphényl)-1-{4-[N-méthyl-N-(2-N',N'-diéthylaminoéthyl)carbamoyl]-2-chlorophényl}pyrazole-3-carboxylique.

(IIa : R₁ = 4-CONMe (CH₂)₂NEt₂ ; R₂ = 2-Cl ; R₃ =H ; R₄ = CH₃).

On chauffe 4 heures à reflux une solution de 2,63 g du produit de la Préparation 3.23 dans 30 ml de DCM et 4,5 ml de SOCl₂. Après évaporation sous vide suivie de 2 évaporations azéotropiques avec 20 ml de DCM, on redissout le chlorure d'acide formé dans 40 ml de DCM puis on verse sur une solution de 1,1 ml de N,N-diéthyl-N'-méthyléthylènediamine et de 1 ml de triéthylamine dans 4 ml de toluène et laisse sous agitation 15 heures à TA. Après évaporation sous vide, extraction du résidu par 100 ml de DCM, 2 lavages à l'eau, séchage sur Na₂SO₄ et évaporation sous vide, on chromatographie sur silice H en éluant avec le mélange DCM/MeOH (90/10 ; v/v) puis DCM/MeOH/H₂O (90/10/0,5 ; v/v/v) pour obtenir 2,6 g de produit attendu.

RMN (DMSO + TFA) : 2,8 : s : 3H ; 3 à 3,7 : m : 17H ; 6,6 : d : 2H ; 6,8 : s : 1H; 7,05 : m : 2H ; 7,3 : m : 2H.

### Préparation 3.25

Ester éthylique de l'acide 5-(2,6-diméthoxyphényl)-1-[4-(N-(2-morpholinoéthyl) carbamoyl)-2-chlorophényl]pyrazole-3-carboxylique.

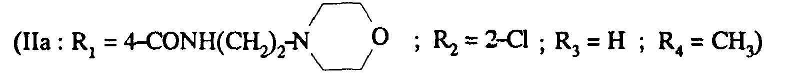

On agite 4 heures à reflux une solution de 2,63 g du produit de la Préparation 3.23 dans 4,5 ml de SOCl₂ et 30 ml de DCM. Après évaporation sous vide suivie de 2 évaporations azéotropiques avec 30 ml de toluène, on redissout le chlorure d'acide formé dans 40 ml de DCM, additionne sur une solution de 0,9 ml de 4-(2-aminoéthyl)-morpholine et 1 ml de triéthylamine dans 4 ml de toluène. Après agitation 15 heures à TA, évaporation sous vide, redissolution du résidu dans 200 ml de DCM, lavage avec 100 ml de solution saturée de NaCl, séchage sur Na₂SO₄ et évaporation sous vide on obtient 3 g de produit attendu.

RMN : 1,3 : t : 3H ; 2,3-2,6 : mt : 6H ; 3,3 : mt : 2H ; 3,5 : mt : 10H ; 4,3 : qd : 2H ; 6,55 : d : 2H ; 6,8 : s : 1H ; 7,1-7,4 : mt : 3H ; 6,75 : d.d : 1H ; 6,9 : d.d : 1H ; 8,6 : t : 1H.

### Préparation 3.26

Ester méthylique de l'acide 5-(2,6-diméthoxyphényl)-1-(3-chloro-4-cyanophényl) pyrazole-3-carboxylique.

(II'a : R₁ = 4-CN ; R₂ = 3-Cl ; R₃ = H ; R₄ = CH₃).

On chauffe au bain marie pendant 2 heures un mélange contenant 4,2 g du composé obtenu à la Préparation 2.8 et 6 g du composé A dans 10 ml d'AcOH. On verse sur de l'eau glacée, le précipité formé est filtré puis séché pour obtenir 3,78 g du produit attendu.

### Préparation 3.27

Ester méthylique de l'acide 5-(2,6-diméthoxyphényl)-1-[4-carboxy-2-cyclopropyl phényl]pyrazole-3-carboxylique.

(II'a : R'₁ =4-CO₂H ; R₂ = 2-cyclopropyle ; R₃ = H ; R₄ =CH₃)

On agite pendant 7 heures à reflux un mélange de 1,28 g de composé A et 1 g du produit de la Préparation 2.9 dans 12 ml d'AcOH. On précipite sur 120 ml d'eau glacée, filtre le précipité, lave à l'eau et sèche sous vide sur P₂O₅ pour obtenir 1,27 g de produit attendu.

RMN : 0,5 : m : 2H ; 0,8 : m : 2H ; 1,3 : t : 3H ; 1,5 : m : 1H ; 3,6 : s : 6H ; 4,3 : qd : 2H ; 6,6 : d : 2H ; 6,9 : s : 1H ; 7,3 : m : 3H ; 7,7 : d : 1H ; 13 : se : 1H.

### Préparation 3.28

Ester méthylique de l'acide 5-(2,6-diméthoxyphényl)-1-{4-[N-méthyl-N-(2-N',N'-diéthylaminoéthyl)carbamoyl]-2-cyclopropylphényl}pyrazole-3-carboxylique.

(IIa : R₁ = 4-CON(Me)(CH₂)₂NEt₂ ; R₂ = 2-cyclopropyle ; R₃ =H ; R₄ = CH₃).

On chauffe 5 heures à 100°C une solution de 1,27 g du produit de la Préparation 3.27 dans 28 ml de toluène et 2 ml de SOCl₂. Après évaporation sous vide suivie de 2 évaporations azéotropiques avec 30 ml de toluène, on redissout le chlorure d'acide obtenu dans 19 ml de DCM, verse lentement sur une solution de 0,53 ml de N,N-diéthyl-N'-méthyl-éthylènediamine et de 0,61 ml de triéthylamine dans 1,9 ml de toluène. Après agitation 12 heures à TA, évaporation sous vide, extraction avec 100 ml de DCM, lavage à l'eau, séchage sur Na₂SO₄ et évaporation sous vide, on obtient 1,29 g de produit attendu.

RMN : 0,4-1 : m : 10H ; 1,2 : t : 3H ; 1,4 : mt : 1H ; 2,1-3 : m : 9H ; 3,5 : m : 8H ; 4,3 : qd : 2H ; 6,5 : d : 2H ; 6,6 : s : 1H ; 6,8 : s : 1H ; 6,9-7,2 : mt : 3H.

### Préparation 3.29

Ester méthylique de l'acide 5-(2,6-diméthoxyphényl)-1-[3-carboxy-4-chlorophényl] pyrazole-3-carboxylique.

(II'a : R'₁ = 3-CO₂H ; R₂ = 4-Cl ; R₃ = H ; R₄ = CH₃)

On chauffe sous agitation 5 heures à reflux un mélange de 4 g de produit de la Préparation 2.10 et 4,6 g du composé A dans 60 ml de AcOH. On verse sur 100 ml d'eau glacée, filtre, lave le précipité avec 5 ml d'eau puis 20 ml d'éther. On sèche sous vide pour obtenir 3 g de produit attendu, F = 206°C.

RMN : 3,55 : s : 6H ; 3,85 : s : 3H ; 6,7 d : 2H ; 6,9 : s : 1H ; 7,35 : d.d : 1H ; 7,4 : t : 1H ; 7,55 : d : 1H ; 7,7 : d : 1H.

### Préparation 3.30

Ester méthylique de l'acide 5-(2,6-diméthoxyphényl)-1-{3-[N-méthyl-N-(2-N',N'-diméthylaminoéthyl)carbamoyl]-4-chlorophényl}pyrazole-3-carboxylique.

(IIa : R₁ = 3-CON(Me)(CH₂)₂NMe₂; R₂ = 4-Cl ; R₃ = H ; R₄ = CH₃).

On chauffe sous agitation 3 heures et demie à 60°C une solution de 1,5 g du produit de la préparation 3.29 dans 20 ml de DCM et 2,6 ml de SOCl₂. Après évaporation sous vide, on redissout le chlorure d'acide dans 10 ml de DCM et verse sur une solution de 0,5 ml de N,N,N'-triméthyléthylènediamine et de 0,6 ml de triéthylamine dans 2,5 ml de toluène, puis on laisse sous agitation 15 heures à TA. Après évaporation sous vide, dissolution du résidu dans 100 ml de DCM, lavage avec 100 ml d'eau, séchage sur Na₂SO₄ et évaporation sous vide, on obtient 0,8 g de produit attendu.

### Préparation 3.31

Ester méthylique de l'acide 5-(2,6-diméthoxyphényl)-1-[5-carboxy-2-méthyl phényl]pyrazole-3-carboxylique.

(II'a : R'₁ = 5-CO₂H ; R₂ = 2-CH₃ ; R₃ = H ; R₄ = CH₃).

On chauffe à reflux sous agitation 1 heure et demie un mélange de 6,15 g du produit de la préparation 2.11 et de 7,76 g du composé A dans 100 ml d'AcOH. Après concentration sous vide à 5 ml, précipitation sur 20 ml d'eau glacée, filtration, lavage du précipité avec 5 ml d'eau, séchage sur P₂O₅ sous vide à 80°C on obtient 9,55 g de produit attendu, F = 189°C.

RMN : 1,20 : t : 3H ; 3,55 : s : 6H ; 4,26 : qd : 2H ; 6,58 : d : 2H ; 6,80 : s : 1H ; 7,20 : t : 1H ; 7,60 : d : 1H ; 7,70 : d : 1H ; 7,80 : d.d : 1H.

### Préparation 3.32

Ester méthylique de l'acide 5-(2,6-diméthoxyphényl)-1-{5-[N-méthyl-N-(3-N',N'-diméthylaminopropyl)carbamoyl]-2-méthylphényl}pyrazole-3-carboxylique.

(IIa : R₁ = 5-CON(Me)(CH₂)₃NMe₂ ; R₂ = 2-CH₃ ; R₃ = H ; R₄ = CH₃)

On laisse sous agitation 5 heures à TA une solution de 2 g du produit de la Préparation 3.31 dans 10 ml de SOCl₂. Après évaporation sous vide suivie de 3 distillations azéotropiques avec 30 ml de DCM, on redissout le chlorure d'acide formé dans 25 ml de DCM, verse sur une solution de 0,81 ml de N,N,N'-triméthyl-1,3-propanediamine et de 0,77 ml de triéthylamine dans 5 ml de DCM et laisse sous agitation 2 heures à TA. Après 2 lavages avec 20 ml d'eau, 2 extractions des phases aqueuses avec 50 ml de DCM, les phases DCM sont lavées 2 fois avec 20 ml de NaHCO₃ 5 %, puis avec 20 ml d'eau, puis séchées sur MgSO₄ et évaporées sous vide pour obtenir 2,3 g de produit attendu (huile).

RMN :1,9 : m : 2H ; 2,1 : s : 3H ; 2,6 : s : 3H ; 2,8 : s : 6H ; 2,9-3,2 : m : 2H ; 3,45 : m : 2H ; 3,6 : s : 6H ; 3,8 : s : 3H ; 6,6 : d : 2H ; 6,85 : s : 1H ; 7,05 : s.e : 1H ; 7,2-7,4 : m : 3H.

En suivant les modes opératoires ci-dessus, on prépare les esters de formule IIa décrits dans le tableau 3 ci-après, soit à partir d'un ester de formule II'a substitué par R'₁, soit par action de l'hydrazine appropriée sur le composé A ou le composé A₁.

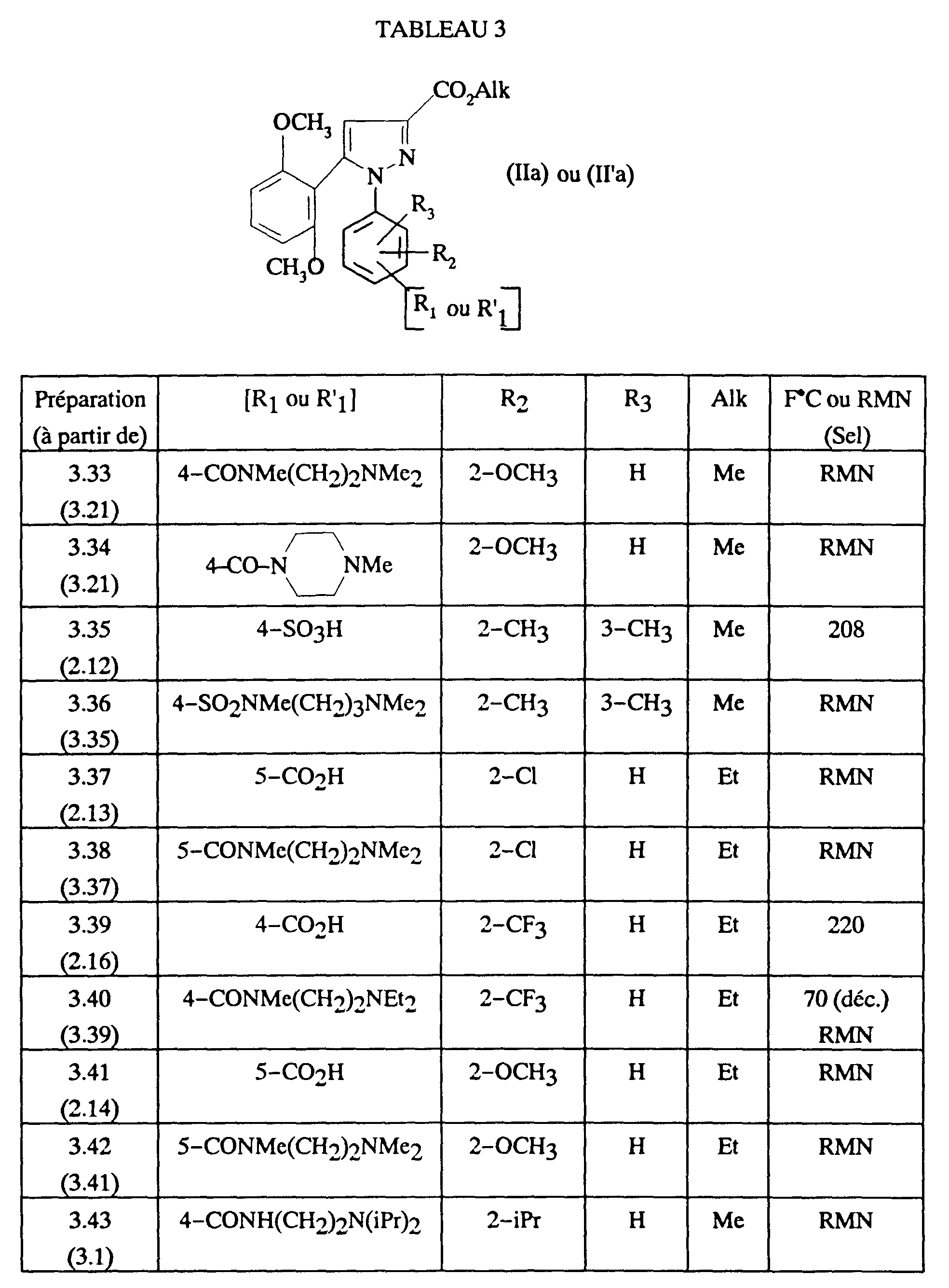

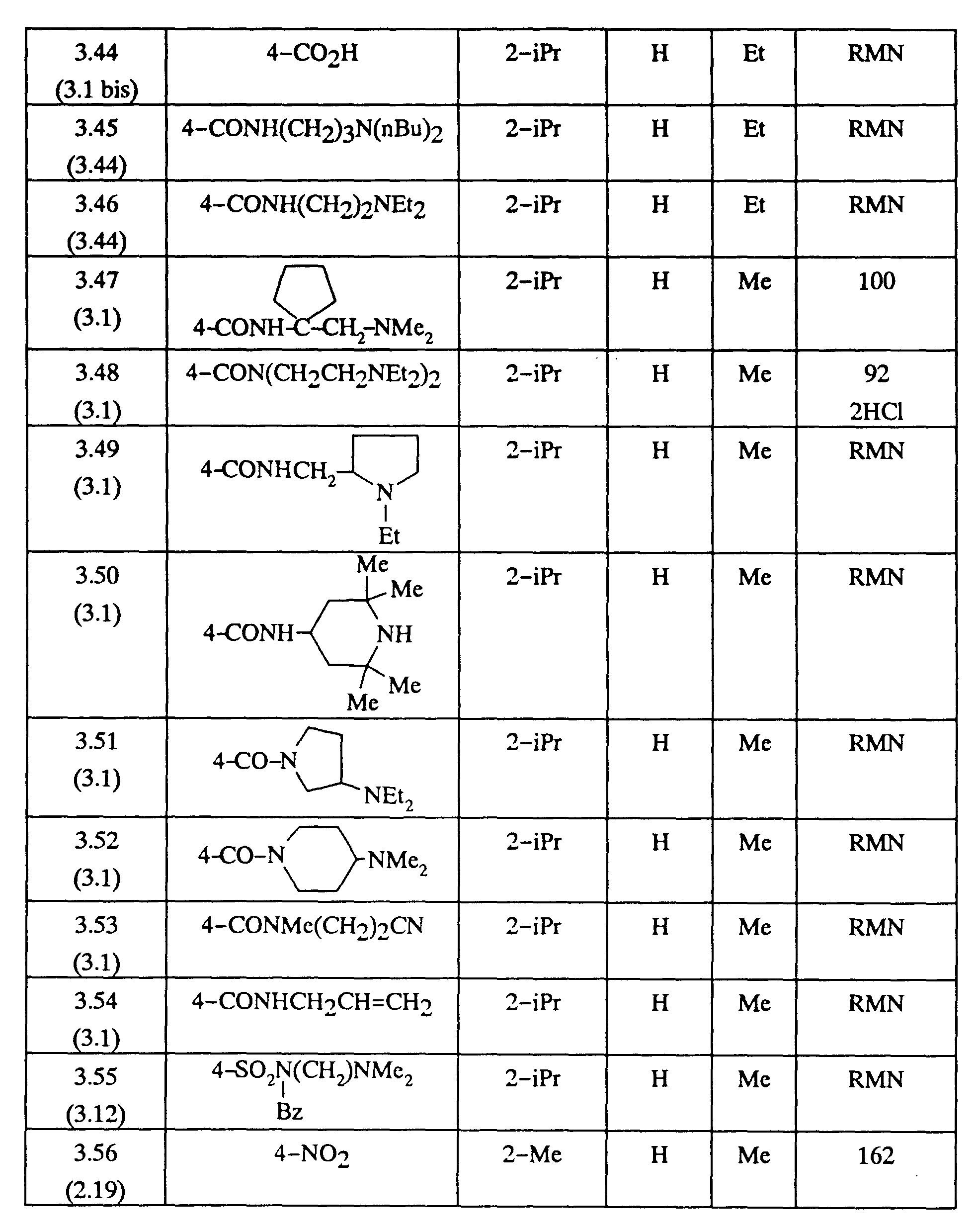

### RMN:

Préparation 3.33 :2 à 3,8 : mt : 1H ; 3,9 : s : 3H ; 6,6 : d : 2H ; 6,85 : s : 1H ; 6,9 à 7,4 : m : 4H.

Préparation 3.36 (DMSO + TFA) :1,9 : m+s : 5H ; 2,5 : s : 3H ; 2,8 : s : 9H ; 3-3,5 : m : 2H ; 3,25 : t : 2H ; 3,6 : s : 6H ; 3,85 : s : 3H ; 6,6 : d : 2H ; 6,9 : s : 1H ; 7,05-7,15 : m : 1H ; 7,3 : t : 1H ; 7,6 : d : 1H.

Préparation 3.37 : 1,2 : t : 3H ; 3,5 : s : 6H ; 4,3 : qd : 2H ; 6,5 : d : 2H ; 6,8 : s : 1H ; 7,2 : t : 1H ; 7,6 : d : 1H ; 7,7 : s : 1H ; 7,85 : d : 1H.

Préparation 3.38 : 1,4 : t : 3H ; 1,8 à 3,3 : m : 13H ; 3,65 : s : 6H ; 4,4 : qd : 2H ; 6,6 : d : 2H ; 6,95 : s : 1H ; 7,2 à 7,6 : m : 4H ; 7,7 : d : 1H.

Préparation 3.40 : 0,6 à 1 : m : 6H ; 1,3 : t : 3H ; 2,2 : m : 2H ; 2,4 à 3,4 : m : 7H ; 3,6 : s : 6H ; 4,1 : m : 2H ; 4,3 : qd : 2H ; 6,6 : d : 2H ; 6,8 : s : 1H ; 7,15 : m : 2H; 7,6 : d : 1H ; 7,8 : s.e : 1H.

Préparation 3.41 : 1,4 : t : 3H ; 3,6 : s : 9H ; 4,4 : qd : 2H ; 6,62 : d : 2H ; 6,81 : s : 1H ; 7,2 : d : 1H ; 7,36 : t : 1H ; 7,8 : d : 1H ; 8,0 : d.d : 1H ; 12,6 : s.e : 1H.

Préparation 3.42 : 1,4 : t : 3H ; 1,8 à 2,4 : s.e : 8H ; 2,8 : s.e : 3H ; 3,2 à 3,8 : s.e : 2H ; 3,6 : s : 9H ; 4,4 : qd : 2H ; 6,6 : d : 2H ; 6,8 : s : 1H ; 7,0 à 7,5 : m : 4H.

Préparation 3.43 : 1,0-1,1 : m : 18H ; 2,4-2,75 : 2mt : 3H ; 2,9-3,1 : mt : 2H ; 3,1-3,35 : mt : 2H ; 3,65 : s : 6H ; 3,9 : s : 3H ; 6,65 : d : 2H ; 6,9 : s : 1H ; 7,3-7,4 : mt : 2H ; 7,7 : d : 1H ; 7,8 : s: 1H ; 8,55 : t: 1H.

Préparation 3.45 : 0,75 : t : 6H ; 0,9 : d : 6H ; 1,05-1,15 : m : 8H ; 1,5 : t : 2H ; 2,5-2,4 : m : 6H ; 2,55 : sp : 1H ; 3,15 : mt : 2H ; 3,55 : s : 6H ; 4,2 : s : 3H ; 6,5 : d : 2H ; 6,72 : s : 1H ; 7,1-7,25 : m : 2H ; 7,55 : dd : 1H ; 7,65 : d : 1H ; 8,45 : t : 1H.

Préparation 3.46 : 0,8-1,1 : m : 12H ; 1,25 : t : 3H ; 2,4-2,8 : m : 6H ; 3,1-3,4 : m : 3H ; 3,5 : s : 6H ; 4,2 : qt : 2H ; 6,5 : d : 2H ; 7,2 : m : 2H ; 7,6 : d: 1H ; 7,8 : s.e: 1H; 8,6 : s.e : 1H.

Préparation 3.49 : 0,9-1,15 : 2mt : 9H ; 1,5-1,9 : m : 4H ; 2,1-2,45 : 2mt : 2H ; 2,65 : mt : 1H ; 2,75-3 : mt : 1H ; 3-3,2 : m : 2H ; 3,2-3,55 : m : 2H ; 3,65 : s : 6H ; 3,9 : s : 3H ; 6,65 : d : 2H ; 6,9 : s : 1H ; 7,25-7,4 : mt : 2H ; 7,7 : d : 1H ; 7.85 : s : 1H ; 8,6 : t : 1H.

Préparation 3.50 : 1,05 : d : 6H ; 1,1-1,6 : 2s + m : 14H ; 1,8 : dd : 2H ; 2,65 : mt : 1H ; 3,7 : s : 6H ; 3,9 : s : 3H ; 4,2-4,5 : m : 1H ; 6,65 : d : 2H ; 6,9 : s : 1H ; 7,25-7,4 : mt : 2H ; 7,7 : d : 1H ; 7,85 : s : 1H ; 8,3 : d : 1H.

Préparation 3.51 (DMSO + TFA) : 1,0 : d : 6H ; 1,1-1,4 : m : 6H ; 2,0-2,5 : m : 2H ; 2,7 : 2m : 1H ; 3,0-4,2 : 3m + s : 18H ; 6,7 : d : 2H ; 6,9 : s : 1H ; 7,3-7,55 : m : 4H.

Préparation 3.52 (DMSO + TFA) : 0,95 : d : 6H ; 1,5-1,8 : m : 2H ; 1,8-2,2 : m : 2H ; 2,6 : mt : 1H ; 2,75 : s : 6H ; 2,9-3,8 : 2m + s : 10H ; 3,85 : s : 3H ; 4,5-4,7 : m : 1H ; 6,6 : d : 2H ; 6,85 : s : 1H ; 7,2-7,35 : mt : 4H.

Préparation 3.53 (DMSO + TFA) : 0,98 : d :6H ; 2,6 : mt : 1H ; 2,7-3,8 : m : 13H ; 3,82 : s : 3H ; 6,58 : d : 2H ; 6,85 : s : 1H ; 7,15-7,37 : m : 4H.

Préparation 3.54 : 1,05 : d : 6H ; 2,7 : mt : 1H ; 3,7 : s : 6H ; 3,85-4,0 : s + mt : 5H ; 5,1-5,3 : m : 2H ; 5,8-6,1 : m : 1H ; 6,55 : d : 2H ; 6,9 : s : 1H ; 7,3-7,4 : m : 2H ; 7,75 : d : 1H ; 7,9 : s : 1H ; 8,8 : t : 1H.

Préparation 3.55 : 1,05 : d : 6H ; 2 : s : 6H ; 2,15 : t : 2H ; 2,75 : qt : 1H ; 3,2 : t : 2H ; 3,7 : s : 6H ; 3,9 : s : 3H ; 4,45 : s : 2H ; 6,7 : d : 2H ; 7 : s : 1H ; 7,3-7,5 : m : 6H ; 7,55 : d : 1H ; 7,7-7,9 : m : 2H.

### Préparation 3.57

Ester méthylique de l'acide 5-(2,6-diméthoxyphényl)-1-[4-[N-méthyl-N-[3-[N'-méthyl-N'-(*tert*-butoxycarbonyl)amino]propyl]carbamoyl]-2-isopropylphényl]pyrazole-3-carboxylique.

(IIa : R₁ = 4-CONMe(CH₂)₃N(Me)COOt-Bu ; R₂ = 2-iPr ; R₃ = H ; R₄ = CH₃)

A) Ester *tert*-butylique de l'acide N-méthyl-N-(3-méthylaminopropyl) carbamique.

On refroidit à 0°C une solution de 4,19 g de N,N'-diméthyl-1,3-propanediamine dans 80 ml de THF, ajoute une solution de 2,68 g de di-*tert*-butyl dicarbonate dans 25 ml de THF et laisse 72 heures sous agitation à TA. On filtre un insoluble et concentre sous vide le filtrat. On extrait le résidu au DCM, lave trois fois la phase organique à l'eau, sèche sur MgSO₄ et évapore sous vide le solvant. On obtient 1,6 g du produit attendu sous forme d'huile jaune.

B) Ester méthylique de l'acide 5-(2,6-diméthoxyphényl)-1-[4-[N-méthyl-N-[3-[N'-méthyl-N'-(*tert*-butoxycarbonyl)amino]propyl]carbamoyl]-2-isopropylphényl]pyrazole-3-carboxylique.

A une solution de 1,31 g du composé obtenu à l'étape précédente et 0,9 ml de triéthylamine dans 4 ml de DCM on ajoute à TA et sous atmosphère d'azote une solution de 2,6 g du composé obtenu à l'étape A de la Préparation 3.2 et laisse une nuit sous agitation à TA. On lave deux fois le mélange réactionnel à l'eau, sèche la phase organique sur MgSO₄ et évapore sous vide le solvant. On chromatographie le résidu sur silice en éluant par le mélange toluène/AcOEt de (65/35 ; v/v) à (60/40 ; v/v). On obtient 2,85 g du produit attendu.

RMN (DMSO + TFA) : 1,0 : d : 6H ; 1,4 : d : 9H ; 1,6-1,9 : m : 2H ; 2,7 : mt : 1H ; 2,7-3,55 : d + se + m : 10H ; 3,65 : s : 6H ; 3,9 : s : 3H ; 6,65 : d : 2H ; 6,9 : s : 1H ; 7,3-7,45 : m : 4H.

### Préparation 3.58

Ester éthylique de l'acide 5-(2,6-diméthoxyphényl)-1-[4-(4-méthylphénylsulfonylamino)-2-isopropylphényl]pyrazole-3-carboxylique.

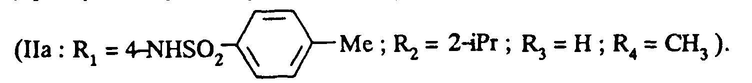

On chauffe 1 heure à 70°C un mélange de 3,44 g du composé obtenu à la Préparation 2.18 et 2,6 g du composé A₁ dans 50 ml d'acide acétique. On verse le mélange réactionnel dans de l'eau, extrait à l'AcOEt, sèche la phase organique sur Na₂SO₄ et évapore sous vide le solvant. On chromatographie le résidu sur silice H en éluant par le mélange DCM/MeOH (100/0,5 ; v/v). On obtient 1,26 g du produit attendu.

RMN : 0,7 : d : 6H ; 1,22 : t : 3H ; 2,2-2,5 : m : 4H ; 3,45 : s : 6H ; 4,2 : t : 2H ; 6,46 : d : 2H ; 6,69 : s : 1H ; 6,75-6,9 : m : 2H ; 7,0 : d : 1H ; 7,15-7,3 : m : 3H ; 7,5 : d : 2H ; 10,2 : s : 1H.

### Préparation 3.59

Ester éthylique de l'acide 5-(2,6-diméthoxyphényl)-1-[4-[4-méthylphényl sulfonyl-N-(3-diéthylaminopropyl)amino]-2-isopropylphényl]pyrazole-3-carboxylique.

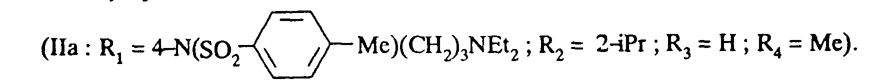

On chauffe à 80°C pendant 2 heures un mélange de 0,65 g du composé obtenu à la Préparation 3.58, 0,338 g de (3-chloropropyl)diéthylamine et 0,65 g de K₂CO₃ dans 5 ml de DMF. On verse le mélange réactionnel dans l'eau, extrait à l'AcOEt, sèche sur Na₂SO₄ et évapore sous vide le solvant. On chromatographie le résidu sur silice H en éluant par le mélange DCM/McOH (100/5 ; v/v). On obtient 0,57 g du produit attendu.

RMN : 0,8 : se : 6H ; 0,95 : t : 6H ; 1,4 : t : 3H ; 1,5 : qt : 2H ; 2,3-2,65 : m : 10H ; 3,5-3,8 : m : 8H ; 4,35 : qd : 2H ; 6,7 : d : 2H ; 6,75 : d : 1H ; 6,9 : s : 1H ; 7,05 : dd : 1H ; 7,2-7,6 : m : 6H.

### Préparation 3.60

Ester éthylique de l'acide 5-[2-(cyclopropylméthyloxy)-6-méthoxyphényl]-1-(4-carboxy-2-isopropylphényl)pyrazole-3-carboxylique.

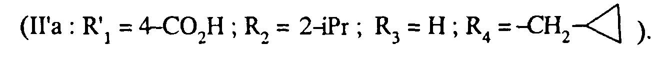

On chauffe à 80°C pendant 8 heures un mélange de 5,26 g du composé obtenu à la Préparation 1.2 et 3,9 g du composé obtenu à la Préparation 2.1 dans 50 ml d'acide acétique. On verse le mélange réactionnel sur un mélange eau/glace, essore le précipité formé et le lave à l'eau puis au pentane. On reprend le précipité au toluène et évapore sous vide le solvant. On obtient 5,2 g du produit attendu.

### Préparation 3.61

Ester éthylique de l'acide 5-[2-(cyclopropylméthyloxy)-6-méthoxyphényl]-1-[4-[N-méthyl-N-(3-diméthylaminopropyl)carbamoyl]-2-isopropylphényl]pyrazole-3-carboxylique.

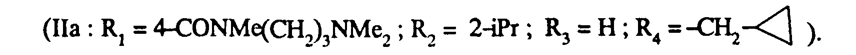

On prépare ce composé selon les modes opératoires décrits aux étapes A et B de la Préparation 3.2 à partir de 5,2 g du composé obtenu à la Préparation 3.60 et 2,4 ml de SOCl₂ puis 1,39 g de N,N,N'-triméthyl-1,3-propanediamine et 1,5 ml de triéthylamine dans 10 ml de toluène. On purifie par chromatographie sur silice en éluant au DCM puis par le mélange DCM/MeOH (88/2 ; v/v). On obtient 3,8 g du produit attendu.

### Préparation 3.62

Ester méthylique de l'acide 5-(2,6-diméthoxyphényl)-1-[4-(4-méthylphényl sulfonylamino)-2-isobutylphényl]pyrazole-3-carboxylique.

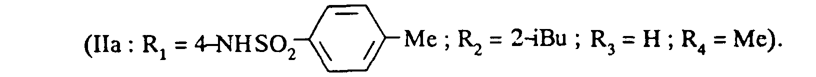

On chauffe 45 minutes à reflux un mélange de 1,9 g du composé obtenu à la Préparation 2.20 et 1,6 g du composé A dans 30 ml d'acide acétique. Après refroidissement à TA, on verse le mélange réactionnel dans l'eau, essore le précipité formé et le sèche. On obtient 1 g du produit attendu après recristallisation dans le propan-2-ol, F = 224°C.

RMN : 0,55 : d : 6H ; 1,5 : mt : 1H ; 1,9 : d : 2H ; 2,3 : s : 3H ; 3,45 : s : 6H ; 3,8 : s : 3H ; 6,5 : d : 2H ; 6,75 : s : 1H ; 6,8 : d : 1H ; 6,9 : dd : 1H ; 7,05 : d : 1H ; 7,15-7,7 : m : 5H ; 10,25 : s : 1H.

### Préparation 3.63

Ester méthylique de l'acide 5-(2,6-diméthoxyphényl)-1-[4-(4-méthylphényl sulfonylamino)-2-cyclopentylphényl]pyrazole-3-carboxylique.

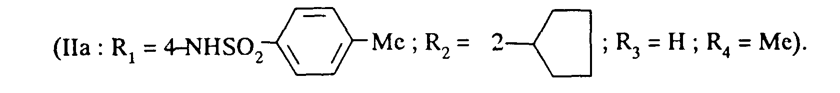

On chauffe 1 heure à 80°C un mélange de 2,42 g du composé obtenu à la Préparation 2.21 et 2,92 g du composé A dans 50 ml d'acide acétique. On verse le mélange réactionnel dans l'eau, extrait à l'AcOEt, sèche la phase organique sur Na₂SO₄ et évapore sous vide le solvant. On chromatographie le résidu sur silice H en éluant par le mélange DCM/McOH (100/1 ; v/v). On obtient 0,95 g du produit attendu après trituration dans l'éther, F = 200-230°C.

RMN : 1,0-1,8 : m : 8H ; 2,37 : s : 3H ; 3,1-3,7 : m : 7H ; 3,82 : s : 3H ; 6,55 : d : 2H ; 6,79 : s : 1H ; 6,85-7,0 : m : 2H ; 7,05 : d : 1H ; 7,21-7,42 : m : 3H ; 7,58 : d : 2H ; 10,3 : s : 1H.

### Préparation 3.64

Ester éthylique de l'acide 5-(2,6-diméthoxyphényl)-1-(4-carboxy-3-isopropyl phényl)pyrazole-3-carboxylique.

(II'a : R'₁ = 4-CO₂H ; R₂ = 3-iPr ; R₃ = H ; R₄ = Me).

On chauffe 5 heures à reflux un mélange de 0,36 g du composé obtenu à la Préparation 2.22 et 0,48 g du composé A₁ dans 10 ml d'acide acétique. On verse le mélange réactionnel sur 160 ml d'eau glacée, essore le précipité formé, le lave à l'eau et le sèche. On obtient 0,52 g du produit attendu, F = 180°C (déc).

### Préparation 3.65

Ester éthylique de l'acide 1-[4-[N-(2-diéthylaminoéthyl)carbamoyl]-3-isopropylphényl]-5-(2,6-diméthoxyphényl)pyrazole-3-carboxylique.

(IIa : R₁ = 4-CONH(CH₂)₂NEt₂ ; R₂ = 3-iPr ; R₃ = H ; R₄ = Me).

On prépare ce composé selon le mode opératoire décrit aux étapes A et B de la Préparation 3.2 à partir de 0,5 g du composé obtenu à la Préparation 3.64 et 5 ml de SOCl₂ dans 20 ml de chloroforme puis 0,2 ml de N,N-diéthyléthylènediamine et 0,8 ml de triéthylamine dans 20 ml de chloroforme. On obtient 0,37 g du produit attendu après cristallisation dans AcOEt, F = 130°C (déc.)

### Préparation 3.66

Ester méthylique de l'acide 5-(2,6-diméthoxyphényl)-1-(4-nitro-5,6,7,8-tétrahydronapht-1-yl)pyrazole-3-carboxylique.

(II'a : R'₁ = 4-NO₂ ; R₂,R₃ = -(CH₂)₄- ; R₄ = Me).

On chauffe 2 heures à reflux un mélange de 6,3 g du composé obtenu à la Préparation 2.23 et 7,45 g du composé A dans 150 ml d'acide acétique. Après refroidissement à TA, on ajoute 100 ml d'eau et 30 ml de MeOH et essore le produit cristallisé. On obtient 4,6 g du produit attendu, F = 212°C.

RMN : 1,7 : mt : 4H ; 2,55 : mt : 2H ; 2,82 : mt : 2H ; 3,65 : s : 6H ; 3,85 : s : 3H ; 6,65 : d : 2H ; 6,9 : s: 1H ; 7,02 ; d: 1H ; 7,33 : t : 1H ; 7,67 : d: 1H.

### Préparation 3.67

Ester méthylique de l'acide 5-(2,6-diméthoxyphényl)-1-(5-(3-(diéthylamino) propanoylamino)-2-isopropylphényl)pyrazole-3-carboxylique.

(IIa : R₁ = 5-NHCO(CH₂)₂NEt₂ ; R₂ = 2-iPr ; R₃ = H ; R₄ = CH₃).

Ce composé est obtenu à partir de l'hydrazine de la Préparation 2.24.

RMN (DMSO + TFA) : 0,65-1,35 : m : 12H ; 2,5 : qt : 1H ; 2,8 : t : 2H ; 3,15 : qd : 4H ; 3,36 : t : 2H ; 3,57 : s : 6H ; 3,8 : s : 3H ; 6,5 : d : 2H ; 6.78 : s : 1H ; 7,1-7,4 : m : 3H ; 7,8 : d : 1H.

### PREPARATIONS DES ACIDES II, II'.

### Préparation 4.1

Acide 1-[4-[N-méthyl-N-(3-N',N'-diméthylaminopropyl)carbamoyl]-2-isopropylphényl]-5-(2,6-diméthoxyphényl)pyrazole-3-carboxylique.

(II, R₁ = 4-CONMe(CH₂)₃NMe₂ ; R₂ =2-iPr ; R₃ = H ; R₄ = CH₃)

On mélange 23 g du composé obtenu à la Préparation 3.2 dans 230 ml de dioxane et 6,2 g de potasse dans 6 ml d'eau. On chauffe à reflux pendant 3 heures et demie. Après refroidissement, on évapore, redissout dans le minimum d'eau, on lave trois fois à l'éther puis on acidifie à pH 4 par addition d'HCl concentré ; on évapore la phase aqueuse puis on redissout le résidu dans le minimum d'EtOH et filtre le KCl (2 fois). Après évaporation on obtient 23,93 g du produit attendu sous forme d'une mousse jaune clair, F = 128°C (déc.).

RMN : 0,95 : d : 6H ; 1,95 : mt : 2H ; 2,45-3,3 : m : 12H ; 3,35 - 3,8 : m : 8H ; 6,6 : d : 2H ; 6,8 : s : 1H ; 7-7,5 : m : 4H.

### Préparation 4.1 bis

Sel de potassium del'acide 1-[4-[N-méthyl-N-(3-N',N'-diméthylaminopropyl) carbamoyl]-2-isopropylphényl]-5-(2,6-diméthoxyphényl)pyrazole-3-carboxylique.

A une solution de 26,6 g du produit de la Préparation 3.2 bis dans 133 ml d'éthanol on ajoute une solution de 8,07 g de KOH dans 133 ml d'eau. On agite la solution pendant 8 heures puis on laisse pendant 15 heures sous agitation et on évapore sous vide pour obtenir le sel de potassium attendu.

### Préparation 4.2

Acide 1-[4-[N-(2-cyanoéthyl)carbamoyl]-2-isopropylphényl]-5-(2,6-diméthoxyphényl)pyrazole-3-carboxylique.

(II : R₁ =4-CONHCH₂CH₂CN ; R₂ = 2-iPr ; R₃ = H ; R₄ = CH₃)

A une solution de 3,04 g du composé obtenu à la Préparation 3.3 dans 30 ml de 1,4-dioxane, et quelques gouttes de MeOH, on ajoute une solution de 0,9 g de KOH dans 3 ml d'eau et laisse une nuit sous agitation à TA. On concentre sous vide, reprend le résidu à l'eau, lave la phase aqueuse à l'éther, acidifie à pH = 2 par ajout d'HCl à 10 %, extrait au DCM, sèche la phase organique sur Na₂SO₄ et évapore sous vide le solvant. On obtient 2,93 g du produit attendu, F = 128°C (déc.).

RMN : 1 : d : 6H ; 2,65 : mt : 1H ; 2,8 : t : 2H ; 3,5 : t : 2H ; 3,6 : s : 6H ; 6,6 : d : 2H ; 6,8 : s : 1H ; 7,2-7,4 : m : 2H ; 7,7 : d.d : 1H ; 7,8 : d : 1H.

A partir des esters de formule (IIa) décrits dans le tableau 2 et en opérant selon le mode opératoire décrit à la Préparation 4.1 ou à la préparation 4.2, on obtient les acides de formule (II) décrits dans le tableau 4 ci-dessous.

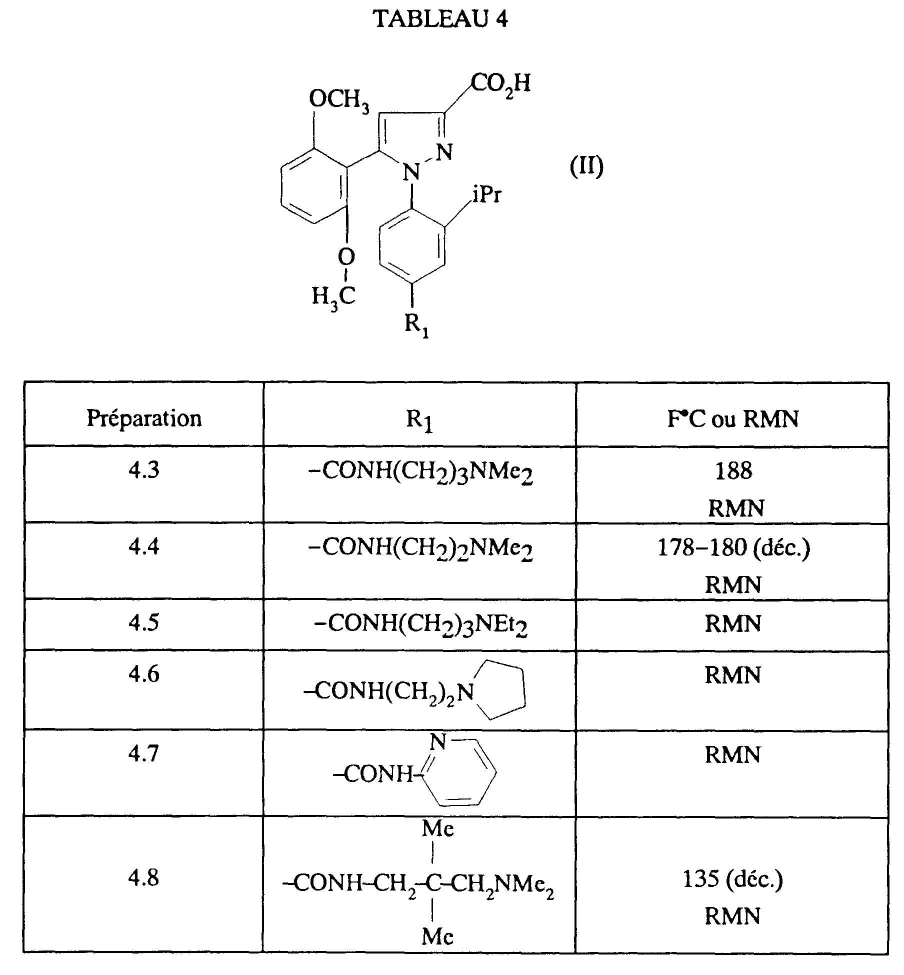

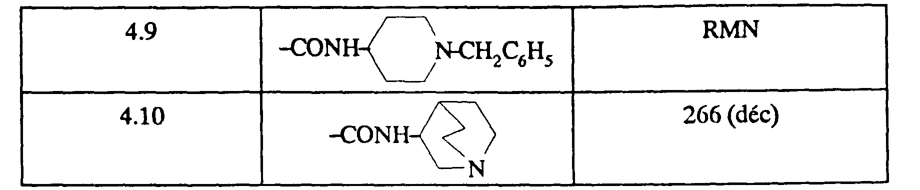

### Préparation 4.3

RMN : 0,9 : d : 6H ; 1,6 : mt : 2H ; 2,15 : s : 6H ; 2,35 : t : 2H ; 2,6 : mt : 1H ; 3,2 : m : 2H ; 3,55 : s : 6H ; 6,5 : d : 2H ; 6,6 : s : 1H ; 7,1-7,2 : m : 2H ; 7,7 : d.d : 1H ; 7,9 : s.e : 1H ; 8,6 : t : 1H.

### Préparation 4.4

RMN (DMSO + TFA) : 1 : d : 6H ; 2,65 : mt : 1H ; 2,85 : s : 6H ; 3,3 : mt : 2H ; 3,6 : mt+s : 8H ; 6,6 : d : 2H ; 6,8 : s : 1H ; 7,25-7,4 : m : 2H ; 7,7 : d.d : 1H ; 7,85 : se : 1H.

### Préparation 4.5

RMN : 0,95 : d : 6H ; 1,15 : t : 6H ; 1,8-2 : m : 2H ; 2,6 : mt : 1H ; 3,1 : mt : 4H ; 3,3 : t : 2H ; 3,6 : m+s : 8H ; 6,5 : d : 2H ; 6,75 : s : 1H ; 7,2 : t : 2H ; 7,6 : d.d : 1H ; 7.75 : d : 1H.

### Préparation 4.6

RMN (DMSO + TFA) : 1 : d : 6H ; 1,8-2,2 : m : 4H ; 2,65 : mt : 1H ; 3 : mt : 2H ; 3,3 : mt : 2H ; 3,6 : se : 10H ; 6,6 : d : 2H : 6,8 : s : 1H ; 7,2-7,4 : m : 2H ; 7,6-7,9 : m : 2H ; 8,9 : t : 1H.

### Préparation 4.7

1,1 : d : 6H ; 2,7 : mt : 1H ; 3,7 : s : 6H ; 6,65 : d : 2H ; 6,9 : s : 1H ; 7,2--7,4 : m : 3H ; 7,8-8,5 m 5H.

### Préparation 4.8

RMN : 1-1,2 : m : 12H ; 2,6-2,9 : m : 9H ; 3,3 : d : 2H ; 3,7 : s : 6H ; 6,6 : d : 2H ; 6,8 : s : 1H ; 7,3-7,4 : m : 2H ; 7,7-7,9 : m : 2H ; 8,8 : t : 1H.

### Préparation 4.9

RMN (DMSO + TFA) : 1 : d : 6H ; 1,8 : mt : 2H ; 2,1 : mt : 2H ; 2,7 : mt : 1H ; 3-3,5 : m : 4H ; 3,6 : s : 6H ; 4 : mt : 1H ; 4,3 : s : 2H ; 6,6 : d : 2H ; 6,8 : s : 1H ; 7,2-7,4 : m : 2H ; 7,4 - 7,6 : m : 5H ; 7,7 : d : 1H ; 7,8 : s : 1H.

### Préparation 4.11

Acide 5-(2,6-diméthoxyphényl)-1-[2-isopropyl-4-(N-méthyl-N-(3-N',N'-diméthylaminopropyl)aminosulfonyl)phényl]pyrazolc-3-carboxylique.

(II : R₁ =4-SO₂NMe(CH₂)₃NMe₂ ; R₂ = 2-iPr ; R₃ =H ; R₄ = CH₃).

On laisse sous agitation pendant 6 heures à TA un mélange contenant 1,1 g de l'ester obtenu à la Préparation 3.13 et 280 mg de potasse dans 10 ml d'eau. On concentre le milieu réactionnel sous vide jusqu'à obtenir 5 ml puis, on agite en présence de 100 ml d'éther et 3 ml d'eau. La phase aqueuse est neutralisée à pH 6 par addition d'HCl 1N ; on filtre puis sèche sur P₂O₅ pour obtenir 860 mg du produit attendu.

RMN : 0,85 : d : 6H ; 1,5 : mt : 2H ; 2,15 : s : 6H ; 2,3 : t : 2H ; 2,6 : s+mt : 4H ; 2,9 : t : 2H ; 3,5 : s : 6H ; 6,5 : d : 2H ; 6,7 : s : 1H ; 7,2 : t : 1H ; 7,4 : d : 1H ; 7,5-7,6 : m : 2H.

### Préparation 4.12

Acide 5-(2,6-diméthoxyphényl)-1-[4-[N-[3-(N',N'-diméthylamino)-propyl] carbamoyl]-5,6,7,8-tétrahydronapht-1-yl]pyrazole-3-carboxylique.

(II : R₁ = 4-CONH(CH₂)₃NMe₂ ; R₂, R₃ = -(CH₂)₄- ; R₄ =CH₃)

On chauffe 3 heures à 40°C un mélange de 0,98 g du composé obtenu à la Préparation 3.15 et de 0,16 g de LiOH dans 5 ml de méthanol et 1 ml d'eau. On évapore sous vide, neutralise à pH 6 avec HCl N puis extrait au DCM. On sèche la phase organique sur MgSO₄ et évapore sous vide pour obtenir 0,47 g de produit attendu.

RMN : 1,5-2,1 : m : 6H ; 2,3-4 : m : 20H ; 6,5 - 7,6 : m : 6H ; 8,4 : t : 1H.

### Préparation 4.13

Sel de potassium de l'acide 5-(2,6-diméthoxyphényl)-1-{4-[N-méthyl-N-(2-N',N'-diméthylaminoéthyl)aminosulfonyl]-5,6,7,8-tétrahydronapht-1-yl}pyrazole-3-carboxylique.

(II : R₁ = 4-SO₂NMe(CH₂)₂NMe₂ ; R₂, R₃ = -(CH₂)₄- ; R₄ = Me )

On laisse sous agitation 8 heures à TA une solution de 0,87 g d'ester obtenu à la Préparation 3.17 dans 5 ml de dioxane avec 320 mg de KOH dans 0,5 ml d'eau. On évapore sous vide et extrait le résidu par un mélange de 10 ml d'eau, 5 ml d'éthanol et 100 ml d'éther. Après décantation, la gomme obtenue est triturée trois fois dans l'éther, le produit cristallise. On filtre pour obtenir 0,9 g du sel attendu.

### Préparation 4.14

Acide 5-(2,6-diméthoxyphényl)-1-(4-carbamoyl-2-méthylphényl)pyrazole-3-carboxylique.

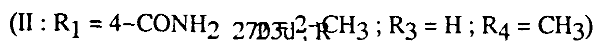

On laisse sous agitation à TA pendant 2 heures une solution contenant 0,4 g du composé obtenu à la Préparation 3.18 dans 5 ml de dioxane et 220 mg de KOH dans 1 ml d'eau. On acidifie à pH = 1 par addition d'HCl concentré puis on concentre sous vide. On ajoute 5 ml d'eau puis la gomme formée est agitée avec 50 ml de DCM et on filtre le précipité formé. On obtient 330 mg du produit attendu, F = 275-276°C.

RMN : 2,05 : s : 3H ; 3,55 : s : 6H ; 6,55 : d : 2H ; 6,7 : s : 1H ; 7 : d : 1H ; 7,2 : t : 1H ; 7.4 : s.e : 1H ; 7,55 : d : 1H ; 7,7 : s : 1H ; 7,9 : s.e : 1H.

### Préparation 4.15

Acide 5-(2,6-diméthoxyphényl)-1-{-2,3-diméthyl-4-[N-(2-N',N'-diméthylaminoéthyl)carbamoyl]phényl }pyrazole-3-carboxylique.

(II : R₁ = 4-CONH(CH₂)₂NMe₂ ; R₂ = 2-CH₃ ; R₃ = 3-CH₃ ; R₄ = CH₃)

On laisse sous agitation 2 heures à 40°C une solution de 1,8 g du produit de la Préparation 3.20 et 0,32 g de LiOH dans 10 ml de MeOH et 2 ml d'eau. On ajuste le pH à 6 avec HCl N et évapore sous vide. Après extraction du résidu avec 50 ml de DCM et évaporation on obtient 1,3 g de produit attendu.

RMN : 1,9 : s : 3H ; 2,2 : s+s : 9H ; 2,5 : t : 2H ; 3,4 : qd : 2H ; 3,7 : s : 6H ; 6,6-6,7 : m : 3H ; 6,9-7,1 : m : 2H ; 7,3 : t : 1H ; 8,3 : t : 1H.

### Préparation 4.16

Acide 5-(2,6-diméthoxyphényl)-1-{4-[N-méthyl-N-(2-N',N'-diéthylaminoéthyl) carbamoyl]-2-méthoxyphényl}pyrazole-3-carboxylique.

(II : R₁ = 4-CONMe(CH₂)₂NEt₂ ; R₂ = 2-OCH₃ ; R₃ = H ; R₄ = CH₃)

On laisse sous agitation 6 heures à reflux un mélange de 1,73 g du produit de la Préparation 3.22, 0,3 g de LiOH, dans 400 ml de MeOH et 6 ml d'eau puis on acidifie à pH = 2 avec HCl concentré. Après évaporation sous vide, agitation 30 minutes à TA de l'huile résiduelle avec 400 ml de chloroforme, décantation, séparation, séchage de la phase organique sur Na₂SO₄ et évaporation, on obtient 1,2 g de produit attendu.

RMN : 1,05-1,4 : mt : 6H ; 3 : s.e : 3H ; 3,1-3,9 : mt : 13H ; 6,6 : d : 2H ; 6,8 : s : 1H ; 7-7,4 : mt : 4H.

### Préparation 4.17

Acide 5-(2,6-diméthoxyphényl)-1-{4-[N-méthyl-N-(2-N',N'-diéthylaminoéthyl) carbamoyl]-2-chlorophényl}pyrazole-3-carboxylique.

(II : R₁ = 4-CONMe(CH₂)₂NEt₂ ; R₂ = 2-Cl ; R₃ = H ; R₄ = CH₃).

On laisse sous agitation 3 jours à TA un mélange de 2,6 g du produit de la Préparation 3.24 en solution dans 100 ml d'éthanol et de 0,53 g de KOH dans 15 ml d'eau. Après acidification à pH 3 avec HCl concentré, évaporation sous vide, trituration du résidu dans 10 ml d'eau, filtration et séchage sous vide sur P₂O₅ on obtient 1,55 g de produit attendu.

### Préparation 4.18

Acide 5-(2,6-diméthoxyphényl)-1-[4-(N-(2-morpholinoéthyl)carbamoyl)-2-chlorophényl]pyrazole-3-carboxylique.

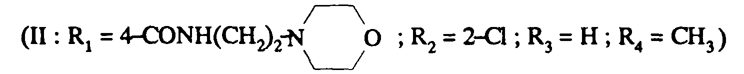

On chauffe sous agitation 1 heure à 60°C une solution de 1,5 g du produit de la Préparation 3.25 dans 75 ml d'éthanol avec une solution de 0,38 g de KOH dans 10 ml d'eau. Après acidification à pH 4,5 avec HCl concentré et évaporation sous vide on obtient 4 g de mélange du produit attendu et de KCl.

RMN (DMSO + TFA) : 2,9-4 : mt : H : 6,5 : d : 2H ; 6,8 : s : 1H ; 7,1-7,4 : mt : 2H ; 7,8 : d.d : 1H ; 8 : d : 1H ; 9,1 : s.e : 1H.

### Préparation 4.19

Acide 5-(2,6-diméthoxyphényl)-1-(3-chloro-4-cyanophényl)pyrazole-3-carboxylique.

(II' : R₁ = 4-CN ; R₂ = 3-Cl ; R₃ = H ; R₄ = CH₃)

On chauffe au bain marie pendant 3 heures un mélange contenant 0,5 g du composé obtenu à la Préparation 3.26 et 60 mg de LiOH dans 5 ml de méthanol aqueux. Après refroidissement, on abaisse le pH à 5 par addition d'HCl 1N. Le précipité formé est filtré et séché pour obtenir 0,36 g du composé attendu.

### Préparation 4.20

Acide 5-(2,6-diméthoxyphényl)-1-(4-carbamoyl-3-chlorophényl)pyrazole-3-carboxylique.

(II : R₁ = 4-CONH₂ ; R₂ = 3-Cl ; R₃ = H ; R₄ = CH₃)

On laisse sous agitation à TA pendant 24 heures un mélange contenant 0,87 g du composé obtenu à la Préparation 4.19, 390 mg de K₂CO₃ et 0,4 ml d'eau oxygénée à 30 % dans 5 ml de DMSO. On acidifie à pH 3 par addition d'HCl 1N puis on ajoute de l'eau, filtre le précipité formé et le sèche pour obtenir 0,73 g du produit attendu.

### Préparation 4.21

Acide 5-(2,6-diméthoxyphényl)-1-{4-[N-méthyl-N-(2-N',N'-diéthylaminoéthyl) carbamoyl]-2-cyclopropylphényl}pyrazole-3-carboxylique.

(II : R₁ = 4-CONMc(CH₂)₂NEt₂ ; R₂ = 2-cyclopropyle ; R₃ = H ; R₄ = CH₃)

On laisse sous agitation 22 heures à TA une solution de 1,29 g du produit de la Préparation 3.28 dans 26 ml d'éthanol avec une solution de 0,33 g de KOH dans 4 ml d'eau. Après acidification à pH 3 avec HCl concentré, évaporation et entraînement azéotropique avec 100 ml de toluène puis avec 100 ml de pentane, on triture le résidu dans le pentane, filtre et sèche pour obtenir 1,4 g de mélange du produit attendu avec KCl.
RMN : 0,4-1,2 : mt : 14H ; 1,5 : m : 1H ; 2,1-3,8 : mt : 13H
6,5 : d : 2H ; 6,7 : s.e : 2H ; 7 : s : 2H ; 7,2 : t : 1H.

### Préparation 4.22

Acide 5-(2,6-diméthoxyphényl)-1-{3-[N-méthyl-N-(2-N',N'-diméthylaminoéthyl) carbamoyl]-4-chlorophényl}pyrazole-3-carboxylique.

(II : R₁ = 3-CONMe(CH₂)₂NMe₂ ; R₂ = 4-Cl ; R₃ = H ; R₄ = CH₃).

On laisse sous agitation 6 heures à TA une solution de 0,8 g du produit de la Préparation 3.30 dans 10 ml de dioxane avec 0,22 g de KOH dans 2 ml d'H₂O. Après évaporation sous vide, le résidu est dissous dans 5 ml d'eau, neutralisé à pH 5 avec HCl concentré, puis saturé avec NaCl. On extrait 2 fois avec 100 ml de DCM, sèche la phase organique sur Na₂SO₄ et évapore sous vide pour obtenir 0,43 g de produit attendu.

RMN : 1,9-3,15 : m : 13H ; 3,6 : s : 6H ; 6,75 : d : 2H ; 6,85 : s.d : 1H ; 7,1-7,35 : m : 2H ; 7,45 : t : 1H ; 7,57 : d : 1H.

### Préparation 4.23

Acide 5-(2,6-diméthoxyphényl)-1-{5-[N-méthyl-N-(3-N',N'-diméthylamino-propyl)carbamoyl]-2-méthylphényl}pyrazole-3-carboxylique.

(II : R₁ = 5-CONMe(CH₂)₃NMe₂ ; R₂ = 2-CH₃ ; R₃ = H ; R₄ = CH₃).

On laisse sous agitation 15 heures à TA une solution de 2,28 g du produit de la Préparation 3.32 dans 10 ml de dioxane avec une solution de 0,65 g de KOH dans 1,5 ml d'eau ; on évapore sous vide puis on dissout le résidu dans 20 ml d'eau et extrait 3 fois avec 50 ml d'éther. Après acidification de la phase aqueuse à pH = 4, par addition d'HCl 1N, distillation azéotropique avec de l'éthanol, on triture le résidu avec 20 ml d'éthanol, filtre KCl puis évapore sous vide. On recommence cette élimination du KCl et évapore sous vide pour obtenir 2 g du produit attendu.

RMN : 1,9 : m : 2H ; 2,2 : s : 3H ; 2,4-3 : m : 11H ; 3,5 : mt : 2H ; 3,65 : s : 6H ; 6,65 : d : 2H ; 6,85 : s : 1H ; 7,1 : se : 1H ; 7,3-7,5 : m : 3H.

En suivant les modes opératoires ci-dessus, on prépare les acides de formule II décrits dans le tableau 5 ci-après.

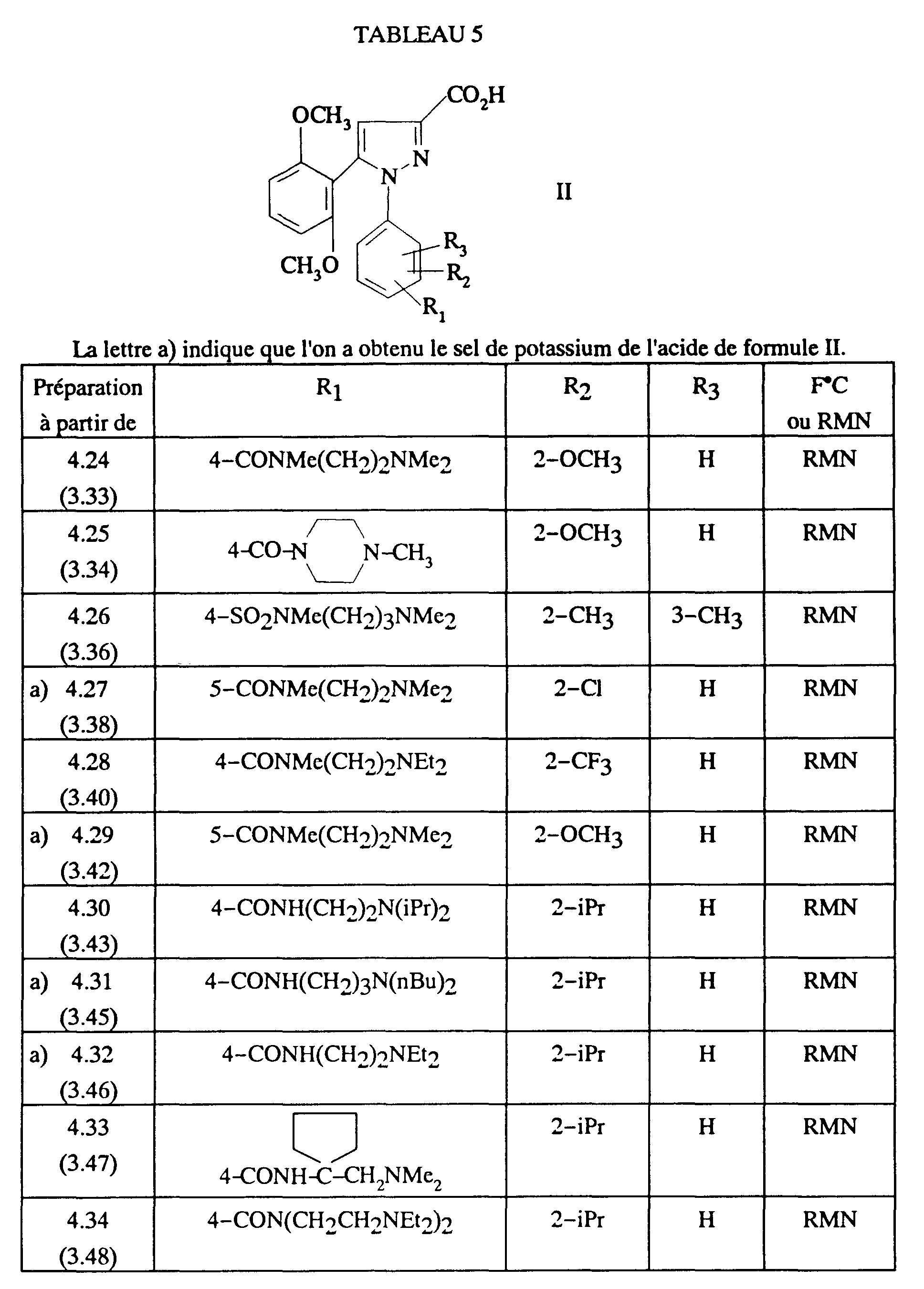

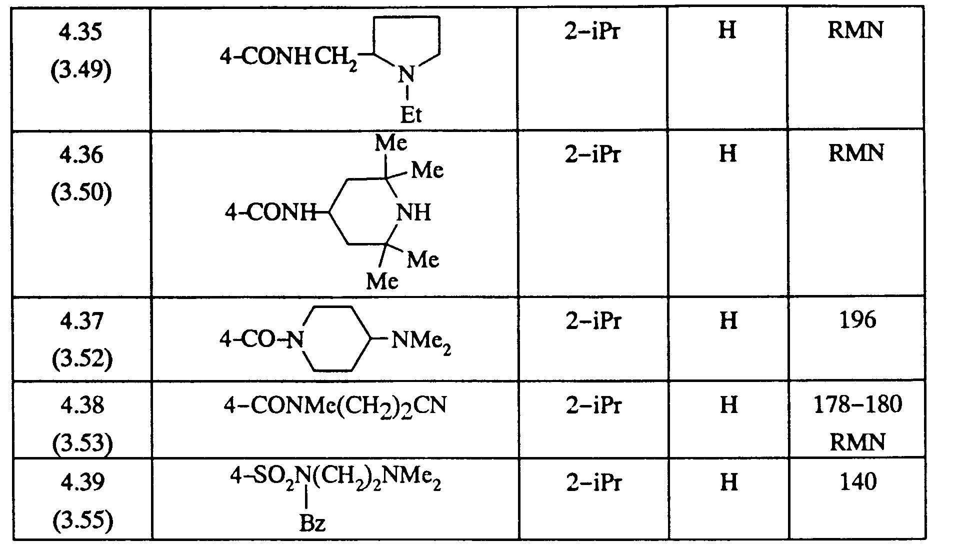

### RMN:

Préparation 4.24 (DMSO) : 2 : m : 2H ; 2,5 : m : 6H ; 2,8-3,5 : m : 5H ; 3,5 : s : 3H ; 3,6 : s : 6H ; 6,6 : d : 2H ; 6,8 : s : 1H ; 7,05 : m : 2H ; 7,15 : m : 2H.

Préparation 4.25 : 2,1 : s : 3H ; 2,3 : m : 4H ; 3,1 à 3,7 : m : 13H ; 6,5 : d : 2H ; 6,7 : s : 1H ; 6,9 : m : 2H ; 7,25 : m : 2H.

Préparation 4.27 : 1,6 - 3,2 : m : 13H ; 3,6 : s : 6H ; 6,4 : s : 1H ; 6,6 : d : 2H ; 7 - 8 : m : 4H.

Préparation 4.28 : 0,9 : m : 6H ; 2-3,4 : m : 11H ; 3,5 : s : 6H ; 6,4 : s : 1H ; 6,6 : d : 2H ; 7,15 : m : 2H ; 7,75 : d : 14H ; 7,85 : se : 1H.

Préparation 4.29 : 1,8 - 2,6 : m : 8H ; 2,8 : s.e : 3H ; 3,4 - 3,8 : s.e : 2H ; 3,6 : s : 6H ; 3,64 : s : 3H ; 6,50 : s : 1H ; 6,60 : d : 2H ; 7 - 7,50 : m : 4H.

Préparation 4.30 (DMSO + TFA) : 1 : d : 6H ; 1,3 : d : 12H ; 2,65 : mt : 1H ; 3,2 : t : 2H ; 3,5-3,75 : m + s : 10H ; 6,55 : d : 2H ; 6,8 : s : 1H ; 7,2-7,35 : mt : 2H ; 7,7 : d : 1H ; 7,8 : s : 1H.

Préparation 4.31 : 0,8 : t : 6H ; 0,95 : d : 6H ; 1,1-1,4 : m : 8H ; 1,58 : t : 2H ; 2,15-2,4 : m : 6H ; 2,55 : sp : 1H ; 3,2 : mt : 2H ; 3,57 : s : 6H ; 6,3 : s : 1H ; 6,5 : d : 2H ; 7,1-7,3 : m : 2H ; 7,74 : dd : 1H ; 7,75 : d : 1H ; 8,45 : t : 1H.

Préparation 4.32 : 0,9-1,1 : m : 12H ; 2,6 : m : 6H ; 2,7 : m : 1H ; 3,2-3,4 : m : 2H ; 3,6 : m : 6H ; 6,3 : s : 1H ; 6,6 : d : 2H ; 7,1-7,3 : m : 2H ; 7,6-7,8 : m : 2H.

Préparation 4.33 : 1 : d : 6H ; 1,5-1,9 : m : 6H ; 2-2,2 : m : 2H ; 2,2 : s : 6H ; 2,7 : s : 2H ; 2,8 : qt : 1H ; 3,6 : s : 6H ; 6,45 : s : 1H ; 6,6 : d : 2H ; 7,2-7,35 : m : 2H ; 7,6 : d : 1H ; 7,7 : s : 1H ; 7,9 : s : 1H.

Préparation 4.34 : 1 : m : 12H ; 1,3 : mt : 6H ; 2,5-3,9 : m + s : 23H ; 6,6 : d : 2H ; 6,8 : s : 1H ; 7,2-7,4 : m : 3H ; 7,55 : s : 1H.

Préparation 4.35 (DMSO + TFA) : 1 : m : 6H ; 1,2 : t : 3H ; 1,75-2,2 ; 2m : 4H ; 2,65 : mt : 1H ; 3 : m : 2H ; 3,4-3,7 : m + s : 11H ; 6,5 : d : 2H ; 6,75 ; s : 1H ; 7.2 : t : 1H ; 7,3 : d : 1H ; 7,65 : d : 1H ; 7,8 : s : 1H.

Préparation 4.36 : 1,05 : d : 6H ; 1,5 : s : 12H ; 1,7 : t : 2H ; 2,0 : d : 2H ; 2,7 : mt : 1H ; 3,7 : s : 6H ; 4,3-4,5 : m : 1H ; 6,7 ; d : 2H ; 6,85 : s : 1H ; 7,25-7,4 : mt : 2H ; 7,75 : d : 1H ; 7,9 : s : 1H ; 8,3-8,5 ; m : 1H ; 8,7 : d : 1H ; 12,8 : m : 1H.

Préparation 4.38 : 0,95 : d : 6H ; 2,6 : mt : 1H ; 2,7-3,8 : m : 13H ; 6,57 : d : 2H ; 6,75 : s : 1H ; 7,12-7,35 : m : 4H ; 12,7 : se : 1H.

### Préparation 4.40

Acide 1-[4-[N-éthyl-N-(2-N',N'-diéthylaminoéthyl)carbamoyl]-2-isopropylphényl]-5-(2,6-diméthoxyphényl)pyrazole-3-carboxylique.

(II : R₁ = 4-CONEt(CH₂)₂NEt₂ ; R₂ = 2-iPr ; R₃ = H ; R₄ = CH₃).

On refroidit à 5°C une solution de 0,48 g du composé obtenu à la Préparation 4.32 et 0,28 ml d'iodure d'éthyle dans 3 ml de THF, puis ajoute par portions 0,063 g d'hydrure de sodium à 60 % dans l'huile et laisse 24 heures sous agitation à TA. On ajoute 0,48 ml d'un mélange THF/eau (50/50 ; v/v) et concentre sous vide le mélange réactionnel. On reprend le résidu à l'eau, lave deux fois la phase aqueuse au pentane, acidifie la phase aqueuse à pH = 1 par ajout d'une solution d'HCl 1N, extrait à l'AcOEt puis au DCM, sèche les phases organiques sur Na₂SO₄ et évapore sous vide les solvants. On obtient 0,14 g du produit attendu.

RMN (DMSO + TFA) : 0,8-1,3 : m : 15H ; 2,6 : m : 1H ; 3,0-3,8 : m 16H ; 6,5 : d : 2H ; 6,75 : s : 1H ; 7,2 : m : 4H.

### Préparation 4.41

Chlorhydrate de l'acide 1-[4-[[3-(diéthylamino)pyrrolidin-1-yl]carbonyl]-2-isopropylphényl]-5-(2,6-diméthoxyphényl)pyrazole-3-carboxylique. 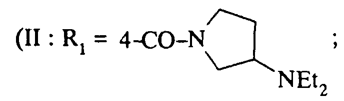 R₂ = 2-iPr ; R₃ = H ; R₄ = CH₃).

A une solution de 0,44 g du composé obtenu à la Préparation 3.51 dans 4 ml de dioxane, on ajoute à TA une solution de 0,11 g de KOH dans 0,5 ml d'eau et laisse une nuit sous agitation à TA. On concentre sous vide, reprend le résidu à l'eau, lave deux fois la phase aqueuse à l'éther, acidifie la phase aqueuse à pH = 2 par ajout d'HCl 1,2 N, ajoute de l'EtOH et concentre sous vide. On reprend le résidu à l'EtOH, filtre le KCI et concentre sous vide le filtrat. On obtient 0,39 g du produit attendu.

RMN (DMSO + TFA) : 1,0 : d : 6H ; 1,15-1,35 : m : 6H ; 2,1-2,45 : m : 2H ; 2,65 : mt : 1H ; 2,9-4,1 : 3m + s : 15 H ; 6,6 : d : 2H ; 6,8 : s : 1H ; 7,2-7,5 : mt : 3H ; 7,55 : s : 1H.

### Préparation 4.42

Acide 1-[4-[N-(propèn-2-yl)carbamoyl]-2-isopropylphényl]-5-(2,6-diméthoxyphényl)pyrazole-3-carboxylique.

(II : R₁ = 4-CONHCH₂CH = CH₂ ; R₂ = 2-iPr ; R₃ = H ; R₄ = CH₃).

On laisse 7 heures sous agitation à TA un mélange de 1,72 g du composé obtenu à la Préparation 3.54 et 0,78 g de LiOH, H₂O dans 10 ml de MeOH et 1 ml d'eau. On concentre sous vide, reprend le résidu à l'eau, lave deux fois la phase aqueuse à l'éther, acidifie à pH = 2-3 par ajout d'HCl 1,2N, extrait au DCM, sèche la phase organique sur MgSO₄ et évapore sous vide le solvant. On obtient 1,64 g du produit attendu.

RMN : 1,0 : d : 6H ; 2,7 : qt : 1H ; 3,7 : s : 6H ; 3,95 : t : 2H ; 5,1-5,3 : m : 2H ; 5,8-6,1 : m : 1H ; 6,65 : d : 2H ; 6,85 : s : 1H ; 7,25-7,4 : m : 2H ; 7,75 : d : 1H ; 7,9 : s : 1H ; 8,8 : t : 1H.

### Préparation 4.43

Acide 1-[4-[N-méthyl-N-[3-[N'-méthyl-N'-(*tert*-butoxycarbonyl) amino]propyl]carbamoyl]-2-isopropylphényl]-5-(2,6-diméthoxyphényl)pyrazole-3-carboxylique.

(II : R₁ = 4-CONMe(CH₂)₃N(Me)COOt-Bu; R₂ = 2-iPr ; R₃ = H ; R₄ = CH₃).

On laisse 3 heures 30 minutes sous agitation à TA un mélange de 2,85 g du composé obtenu à la Préparation 3.57 et 0,98 g de LiOH, H₂O dans 20 ml de MeOH et 1 ml d'eau. On concentre sous vide, reprend le résidu à l'eau, acidifie à pH = 2 par ajout d'une solution tampon pH = 2, essore le précipité formé et le lave à l'eau. On obtient 2,47 g du produit attendu après séchage sur P₂O₅, F = 112-114°C.

### Préparation 4.44

Acide 1-[4-(4-méthylphénylsulfonylamino)-2-isopropylphényl]-5-(2,6-diméthoxyphényl]pyrazole-3-carboxylique.

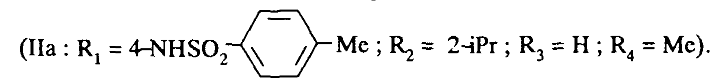

On chauffe 3 heures à 60°C un mélange de 1,05 g du composé obtenu à la Préparation 3.58 et 0,33 g de LiOH, H₂O dans 5 ml de MeOH et 0,5 ml d'eau. On verse le mélange réactionnel dans l'eau, acidifie à pH = 2-3 par ajout d'une solution à 10 % d'HCl, essore le précipité formé et le sèche. On obtient 0,92 g du produit attendu.

RMN : 0,7 : d : 6H ; 2,3-2,6 : m : 4H ; 3,55 : s : 6H ; 6,6 : d : 2H ; 6,75 : s : 1H ; 6,85-7,01 : m : 2H ; 7,11 : d : 1H ; 7,25-7,42 : m : 3H ; 7,6 : d : 2H ; 10,3 : s : 1H ; 12,75 : se : 1H.

### Préparation 4.45

Acide 1-(4-amino-2-isopropylphényl)-5-(2,6-diméthoxyphényl)pyrazole-3-carboxylique.

(II' : R'₁ = 4-NH₂ ; R₂ = 2-iPr ; R₃ = H ; R₄ = Me).

On chauffe à reflux pendant 10 minutes un mélange de 0,9 g du composé obtenu à la Préparation 3.58, 11 ml d'acide acétique et 25 ml d'acide perchlorique à 70 %. On verse le mélange réactionnel sur un mélange eau/glace, filtre un insoluble, amène le filtrat à pH = 5 par ajout de NaOH 10 %, filtre, extrait à l'AcOEt, sèche la phase organique sur Na₂SO₄ et évapore sous vide le solvant. On reprend le résidu à l'éther et essore le précipité formé. On obtient 0,54 g du produit attendu, F = 190°C (déc).

RMN : 0,92 : d : 6H ; 2,42 : mt : 1H ; 3,65 : s : 6H ; 5,42 : se : 2H ; 6,3 : dd : 1H ; 6,4 : d : 1H ; 6,6 : d : 2H ; 6,67 : s : 1H ; 6,85 : d : 1H ; 7,3 : t : 1H.

### Préparation 4.46

Acide 1-[4-[3-(diéthylamino)propanoylamino]-2-isopropylphényl]-5-(2,6-diméthoxyphényl)pyrazole-3-carboxylique.

(II : R₁ = 4-NHCO(CH₂)₂NEt₂ ; R₂ = 2-iPr ; R₃ = H ; R₄ = Me).

On chauffe 1 heure à reflux un mélange de 0,22 g de chlorhydrate de l'acide 3-diéthylaminopropanoïque et 2 ml de SOCl₂ dans 2 ml de DCM puis concentre sous vide. Le chlorure d'acide ainsi obtenu est utilisé tel quel. D'autre part on chauffe à 70°C pendant 1 heure un mélange de 0,47 g du composé obtenu à la Préparation 4.45 et 0,95 ml de bis(triméthylsilyl)acétamide dans 5 ml d'acétonitrile. Après refroidissement à TA, on ajoute le chlorure d'acide préparé ci-dessus en solution dans du DCM puis 0,17 ml de triéthylamine et laisse 1 heure sous agitation à TA. On concentre sous vide, reprend le résidu à l'eau, amène le pH à 5 par ajout de NaOH 10 %, extrait au DCM, sèche la phase organique sur Na₂SO₄ et évapore sous vide le solvant. On reprend le résidu à l'éther et essore le précipité formé. On obtient 0,27 g du produit attendu.

RMN (DMSO + TFA) : 0,91 : d : 6H ; 1,2 : mt : 6H ; 2,55 : mt : 1H ; 2,8 : t : 2H ; 3,1-3,22 : m : 4H ; 3,35 : t : 2H ; 3,6 : s : 6H ; 6,58 : d : 2H ; 6,75 : s : 1H ; 7,1-7,3 : m : 2H ; 7,4-7,55 : m : 2H.

### Préparation 4.47

Acide 1-[4-[(3-diéthylaminopropyl)amino]-2-isopropylphényl]-5-(2,6-diméthoxyphényl)pyrazole-3-carboxylique.

(II : R₁ = 4-NH(CH₂)₃NEt₂ ; R₂ = 2-iPr ; R₃ = H ; R₄ = Me).

On chauffe 10 minutes à reflux un mélange de 0,55 g du composé obtenu à la Préparation 3.59, 6,5 ml d'acide acétique et 14 ml d'acide perchlorique à 70 %. On verse le mélange réactionnel dans l'eau, amène à pH = 5 par ajout de NaOH 10 %, extrait à l'AcOEt, sèche sur Na₂SO₄ et évapore sous vide le solvant. On obtient 0,5 g du produit attendu.

RMN : 1,0 : mt : 6H ; 1,25 : t : 6H ; 1,9 : mt : 2H ; 2,55 : s : 1H ; 3,0-3,3 : m : 8H ; 3,7 : s : 6H ; 5,9 : s : 1H ; 6,3-6,55 : m : 2H ; 6,65 : d : 2H ; 6,75 : s : 1H ; 7,0 : d : 1H ; 7,3 : t : 1H.

### Préparation 4.48

Acide 1-[4-[N-acétyl-N-(3-diéthylaminopropyl)amino]-2-isopropylphényl]-5-(2,6-diméthoxyphényl)pyrazole-3-carboxylique.

(II : R₁ = 4-N(COMe)(CH₂)₃NEt₂; R₂ = 2-iPr ; R₃ = H ; R₄ = Me).

On chauffe 1 heure à 60°C un mélange de 0,38 g du composé obtenu à la Préparation 4.47 et 0,36 ml de bis(triméthylsilyl)acétamide dans 10 ml de toluène. On ajoute 0,052 ml de chlorure d'acétyle puis 0,1 ml de triéthylamine et laisse 2 heures sous agitation à TA. On concentre sous vide, reprend le résidu par une solution saturée de NaCl, extrait à l'AcOEt, sèche sur Na₂SO₄ et évapore sous vide le solvant. On obtient 0,39 g du produit attendu.

RMN : 0,95 : d : 6H ; 1,25 : t : 6H ; 1,6-2,0 : m : 5H ; 2,65 : sp : 1H ; 3,0-3,3 : m : 6H ; 3,65 : s : 6H ; 3,75 : t : 2H . 6,65 : d : 2H . 6,85 : s : 1H ; 7,2-7,6 : m : 4H.

### Préparation 4.49

Sel de potassium de l'acide 1-[4-[N-méthyl-N-(3-diméthylaminopropyl) carbamoyl]-2-isopropylphényl]-5-[2-(cyclopropylméthyloxy)-6-méthoxyphényl] pyrazole-3-carboxylique.

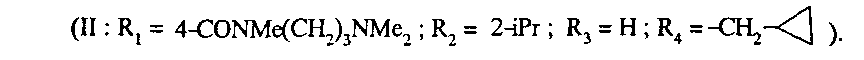

On laisse 20 heures sous agitation à TA un mélange de 3,8 g du composé obtenu à la Préparation 3.61 et 0,92 g de KOH dans 76 ml d'EtOH et 12 ml d'eau. On concentre sous vide, reprend le résidu au toluène et évapore sous vide le solvant. On obtient 3,9 g du produit attendu.

### Préparation 4.50

Acide 1-(2-méthyl-4-nitrophényl)-5-(2,6-diméthoxyphényl)pyrazole-3-carboxylique.

(Il' : R'₁ = 4-NO₂ ; R₂ = 2-Me ; R₃ = H ; R₄ = Me).

On laisse une nuit sous agitation un mélange de 3,5 g du composé obtenu à la Préparation 3.56 et 0,44 g de LiOH, H₂O dans 20 ml de MeOH et 4 ml d'eau. On concentre sous vide, reprend le résidu à l'eau, acidifie à pH = 3 par ajout d'HCl 10 %, essore le précipité formé et le sèche. On obtient 3,2 g du produit attendu.

RMN : 2,9 : s : 3H ; 3,58 : s : 6H ; 6,6 : d : 2H ; 6,88 : s : 1H ; 7,2-7,38 : m : 2H ; 7,95 : dd : 1H ; 8,22 : d : 1H.

### Préparation 4.51

Acide 1-(4-amino-2-isobutylphényl)-5-(2,6-diméthoxyphényl)pyrazole-3-carboxylique.

(II' : R'₁ = 4-NH₂ ; R₂ = 2-iBu ; R₃ = H ; R₄ = Me).

On prépare ce composé selon le mode opératoire décrit à la Préparation 4.45 à partir de 0,5 g du composé obtenu à la Préparation 3.62, 6 ml d'acide acétique et 14 ml d'acide perchlorique à 70 %. On obtient 0,3 g du produit attendu.

RMN : 0,65 : d : 6H ; 1,55 : mt : 1H ; 1,9 : d : 2H ; 3,5 : s : 6H ; 5,05 : s : 2H ; 6,0-7,3 : m : 7H.

### Préparation 4.52

Acide 1-[4-[3-(diéthylamino)propanoylamino]-2-isobutylphényl]-5-(2,6-diméthoxyphényl)pyrazole-3-carboxylique.

(II : R₁ = 4-NHCO(CH₂)₂NEt₂ ; R₂ = 2-iBu ; R₃ = H ; R₄ = Me).

On prépare ce composé selon le mode opératoire décrit à la Préparation 4.46 à partir de 0,133 g de chlorhydrate de l'acide 3-diéthylaminopropanoïque et 1 ml de SOCl₂ dans 1 ml de DCM et 0,29 g du composé obtenu à la Préparation 4.51 et 4 ml de bis(triméthylsilyl)acétamide dans 2 ml d'acétonitrile. On obtient 0,15 g du produit attendu.

RMN : 0,7 : d : 6H ; 1,1 : t : 6H ; 1,65 : mt : 1H ; 2,0 : d : 2H ; 2,75 : t : 2H ; 3,1 : qd : 4H ; 3,3 : t : 2H ; 3,6 : s : 6H ; 6,5 : d : 2H ; 6,7 : s : 1H ; 7,1 : d : 1H ; 7,2 : t : 1H ; 7,3-7,5 : m : 2H ; 10,3 : s : 1H ; 15 : s : 1H.

### Préparation 4.53

Acide 1-(4-amino-2-cyclopentylphényl)-5-(2,6-diméthoxyphényl)pyrazole-3-carboxylique.

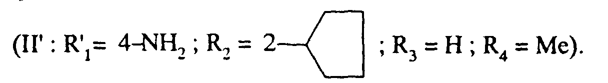

On prépare ce composé selon le mode opératoire décrit à la Préparation 4.45 à partir de 0,9 g du composé obtenu à la Préparation 3.63, 11 ml d'acide acétique et 27 ml d'acide perchlorique à 70 %. On obtient 0,52 g du produit attendu.

RMN (DMSO + TFA) : 1,18-1,9 : m : 8H ; 2,6 : mt : 1H ; 3,6 : s : 6H ; 6,6 : d : 2H 6,75 : s : 1H ; 7,1-7,4 : m : 4H.

### Préparation 4.54

Acide 1-[4-[3-(diéthylamino)propanoylamino]-2-cyclopentylphényl]-5-(2,6-diméthoxyphényl)pyrazole-3-carboxylique.

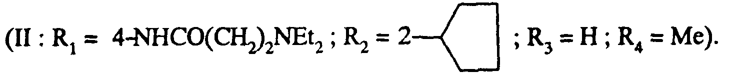

On prépare ce composé selon le mode opératoire décrit à la Préparation 4.46 à partir de 0,22 g de chlorhydrate de l'acide 3-diéthylaminopropanoïque, 2 ml de SOCl₂ dans 5 ml de DCM, 0,5 g du composé obtenu à la Préparation 4.53 et 0,73 ml de bis(triméthylsilyl)acétamide dans 2 ml d'acétonitrile. On obtient 0,32 g du produit attendu.

RMN (DMSO + TFA) : 1,1-1,8 : m : 14H ; 2,5 : mt : 1H ; 2,72 : t : 2H ; 3,02 : m : 4H ; 3,22 : mt : 2H ; 3,58 : s : 6H ; 6,5 : d : 2H ; 6,65 : s : 1H ; 7,03 : d : 1H ; 7,2 : t : 1H ; 7,35 : dd : 1H ; 7,5 : d : 1H.

### Préparation 4.55

Sel de potassium de l'acide 1-[4-[N-(2-diéthylaminoéthyl)carbamoyl]-3-isopropylphényl]-5-(2,6-diméthoxyphényl)pyrazole-3-carboxylique.

(II : R₁ = 4-CONH(CH₂)₂NEt₂ ; R₂ = 3-iPr ; R₃ = H ; R₄ = Me).

On laisse 2 jours sous agitation à TA un mélange de 0,34 g du composé obtenu à la Préparation 3.65 et 0,073 g de KOH dans 6 ml de dioxane et 2 ml d'eau. On concentre sous vide, reprend le résidu au toluène et concentre sous vide. On obtient 0,37 g du produit attendu, F > 260°C.

### Préparation 4.56

Acide 5-(2,6-diméthoxyphényl)-1-(4-nitro-5,6,7,8-tétrahydronapht-1-yl) pyrazole-3-carboxylique.

(II' : R'₁ = 4-NO₂ ; R₂, R₃ = -(CH₂)₄- ; R₄ = Me).

On chauffe à 70°C pendant 2 heures un mélange de 4,2 g du composé obtenu à la Préparation 3.66 avec 0,8 g de LiOH, H₂O dans 95 ml d'EtOH et 5 ml d'eau. Après refroidissement à TA, on ajoute de l'eau, acidifie à pH = 3 par ajout d'HCl 10 %, essore le précipité formé et le sèche. On obtient 4,16 g du produit attendu, F = 130°C.

RMN : 1,7 : mt : 4H ; 2,55 : mt : 2H ; 2,82 : mt : 2H ; 3,65 : s : 6H ; 6,65 : d : 2H ; 6,85 : s : 1H ; 7,05 : d : 1H ; 7,35 : t : 1H ; 7,7 : d : 1H ; 12,95 : se : 1H.

### Préparation 4.57

Acide 5-(2,6-diméthoxyphényl)-1-(5-(3-diéthylamino)propanoylamino)-2-isopropylphényl)pyrazole-3-carboxylique.

(II : R₁ = 5-NHCO(CH₂)₂NEt₂ ; R₂ = 2-iPr ; R₃ = H ; R₄ = CH₃).

Ce composé est obtenu à partir de l'ester méthylique de la Préparation 3.67. Après recristallisation dans le méthanol, F = 195-198°C.

RMN (DMSO + TFA) : 0,65-1,35 : m : 12H ; 2,5 : qt : 1H ; 2,82 : t : 2H ; 3,15 : mt : 4H ; 3,36 : t : 2H ; 3,6 : s : 6H ; 6,55 : d : 2H ; 6,76 : s : 1H ; 7,15-7,4 : m : 3H ; 7,8 : d : 1H.

On chauffe à 70°C pendant 2 heures un mélange de 4,2 g du composé obtenu à la Préparation 3.66 avec 0,8 g de LiOH, H₂O dans 95 ml d'EtOH et 5 ml d'eau. Après refroidissement à TA, on ajoute de l'eau, acidifie à pH = 3 par ajout d'HCl 10 %, essore le précipité formé et le sèche. On obtient 4,16 g du produit attendu, F = 130°C.

RMN : 1,7 : mt : 4H ; 2,55 : mt : 2H ; 2,82 : mt : 2H ; 3,65 : s : 6H ; 6,65 : d : 2H ; 6,85 : s : 1H ; 7,05 : d : 1H ; 7,35 : t : 1H ; 7,7 : d : 1H ; 12,95 : se : 1H.

### Préparation 4.57

Acide 5-(2,6-diméthoxyphényl)-1-(5-(3-diéthylaminopropanoylamino)-2-isopropylphényl)pyrazole-3-carboxylique.

(II : R₁ = 5-NHCO(CH₂)₂NEt₂ ; R₂ = 2-iPr ; R₃ = H ; R₄ = CH₃).

Ce composé est obtenu à partir de l'ester méthylique de la Préparation 3.67. Après recristallisation dans le méthanol, F = 195-198°C.

RMN (DMSO + TFA) : 0,65-1,35 : m : 12H ; 2,5 : qt : 1H ; 2,82 : t : 2H ; 3,15 : mt : 4H ; 3,36 : t : 2H ; 3,6 : s : 6H ; 6,55 : d : 2H ; 6,76 : s : 1H ; 7,15-7,4 : m : 3H ; 7,8 : d : 1H.

### EXEMPLE 1

Chlorhydrate de l'acide 2-[5-(2,6-diméthoxyphényl)-1-[4-[N-méthyl-N-(3-diméthylaminopropyl)carbamoyl]-2-isopropylphényl]pyrazol-3-yl-carbonylamino] adamantane-2-carboxylique.

(I : R₁ = 4-CONMe(CH₂)₃NMe₂ ; R₂ =2-iPr ; R₃ = H ; R₄ = CH₃ ; AA(OH) = 2-carboxyadamant-2-yl).

A) Chlorhydrate du chlorure de l'acide 1-[4-[N-méthyl-N-(3-diméthylamino propyl)carbamoyl]-2-isopropylphényl]-5-(2,6-diméthoxyphényl)pyrazole-3-carboxylique.

On laisse agiter sous azote à TA pendant 5 heures 1,07 g de l'acide obtenu à la Préparation 4.1 dans 2 ml de chlorure de thionyle. On évapore puis reprend par du DCM (3 fois) pour obtenir le produit attendu que l'on utilise tel quel à l'étape suivante.

On peut aussi préparer le chlorure d'acide selon le mode opératoire ci-dessous :

A') Chlorhydrate du chlorure de l'acide 1-[4-[N-méthyl-N-(3-diméthylamino propyl)carbamoyl]-2-isopropylphényl]-5-(2,6-diméthoxyphényl)pyrazole-3-carboxylique.

On redissout le sel de potassium de la Préparation 4.1 bis dans 130 ml d'éthanol, ajoute 50 ml d'éthanol chlorhydrique, filtre le minéral et évapore sous vide. On redissout le résidu dans 100 ml de DCM, ajoute lentement 11 ml de SOCl₂ et chauffe à reflux pendant 4 heures. On évapore sous vide, redissout dans 30 ml de DCM et évapore sous vide, on répète 3 fois l'opération.

B) Chlorhydrate de l'acide 2-[5-(2,6-diméthoxyphényl)-1-[4-[N-méthyl-N-(3-diméthylaminopropyl)carbamoyl]-2-isopropylphényl]pyrazol-3-yl-carbonylamino]adamantane-2-carboxylique.

On chauffe à reflux sous azote pendant 40 minutes un mélange contenant 0,37 g d'acide 2-aminoadamantane-2-carboxylique (composé B), 5 ml d'acétonitrile et 0,82 ml de bis(triméthylsilyl)acétamide. Après retour à TA, on ajoute 0,3 ml de triéthylamine et le produit obtenu à l'étape précédente dissous dans 15 ml d'acétonitrile. On laisse sous agitation à TA pendant 1 semaine, on concentre les solvants. Par addition d'éther on obtient une cristallisation. Les cristaux sont agités dans un mélange de 1,5 ml de toluène et 1,5 ml d'acétonitrile. On filtre l'insoluble, rince et évapore les solvants. Le résidu est agité dans du méthanol aqueux puis on évapore à nouveau et reprend avec de l'éthanol. On extrait au DCM, lave la phase organique par une solution saturée de NaCl puis chromatographie sur silice en éluant par le mélange DCM/MeOH/H₂O (92/8/0,7 ; v/v/v). On obtient 0,18 g du produit attendu après trituration dans l'éther, F = 185°C (déc.).

RMN (DMSO+TFA) : 0,95 : d : 6H ; 1,6-2,2 : m : 14H ; 2,4-3 : m : 12H ; 3,1 : mt : 2H ; 3,5 : mt : 2H ; 3,65 : s : 6H ; 6,6 : d : 2H ; 6,7 : s : 1H ; 7,1-7,5 : m : 4H.

Alternativement l'étape B peut être effectuée de la façon suivante :

On chauffe à reflux sous azote pendant 40 minutes un mélange de 8,79 g de composé B, 22 ml de bis(triméthylsilyl)acétamide dans 120 ml d'acétonitrile sec et refroidit à TA. On ajoute ensuite une solution du chlorure d'acide obtenu selon A à partir de 23,83 g de l'acide obtenu à la Préparation 4.1 et 140 ml de chlorure de thionyle dans 300 ml d'acétonitrile sec. Après agitation 15 heures à TA et évaporation sous vide on redissout le résidu dans 180 ml de MeOH, ajoute lentement 180 ml d'eau, agite une heure, filtre l'insoluble et évapore le filtrat sous vide après addition d'éthanol. Après agitation dans 200 ml d'HCl 1N, on filtre, lave le précipité avec HCl 1N et sèche sous vide sur P₂O₅ pour obtenir 29,8 g du produit de l'EXEMPLE 1, F = 211°C (déc) après recristallisation dans la propan-2-ol.

### EXEMPLE 1'

Sel interne de l'acide 2-[5-(2,6-diméthoxyphényl)-1-[4-[N-méthyl-N-(3-diméthylaminopropyl)carbamoyl]-2-isopropylphényl]pyrazol-3-yl-carbonylamino] adamantane-2-carboxylique.

A partir du chlorhydrate du composé obtenu à l'EXEMPLE 1 on libère le sel interne de la façon suivante :

On dissout 0,97 g du produit de l'EXEMPLE 1 dans 10 ml d'eau, remonte le pH à 7 par addition de soude 1,3N. On filtre, lave à l'eau et sèche sous vide sur P₂O₅ pour obtenir 0,86 g de sel interne que l'on recristallise dans 3 ml d'acétonitrile pour obtenir 0,5 g du sel interne attendu.

RMN : (DMSO + TFA) : 1 : mt : 6H ; 1,4-2,3 : m: 14H ; 2,3-3,4 : m : 14H ; 3,5 : m : 2H : 3,65 : s : 6H ; 6,6 : d : 2H ; 6,7 : s : 1H ; 7,1-7,5 : m : 4H.

Après recristallisation dans le propan-2-ol, F = 238°C.

RMN (DMSO + TFA) : 1,05 : d : 6H ; 1,5-2,2 : m : 14H ; 2,5 : se : 2H ; 2,6-3 : m : 10H ; 3,1 : mt : 2H ; 3,5 : mt : 2H ; 3,6 : s : 6H ; 6,6 : d : 2H ; 6,75 : s : 1H ; 7,1-7,5 : m : 4H.

On peut aussi préparer le produit de l'EXEMPLE 1' sans isoler le produit de l'EXEMPLE 1 selon le mode opératoire suivant :

On chauffe à reflux sous azote pendant 2 heures un mélange de 9,7 g du composé B et de 27 ml de bis(triméthylsilyl)acétamide dans 100 ml de DCM anhydre. Après refroidissement on verse la solution ainsi obtenue sur la solution du produit de l'étape A' de l'EXEMPLE 1 dans 100 ml de DCM et agite pendant une nuit à TA. On évapore sous vide, traite le résidu par agitation pendant 3 heures avec 100 ml de MeOH et 100 ml d'eau et remonte le pH à 7-7,5 par addition d'une solution saturée de NaHCO₃. Après 1 heure d'agitation on filtre pour obtenir 22,1 g du produit attendu (pureté HPLC 98,5 %).

On peut aussi transformer le sel interne en son chlorhydrate (produit de l'EXEMPLE 1) selon le mode opératoire suivant :

On chauffe en agitant 6,85 g de sel interne dans un mélange de 3,5 ml d'HCl concentré et de 40 ml d'eau. Après dissolution on laisse refroidir sous agitation, filtre et sèche sous vide pour obtenir 6,5 g de chlorhydrate.

A partir du sel interne on peut obtenir son chlorhydrate de la façon suivante :

On dissout en chauffant 0,3 g de sel interne dans 3 ml de MeOH et 2 ml de DCM, refroidit à TA, ajoute 0,5 ml d'HCl 1,2N, concentre sous vide à 0,5 ml, refroidit à -20°C et filtre pour obtenir 0,2 g du produit de l'EXEMPLE 1.

### EXEMPLE 2

Acide 2-{1-[4-[N-(2-cyanoéthyl)carbamoyl]-2-isopropylphényl]-5-(2,6-diméthoxyphényl)pyrazol-3-ylcarbonylamino}adamantane-2-carboxylique.

(I : R₁ = 4-CONHCH₂CH₂CN ; R₂ = 2-iPr ; R₃ = H ; R₄ = CH₃) ; AA(OH) = 2-carboxyadamant-2-yl).

On laisse 5 heures sous agitation à TA un mélange de 2,6 g du composé obtenu à la Préparation 4.2 et 20 ml de chlorure de thionyle. On concentre sous vide, reprend le résidu au DCM et évapore sous vide le solvant. On utilise tel quel le chlorure d'acide ainsi obtenu. D'autre part, on chauffe à reflux, sous atmosphère d'azote, pendant 30 minutes un mélange de 1,09 g d'acide 2-aminoadamantane-2-carboxylique, 4,2 ml de bis(triméthylsilyl)acétamide dans 20 ml d'acétonitrile. Après refroidissement, on ajoute lentement une solution du chlorure d'acide préparé ci-dessus dans 40 ml d'acétonitrile et laisse une nuit sous agitation à TA. On concentre sous vide, reprend le résidu au MeOH, ajoute quelques gouttes d'eau et laisse 2 heures sous agitation à TA. On essore le précipité, lave au MeOH et sèche. On chromatographie le précipité sur silice H en éluant par le mélange DCM/McOH (100/3 ; v/v) puis par le mélange DCM/MeOH/AcOH (100/3/0,5 ; v/v/v). On obtient 1,96 g du produit attendu après cristallisation dans l'éther, F = 269°C.

RMN : 1 : d : 6H ; 1,4-2,1 : m : : 12H ; 2,4-2,8 : m : 5H ; 3,4 : mt : 2H ; 3,5 : s : 6H ; 6,5 : d : 2H ; 6,6 : s : 1H ; 7,1-7,4 : m : 2H ; 7,5 : d : 1H ; 7,8 : s : 1H ; 8,9 : t : 1H.

### EXEMPLE 3

Acide 2-{1-[4-[N-(3-aminopropyl)carbamoyl]-2-isopropylphényl]-5-(2,6-diméthoxyphényl)pyrazol-3-ylcarbonylamino}adamantane-2-carboxylique.

(I : R₁ = 4-CONH(CH₂)₃NH₂ ; R₂ = 2-iPr ; R₃ = H ; R₄ = CH₃ ; AA(OH) = 2-carboxyadamant-2-yl).

On hydrogène pendant une nuit à TA et à pression atmosphérique un mélange de 0,3 g du composé obtenu à l'EXEMPLE 2, 0,03 g de nickel de Raney® dans 10 ml de MeOH, filtre le catalyseur, lave au MeOH et concentre partiellement le filtrat sous vide. On filtre les cristaux formés et lave à l'EtOH pour obtenir un premier jet du produit attendu. On concentre partiellement le filtrat sous vide et laisse sous agitation à TA. On essore les cristaux formés et lave à l'EtOH pour obtenir un deuxième jet. En rassemblant les deux jets on obtient 0,045 g du produit attendu, F = 280°C (déc.)

RMN : 1,1 : d : 6H ; 1,5-2,2 : m : 14H ; 2,4-3 : m : 5H ; 3,3 : mt : 2H ; 3,6 : s : 6H ; 6,6 : d : 2H ; 6,7 : s : 1H ; 7,2-7,4 : m : 2H ; 7,6 : d : 1H ; 7,7 : s : 1H.

### EXEMPLE 4

Acide 2-{1-[4-[N-(2-amidinoéthyl)carbamoyl]-2-isopropylphényl]-5-(2,6-diméthoxyphényl)pyrazol-3-ylcarbonylamino}adamantane-2-carboxylique.

(I : R₁ = 4-CONH(CH₂)₂C(=NH)NH₂; R₂ =2-iPr ; R₃ = H ; R₄=CH₃ ; AA(OH)=2-carboxyadamant-2-yl)

Etape A. On refroidit au bain de glace une solution de 0,37 g du composé obtenu à l'EXEMPLE 2 dans 10 ml d'EtOH et 10 ml d'éther anhydre, puis on fait barboter pendant 50 minutes de l'HCl gaz. On abandonne 3 jours à + 5°C puis concentre sous vide pour obtenir le chlorhydrate de l'imidate intermédiaire (R₁ = 4-CONH(CH₂)₂C (=NH)OEt).

Etape B. On reprend le résidu dans 20 ml d'EtOH anhydre, refroidit au bain de glace et fait barboter pendant 35 minutes de l'ammoniac. On laisse 30 minutes sous agitation à TA, concentre sous vide, reprend le résidu dans l'eau et laisse cristalliser. Après essorage puis séchage des cristaux, on recristallise dans l'EtOH à chaud. On obtient 0,3 g du produit attendu, F = 257°C (déc.).

RMN : (DMSO + TFA). 1,1 : d : 6H ; 1,5-2,2 : m : 14H ; 2,4-2,8 : m : 3H ; 3,4-3,7 : m+s : 8H ; 6,6 : d : 2H ; 6,7 : s : 1H ; 7,2-7,4 : m : 2H ; 7,6 : d.d : 1H ; 7,8 : s.e : 1H.

### EXEMPLE 5

Dichlorhydrate de l'acide 2-[5-(2,6-diméthoxyphényl)-1-[4-[N-(2-dihydro imidazol-2-yléthyl)carbamoyl]-2-isopropylphényl]pyrazol-3-yl-carbonylamino] adamantane-2-carboxvlique.

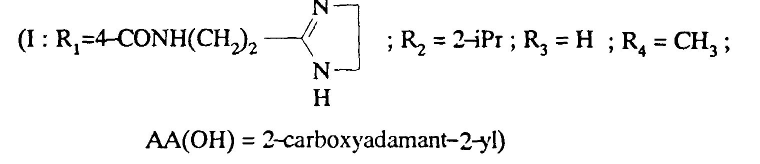

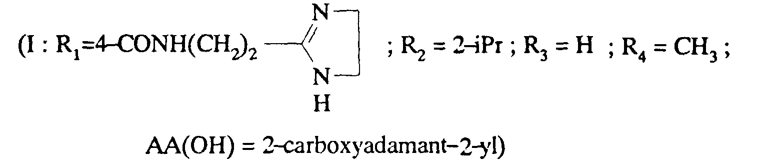

On agite pendant 30 minutes un mélange contenant 0,49 g de l'imidate intermédiaire décrit dans l'EXEMPLE 4 étape A et 5 ml de 1,2-diaminoéthane. On évapore le milieu réactionnel. Par addition d'eau, il se forme un précipité qui est filtré puis rincé à l'eau. Ce produit est mis en suspension dans l'éthanol et on ajoute de l'éther chlorhydrique. Après évaporation du solvant, le produit est trituré dans l'éther, filtré, rincé à l'éther puis séché à 60°C sur P₂O₅. On obtient 0,3 g du produit attendu, F = 220°C (déc.).

RMN : 1,05 : d : 6H ; 1,5-2,2 : m : 12H ; 2,5 : s.e : 2H ; 2,6-2,8 : m : 3H ; 3,55 : mt : 2H ; 3,65 : s : 6H ; 3,8 : s : 1H ; 6,6 : d : 2H ; 6,7 : s : 1H ; 7,2-7,4 : d.d : 1H ; 7,8 : d : 1H.

### EXEMPLE 6

Chlorhydrate de l'acide 2-[5-(2,6-diméthoxyphényl)-1-[4-[N-(3-N',N'-diméthyl-aminopropyl)carbamoyl]-2-isopropylphényl]pyrazol-3-ylcarbonylamino]adamantane -2-carboxylique.

(I : R₁ = 4-CONH(CH₂)₃NMe₂ ; R₂ = 2-iPr ; R₃ = H ; R₄ = Me ; AA(OH) = 2-carboxyadamant -2-yl).

On prépare un mélange contenant 1,45 g du composé de la Préparation 4.3, 30 ml de DCM, 0,38 ml de chloroformiate d'isobutyle, 0,82 ml de triéthylamine et 17 ml d'acétonitrile. On laisse sous agitation à TA pendant 5 heures et demie. D'autre part on mélange 0,57 g d'acide 2-aminoadamantane-2-carboxylique, 10 ml d'acétonitrile et 2,2 ml de bis(triméthylsilyl)acétamide et on chauffe à reflux sous azote pendant 30 minutes. Après refroidissement on ajoute l'anhydride mixte formé précédemment et on laisse sous agitation à TA pendant 1 journée. On filtre et élimine l'insoluble ; évapore les solvants puis on ajoute de l'eau, agite 30 minutes et filtre le précipité formé. Une seconde fraction est obtenue à partir du filtrat après addition d'éthanol, extraction au DCM (2 fois), séchage sur MgSO₄ et évaporation des solvants. Les 2 fractions réunies sont chomatographiées sur silice en éluant par un mélange DCM/MeOH/H₂O (90/10/0,8 puis 88/12/1, v/v/v). On obtient 40 mg du produit attendu après trituration dans l'éther isopropylique, et filtration, F = 220°C (déc.).

RMN (DMSO + TFA) : 1,1 : d : 6H ; 1,5-2,2 : m : 14H ; 2,5 : s.e : 2H ; 2,75 : s+mt : 7H : 3,1 : m : 2H ; 3,3 : m : 2H ; 3,65 : s : 6H : 6,6 : d : 2H ; 6,7 : s : 1H ; 7,2-7,4 : m : 2H ; 7,6 : d.d : 1H ; 7,8 : d : 2H.

A partir des acides de formule (II) décrits dans le tableau 4 et en opérant selon le mode opératoire décrit à l'EXEMPLE 1, on obtient les composés selon l'invention de formule (I) décrits dans le tableau 6 ci-dessous.

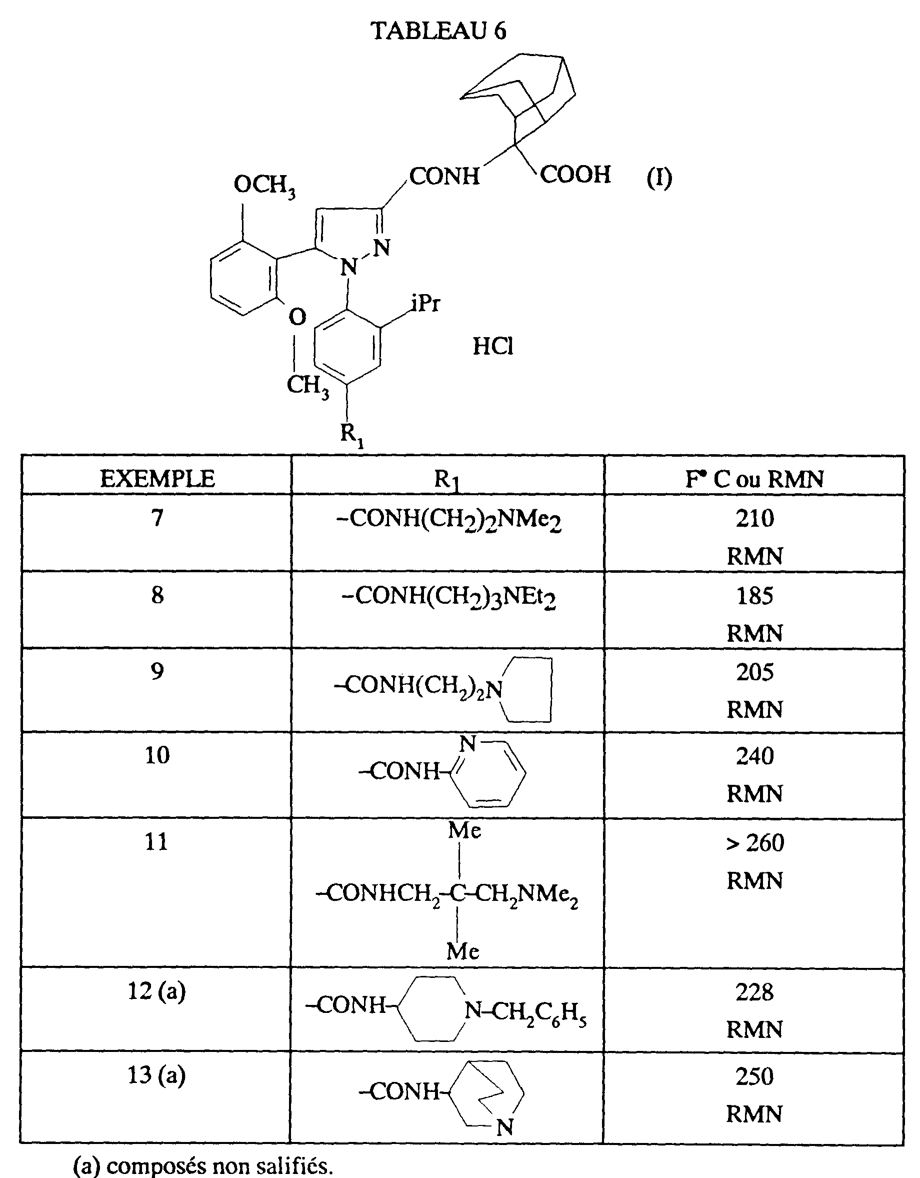

### RMN

EXEMPLE 7 (DMSO + TFA) : 1,1 : d : 6H ; 1,5-2,2 : m : 12H ; 2,4-2,8 : m : 3H ; 2,8 : s : 6H ; 3,25 : mt : 2H ; 3,5-3,7 : m : 8H ; 6,6 : d : 2H ; 6,7 : s : 1H ; 7,2-7,5 : m : 2H ; 7,7 : d.d : 1H ; 7,9 : d : 1H.

EXEMPLE 8 : 0,8-1,2 : m : 12H ; 1,4-2,2 : m : 14H ; 2,45 : s.e : 2H ; 2,6 : mt : 1H ; 2,8 : mt : 6H ; 3,3 : mt : 2H ; 3,5 : s : 6H ; 6,5 : d : 2H ; 6,65 : s : 1H ; 7,1-7,4 : m : 3H ; 7,6 : d.d : 1H ; 7,8 : s.e : 1H ; 8,7 : t : 1H.

EXEMPLE 9 (DMSO + TFA) : 0,8-1,3 : m : 8H ; 1,6-2,2 : m : 14H ; 2,3-2,8 : m : 3H ; 3 : mt : 2H ; 3,2-3,8 : m : 12H ; 6,6 : d : 2H ; 6,7 : s : 1H ; 7,2-7,4 : m : 2H ; 7,65 : d : 1H ; 7,9 : s.e : 1H.

EXEMPLE 10 : 1,05 : d : 6H ; 1,6-2,2 : m : 12H ; 2,5 : s.e : 2H ; 2,7 : mt ; 1H ; 3,6 : s : 6H ; 6,6 : d : 2H ; 6,7 : s : 1H ; 7,05-7,25 : m : 4H ; 7,8-8,2 : s : 1H ; 11 : s : 1H.

EXEMPLE 11 (DMSO + TFA) : 1,1-1,3 : m : 12H ; 1,6-2,2 : m : 12H ; 2,6 : s.e : 2H ; 2,7 : mt : 1H ; 2,9 : s : 6H ; 3,05 : s.e : 2H ; 3,3 : s.e : 2H ; 3,7 : s : 6H ; 6,65 : d : 2H ; 6,75 : s : 1H ; 7,25-7,45 : m : 3H ; 7,75 : d : 1H ; 7,95 : s.e : 1H.

EXEMPLE 12 : 1 : d : 6H ; 1,3-2,1 : m : 16H ; 2,6 : mt : 1H ; 2,75 : mt : 2H ; 3,1-3,8 : m : 5H ; 3,4 : s : 2H ; 3,6 : s : 6H ; 6,5 : d : 2H ; 6,6 : s : 1H ; 7,1-7,3 : m : 8H ; 7,5 : d : 1H ; 7,7 : s : 1H ; 8,2 : d : 1H.

EXEMPLE 13 (DMSO + TFA) : 1,05 : d : 6H ; 1,3-2,3 : m : 17H ; 2,3-2,6 : m : 2H ; 2,65 : mt : 1H ; 2,9-3,7 : m + s : 12 H ; 4,2 : m : 1H ; 6,4 : m : 2H ; 6,7 : se : 1H ; 7,0-7,3 : m : 2H ; 7,4-7,6 : m : 1H ; 7,7 : se : 1H.

### EXEMPLE 14 :

Chlorhydrate de l'acide 2-[5-(2,6-diméthoxyphényl)-1-[4-[N-méthyl-N-(3-N',N'-diméthylaminopropyl)aminosulfonyl]-2-isopropylphényl]pyrazol-3-yl-carbonylamino]adamantane-2-carboxylique.

(I : R₁ = 4-SO₂NMe(CH₂)₃NMe₂ ; R₂ = 2-iPr ; R₃ = H ; R₄ = CH₃ ; AA(OH) = 2-carboxyadamant-2-yl).

On laisse sous agitation à TA pendant 5 heures et demie 850 mg de l'acide obtenu à la Préparation 4.11 et 3 ml de chlorure de thionyle. On ajoute 10 ml de toluène puis le milieu réactionnel est évaporé sous vide (2 fois). On obtient ainsi 1 g de chlorure de l'acide obtenu à la préparation 4.11.On laisse sous agitation pendant 4 heures et demie un mélange contenant 0,41 g d'acide 2-aminoadamantane-2-carboxylique et 3,5 ml de bis(triméthylsilyl)acétamide dans 5 ml d'acétonitrile. A ce milieu réactionnel, on ajoute la solution du chlorure d'acide préparé ci-dessus dans 5 ml d'acétonitrile et 1 ml de triéthylamine et on laisse 4 jours sous agitation à TA. On ajoute 3 ml d'eau, 5 ml de méthanol et laisse sous agitation 4 heures à TA puis on filtre et évapore sous vide. Le résidu est trituré dans 6 ml d'HCl 1N puis on ajoute de l'éthanol et évapore sous vide. Le résidu est agité avec 200 ml de DCM et 5 ml d'eau, on décante, on sépare la phase organique, sèche sur Na₂SO₄ et évapore sous vide pour obtenir 1,26 g de produit brut.
On recristallise dans 5 ml de MeCN, refroidit à -20°C et filtre 0,6 g de produit attendu. F = 211°C.

RMN : 1 : d : 6H ; 1,4-2,1 : m : 14H ; 2,4-2,5 : mt : 2H ; 2,5-2,65 : mt : 1H ; 2,6 : s : 3H : 2,65 : s : 6H ; 2,9 : mt : 4H ; 3,5 : s : 6H ; 6,5 : d : 2H ; 6,7 : s : 1H ; 7,15-7,4 : m : 3H ; 7,45-7,6 : m : 2H.

### EXEMPLE 15

Acide 2-{5-(2,6-diméthoxyphényl)-1-[4-[N-[3-(N',N'-diméthylamino)propyl] carbamoyl]-5,6,7,8-tétrahydronapht-1-yl]pyrazol-3-ylcarbonylamino}adamantane-2-carboxylique.

(I : R₁ = 4-CONH(CH₂)₃NMe₂; R₂, R₃ = -(CH₂)₄-; R₄ = Me ; AA(OH) = 2-carboxyadamant-2-yl).

On chauffe 1 heure et demie à 40°C 0,47 g du composé obtenu à la Préparation 4.12 en solution dans 5 ml de SOCl₂ et 30 ml de DCM. On évapore sous vide pour obtenir le chlorure d'acide qui est redissous dans 5 ml d'acétonitrile et ajouté à la solution obtenue par reflux 2 heures d'un mélange de 0,28 g du composé B, 0,69 ml de bis(triméthylsilyl)acétamide et 3 ml d'acétonitrile. On ajoute 0,26 ml de triéthylamine et laisse sous agitation 2 heures à TA. On évapore sous vide, triture le résidu dans 2 ml d'une solution saturée de NaCl, filtre et sèche sous vide pour obtenir 0,77 g. On chromatographie sur silice H en éluant avec le mélange DCM/MeOH/eau (80/20/2,5; v/v/v). On évapore, triture dans l'éther et filtre pour obtenir 0,11 g de produit attendu. F = 200°C (gomme).

RMN :1,4-2,05 : m : 18H ; 2,1 : s : 6H ; 2,2 : t : 3H ; 2,4-2,8 : m : 6H ; 3,2 : qd : 2H ; 3,6 : s : 6H ; 6,6 : d : 2H ; 6,65 : s : 1H : 6,8-7 : d.d : 2H ; 7,2-7,3 : m : 2H ; 8,2 : t : 1H.

### EXEMPLE 16

Chlorhydrate de l'acide 2-{5-(2,6-diméthoxyphényl)-1-[4-[N-méthyl-N-(2-N',N'-diméthylaminoéthyl)aminosulfonyl]-5,6,7,8-tétrahydronapht-1-yl]pyrazol-3-ylcarbonylamino}adamantane-2-carboxylique.

(I : R₁ = 4-SO₂NMe(CH₂)₂NMe₂ ; R₂, R₃ = -(CH₂)₄- ; R₄ = CH₃ ; AA(OH) = 2-carboxyadamant-2-yl).

On laisse sous agitation 15 heures à TA 0,5 g du sel obtenu à la Préparation 4.13 dans 10 ml de SOCl₂ puis on évapore avec du toluène pour obtenir le chlorure d'acide sur lequel on verse une solution du composé B silylé obtenue par agitation 6 heures à TA d'un mélange de 0,279 g du composé B, 2 ml de bis(triméthylsilyl) acétamide et 8 ml d'acétonitrile. Après agitation 20 jours à TA, on évapore sous vide puis agite 1 heure à TA le résidu avec 5 ml d'eau et 5 ml de méthanol ; on filtre et évapore sous vide puis on chomatographie sur silice H, en éluant avec le mélange DCM/MeOH/AcOH. On triture le résidu dans l'éther et filtre pour obtenir 0,31 g du produit attendu. F > 260°C.

RMN(DMSO + TFA) : 1,6 - 2,3 : m : 16H ; 2,6 : m : 2H ; 2,9 : s.e : 9H ; 3,1 : mt : 2H ; 3,4 : mt : 2H ; 3,6 : mt : 2H ; 3,7 : s : 6H ; 6,7 : d : 2H ; 6,85 : s : 1H ; 7,15 : d : 1H ; 7,4 : t : 1H ; 7,5 : s.e : 1H ; 7,6 : d : 1H.

### EXEMPLE 17

Acide 2-[5-(2,6-diméthoxyphényl)-1-(2-méthyl-4-carbamoylphényl)pyrazol-3-yl-carbonylamino]adamantane-2-carboxylique.

(I : R₁ = 4-CONH₂ ; R₂ = 2-CH₃ ; R₃ = H ; R₄ = CH₃ ; AA(OH) = 2-carboxyadamant-2-yl). 1).

On chauffe à reflux pendant 1 heure une suspension de 220 mg de composé B et 0,4 mg de bis(triméthylsilyl)acétamide dans 10 ml d'acétonitrile.

Par ailleurs on prépare une solution contenant 330 mg du composé obtenu à la préparation 4.14 et 0,15 ml de triéthylamine dans 10 ml d'acétonitrile, on refroidit à -5°C, on ajoute 0,13 ml de chloroformiate d'isobutyle puis on laisse sous agitation 1 heure à TA et on ajoute l'anhydride mixte obtenu à la solution du composé B silylé préparée ci-dessus. On abandonne 8 jours à TA, puis on filtre l'insoluble, évapore à sec, puis on dissout le résidu dans 5 ml de DCM. On lave par une solution, de HCl 1,2 N, sèche sur Na₂SO₄ et évapore ; on ajoute 5 ml de DCM filtre le précipité formé puis on le dissout dans 1 ml de MeOH. On filtre les cristaux formés pour obtenir 100 mg du produit attendu, F = 290°C (déc.).

RMN : 1,4-2,3 : m : 15H ; 2,55 : m : 2H ; 3,6 : s ; 6H ; 6,6 : d : 2H ; 6,7 : s : 1H ; 7,05 : d : 1H ; 7,3 : t : 1H ; 7,4 : s : 2H ; 7,6 : d.d : 1H ; 7,75 : s : 1H ; 7,9 : s : 1H.

### EXEMPLE 18

Acide 2-{5-(2,6-diméthoxyphényl)-1-[2,3-diméthyl-4-[N-(2-N',N'-diméthyl aminoéthyl)carbamoyl]phényl]pyrazol-3-yl-carbonylamino}adamantane-2-carboxylique.

(I : R₁ = 4-CONH(CH₂)₂NMe₂ ; R₂ = 2-CH₃ ; R₃ = 3-CH₃ ; R₄ = CH₃ ; AA(OH) = 2-carboxyadamant-2yl).

On chauffe 1 heure et demie à reflux une solution de 1,3 g du produit de la Préparation 4.15 dans 10 ml de SOCl₂ et 50 ml de DCM. On évapore sous vide pour obtenir le chlorure d'acide qui est redissous dans 10 ml d'acétonitrile et ajouté à la solution obtenue après 2 heures de chauffage à reflux d'un mélange de 0,82 g du composé B, 2 ml de bis(triméthylsilyl)acétamide dans 10 ml d'acétonitrile. On ajoute 0,77 ml de triéthylamine et laisse sous agitation 15 heures à TA. Après évaporation sous vide, trituration dans 10 ml de solution saturée de NaCl, filtration et séchage sous vide, on chromatographie sur silice H, en éluant avec le mélange DCM/MeOH/eau (100/10/1 ; v/v/v). Après évaporation des solvants et trituration dans l'éther, on filtre pour obtenir 0,7 g de produit attendu. F = 210°C.

RMN : 1,6-2,2 : m : 12H ; 2 : s : 3H ; 2,25 : s : 3H ; 2,35 : s : 6H : 2,5-2,7 : m : 2H+2H ; 3,4 : qd : 2H ; 3,7 : s : 6H ; 6,65 : d : 2H ; 6,8 : s : 1H ; 7,0-7,2 : m : 2H ; 7,3-7,45 m : 2H ; 8,4 : t : 1H.

### EXEMPLE 19

Chlorhydrate de l'acide 2-{5-(2,6-diméthoxyphényl)-1-[4-[N-méthyl-N-(2-N',N'-diéthylaminoéthyl)carbamoyl]-2-méthoxyphényl]pyrazol-3yl-carbonylamino}adamantane-2-carboxylique.

(I : R₁ = 4-CONMe(CH₂)₂NEt₂ ; R₂ = 2-OCH₃ ; R₃ = H ; R₄ = CH₃ ; AA(OH) = 2-carboxyadamant-2yl).

On laisse sous agitation 24 heures à TA 1,2 g du produit de la Préparation 4.16 dans 12 ml de SOCl₂ et 12 ml de DCM. Après évaporation sous vide suivie de 2 évaporations azéotropiques avec 30 ml de toluène, le chlorure d'acide obtenu est redissous dans 10 ml d'acétonitrile et ajouté à la solution obtenue par reflux pendant 1 heure 15 minutes d'un mélange de 0,43 g de composé B, 1,1 ml bis(triméthylsilyl)acétamide dans 20 ml d'acétonitrile. On laisse sous agitation 4 heures à reflux, évapore sous vide, agite le résidu dans 4 ml de MeOH et 0,5 ml d'H₂O ; on évapore sous vide, puis on chromatographie sur silice H, en éluant avec les mélanges DCM/MeOH/NH₄OH 20 % (95/5/0,5 ; 90/10/0,5 ; 85/15/0,5 ; v/v/v) pour obtenir 0,3 g de produit attendu. F = 170°C (déc.).

RMN : 0,8 : mt : 3H ; 1 : mt : 3H ; 1,5-3,6 : mt : 34H ; 6,5 : d : 2H ; 6,65 : s : 1H ; 6,95 : mt : 2H ; 7,3 : mt : 3H.

### EXEMPLE 20

Chlorhydrate de l'acide 2-{5-(2,6-diméthoxyphényl)-1-[4-[N-méthyl-N-(2-N',N'-diéthylaminoéthyl)carbamoyl]-2-chlorophényl]pyrazol-3yl-carbonylamino}adamantane-2-carboxylique.

(I : R₁ = 4-CONMe(CH₂)₂NEt₂ ; R₂ = 2-Cl ; R₃ = H ; R₄ = CH₃ ; AA(OH) = 2-carboxyadamant-2yl).

On laisse sous agitation 24 heures à TA 1,3 g du produit de la Préparation 4.17 dans 12 ml de DCM et 12 ml de SOCl₂. Après évaporation sous vide suivie de 2 évaporations azéotropiques avec 30 ml de toluène, le chlorure d'acide obtenu est redissous dans 10 ml d'acétonitrile et ajouté à la solution obtenue par reflux pendant 1 heure 15 minutes d'un mélange de 0,46 g du composé B, 1,2 ml de bis(triméthylsilyl)acétamide dans 20 ml d'acétonitrile. On laisse sous agitation 4 heures à reflux puis on évapore sous vide, triture le résidu dans 4 ml de MeOH et 2 ml d'eau, agite 30 minutes à TA et évapore sous vide. On chromatographie le résidu sur silice H, en éluant avec le mélange DCM/MeOH/NH₄OH 20 % (80/20/0,5 ; v/v/v) pour obtenir 0,3 g de produit attendu. F = 160°C (déc.).

### EXEMPLE 21

Chlorhydrate de l'acide 2-{5-(2,6-diméthoxyphényl)-1-[4-[N-(2-morpholinoéthyl) carbamoyl]-2-chlorophényl]pyrazol-3yl-carbonylamino} adamantane-2-carboxylique. 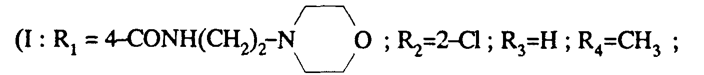 AA(OH) = 2-carboxyadamant-2-yl).

On laisse sous agitation 18 heures à TA un mélange de 1,4 g du produit de la Préparation 4.18, 14 ml de SOCl₂ et 80 ml de DCM. Après évaporation sous vide suivie de 2 évaporations azéotropiques avec 30 ml de toluène et redissolution du chlorure d'acide formé dans 20 ml d'acétonitrile on ajoute sur la solution obtenue par reflux pendant 4 heures d'une suspension de 0,53 g de composé B dans 1,33 ml de bis(triméthylsilyl)acétamide et 30 ml d'acétonitrile. Après agitation 4 heures à reflux on évapore sous vide, agite le résidu avec 10 ml de MeOH et 1 ml d'eau et filtre. Après agitation pendant 1 heure du précipité avec 5 ml d'H₂O et 5 gouttes d'HCl concentré, filtration, lavage avec 1 ml d'H₂O, 5 ml de pentane et 5 ml d'éther isopropylique on obtient 0,7 g de produit attendu. F = 200°C.

RMN : 1,5-2,2 : m : 12 ; 2,6 : s.e : 2 ; 2,85 : s.e : 4 ; 3,3-3,8 : mt : 20 ; 6,6 : d : 2H ; 6,75 : s: 1H ; 7,3 : t : 1H ; 7,45 : mt : 2H ; 7,8 : d.d : 1H ; 7,95 : d : 1H ; 8,85 : s.e : 1H.

### EXEMPLE 22

Acide 2-[5-(2,6-diméthoxyphényl)-1-(3-chloro-4-cyanophényl)pyrazol-3-yl-carbonylamino]adamantane-2-carboxylique.

(I : R₁ = 4-CN ; R₂ = 3-Cl ; R₃ = H ; R₄ = CH₃ ; AA(OH) = 2-carboxyadamant-2yl).

On laisse sous agitation à TA pendant 3 jours un mélange contenant 0,36 g du composé obtenu à la Préparation 4.19, 0,145 ml de chloroformiate d'isobutyle et 0,145 ml de triéthylamine dans 5 ml de DCM. Par ailleurs on porte à reflux pendant 1 heure un mélange contenant 0,23 g du composé B et 0,34 ml de bis(trifluorométhyl)acétamide dans 2 ml d'acétonitrile. On mélange les 2 solutions ainsi préparées et on laisse sous agitation à TA pendant 48 heures. Après filtration et lavage au méthanol, on évapore les solvants puis on chromatographie le résidu sur silice en éluant par le mélange DCM/MeOH/AcOH (100/1/0,5 ; v/v/v) pour obtenir 120 mg du produit attendu. F = 292°C.

### EXEMPLE 23

Acide 2-[5-(2,6-diméthoxyphényl)-1-(4-carbamoyl-3-chlorophényl)pyrazol-3-yl carbonylamino]adamantane-2-carboxylique.

(I : R₁ = 4-CONH₂ ; R₂ = 3-Cl ; R₃ = H ; R₄ = CH₃ ; AA(OH) = 2-carboxyadamant-2-yl).

On laisse sous agitation à TA pendant 24 heures un mélange contenant 0,73 g du composé obtenu à la Préparation 4.20, 0,263 ml de chloroformiate d'isobutyle et 0,26 ml de triéthylamine dans 5 ml de DCM.

Par ailleurs, on porte à reflux pendant 1 heure un mélange contenant 0,34 g de composé B et 0,65 ml de bis(triméthylsilyl)acétamide dans 2 ml d'acétonitrile. On mélange les 2 solutions ainsi préparées et on laisse sous agitation à TA pendant 4 jours. Après filtration, lavage par HCl 1N, puis EtOH et séchage sur MgSO₄, le résidu est chromatographié sur silice H en éluant par le mélange DCM/MeOH/AcOH (100/2/1 ; v/v/v). F = 293°C.

### EXEMPLE 24

Chlorhydrate de l'acide 2-{1-[4-[N-méthyl-N-(2-N',N'-diéthylaminoéthyl) carbamoyl]-2-cyclopropylphényl]-5-(2,6-diméthoxyphényl)pyrazol-3-ylcarbonylamino}adamantane-2-carboxylique.

(I : R₁ = 4-CONMe(CH₂)₂NEt₂ ; R₂ = 2-cyclopropyle ; R₃ = H ; R₄ = CH₃ ; AA(OH) = 2-carboxyadamant-2-yl).

On laisse sous agitation 24 heures à TA 0,5 g du produit de la Préparation 4.21 dans 5 ml de DCM et 1 ml de SOCl₂. Après évaporation sous vide suivie de 2 distillations azéotropiques avec 30 ml de toluène, on redissout le chlorure d'acide formé dans 5 ml d'acétonitrile, l'ajoute sur la solution obtenue par reflux pendant 2 heures et demie d'une suspension de 0,19 g de composé B dans 0,5 ml de bis(triméthylsilyl)acétamide et 8,5 ml d'acétonitrile et agite 12 heures à TA. On évapore sous vide, agite le résidu 45 minutes avec 3,5 ml de MeOH et 1 ml d'H₂O puis on ajoute goutte à goutte 2,5 ml d'H₂O et filtre. Après évaporation sous vide du filtrat, et séchage sous vide 24 heures à 60°C on obtient 0,14 g de produit attendu. F = 135°C (déc.).

RMN (DMSO + TFA) : 0,6 : m : 2H ; 0,9 : m : 2H ; 1,2 : m : 6H ; 1,5-2,2 : m : 12H ; 2,6 : m : 2H ; 2,9 : s.e : 3H ; 3,2 : m : 6H ; 3,6 : s : 6H ; 3,7 : m : 2H ; 6,6 : d : 2H ; 6,75 : s : 1H ; 6,85 : s : 1H ; 7,1-7,4 : m : 3H.

### EXEMPLE 25

Chlorhydrate de l'acide 2-{1-[3-[N-méthyl-N-(2-N'N'-diméthylamino-éthyl) carbamoyl]-4-chlorophényl]-5-(2,6-diméthoxyphényl)pyrazol-3-ylcarbonylamino}adamantane-2-carboxylique.

(I : R₁ = 3-CONMe(CH₂)₂NMe₂ ; R₂ = 4-Cl ; R₃ = H ; R₄ = CH₃ ; AA(OH) = 2-carboxyadamant-2-yl).

On laisse sous agitation 15 heures à TA une solution de 0,43 g du produit de la Préparation 4.22 dans 10 ml de DCM et 6 ml de SOCl₂. Après évaporation sous vide suivie de 2 distillations azéotropiques avec 30 ml de toluène, on redissout le chlorure d'acide dans 3 ml d'acétonitrile, ajoute sur la solution obtenue par reflux 3 heures d'une suspension de 0,17 g du composé B dans 0,5 ml de bis(triméthylsilyl)acétamide et 10 ml d'acétonitrile. On agite 3 heures à reflux puis 15 heures à TA. On évapore sous vide, agite 1 heure le résidu avec 12 ml de MeOH et 6 ml d'eau. On évapore le MeOH sous vide, extrait 2 fois avec 50 ml de DCM, sèche la phase organique sur Na₂SO₄ et évapore sous vide pour obtenir 0,16 g de produit attendu. F = 206°C (Déc.).

RMN : 1,5-2,3 : m : 12H ; 2,6-3,8 : m : 21H ; 6,7 : d : 2H ; 6,8 : s : 1H ; 7,1-7,7 : m : 4H.

### EXEMPLE 26

Acide 2-{1-[5-[N-méthyl-N-(3-N',N'-diméthylaminopropyl)carbamoyl]-2-méthylphényl]-5-(2,6-diméthoxyphényl)pyrazol-3-ylcarbonylamino}adamantane-2-carboxylique.

(I : R₁ = 5-CONMe(CH₂)₃NMe₂ ; R₂ = 2-CH₃ ; R₃ = H ; R₄ = CH₃ ; AA(OH) = 2-carboxyadamant-2-yl).

On laisse sous agitation 5 heures et demie à TA une solution de 1,7 g du produit de la préparation 4.23 dans 15 ml de SOCl₂. Après évaporation sous vide suivie de 3 distillations azéotropiques avec 30 ml de DCM on redissout le chlorure d'acide formé dans 30 ml d'acétonitrile, ajoute sur la solution obtenue par reflux 1 heure d'une suspension de 0,69 g de composé B dans 1,75 ml de bis(triméthylsilyl)acétamide et 6 ml d'acétonitrile. Après agitation 15 heures à TA et évaporation sous vide, on dissout le résidu dans 13 ml de MeOH, ajoute lentement 12 ml d'eau et agite 30 minutes à TA. Après évaporation sous vide, trituration du résidu dans 5 ml d'HCl N, décantation, 3 extractions de la gomme résiduelle avec 100 ml de DCM, séchage sur MgSO₄, on évapore sous vide puis dissout le résidu dans 15 ml d'eau. On alcalinise avec NaOH 30 % jusqu'à pH 8, puis cristallise sous ultrasons. Après filtration, on recristallise dans le toluène et sèche sous vide à 60°C pour obtenir 1,32 g de produit attendu. F = 165°C.

RMN (DMSO + TFA) : 1,6-2,2 : m : 14H ; 2,2 : s : 3H ; 2,5-3,2 : m : 13H ; 3,5 : mt : 2H ; 3,7 : s : 6H ; 6,65 : d : 2H ; 6,8 : s : 1H ; 7,3-7,6 : m : 3H.

A partir des acides II décrits dans le tableau 5 ou dans les Préparations, en suivant les modes opératoires énoncés ci-dessus, on prépare les composés selon l'invention rassemblés dans le tableau 7 ci-après.

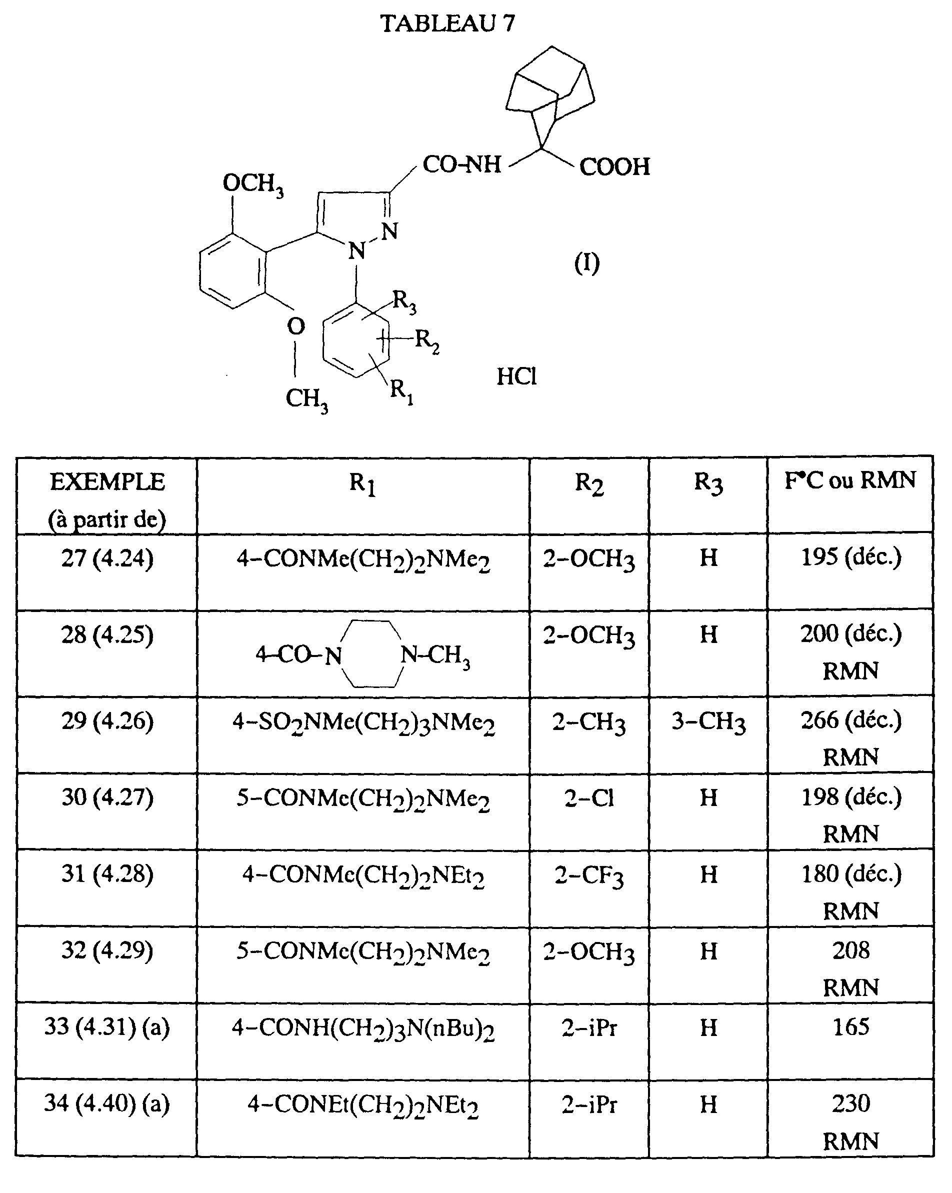

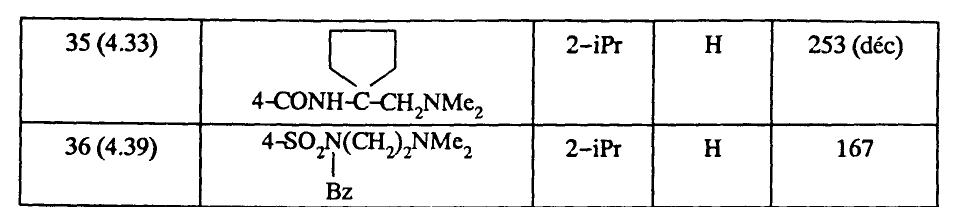

### (a) composé non salifié

### RMN:

EXEMPLE 28 : 1,3-3,7 : mt : 26H ; 6,55 : d : 2H ; 6,65 : s : 1H ; 6,9 : mt : 3H ; 7,15-7,5 : mt : 4H.

EXEMPLE 29 : 1,5-2,3 : m : 17H ; 2,55 : s : 2H ; 2,8 : s : 9H ; 2,95-3,3 : m 4H ; 3,6 : s : 6H ; 6,6 : d : 2H ; 6,7 : s : 1H ; 7-7,7 : m : 3H.

EXEMPLE 30 : 1,4-2,3 : m : 12H ; 2,6 : s.e : 2H ; 2,7 : s.e : 3H ; 2,8 : s.e : 6H ; 3,4 : m : 2H ; 3,6 : s : 6H ; 3,8 : m : 2H ; 6,6 : d : 2H ; 6,82 : s : 1H ; 7,2-7,8 : m : 5H ; 13,0 : s.e : 1H.

EXEMPLE 31 (DMSO, TFA) : 1,2 : t : 6H ; 1,5-2,2 : m : 12H ; 2,5 : s.e : 2H ; 2,9 : s : 3H ; 3,1-3,5 : m : 9H ; 3,6 : s : 6H ; 3,8 : m : 2H ; 6,6 : d : 2H ; 6,8 : s : 1H ; 7,3 : m : 3H ; 7,7 : d : 1H ; 8 : s : 1H.

EXEMPLE 32 : 1,6-2,2 : m : 12H ; 2,45 : s : 6H ; 2,60 : s.e : 2H ; 2,85 : s : 3H ; 3,40 : m : 2H ; 3,56 : s : 3H ; 3,60 : s : 6H ; 3,70 : m : 2H ; 6,60 : d : 2H ; 6,78 : s : 1H ; 7,00 : t : 1H ; 7,20 : m : 3H ; 7,40 : s.e : 1H.

EXEMPLE 34 (DMSO + TFA) : 0,9-1,3 : m : 15H ; 1,6-2,2 : m : 8H ; 2,7 : m : 1H ; 3,1-3,4 : m : 8H ; 3,6-3,8 : m : 8H ; 6,55 : d : 2H ; 6,75 : s : 1H ; 7,1-7,4 : m : 4H.

### EXEMPLE 37

Hydrogénosulfate de l'acide 2-[5-(2,6-diméthoxyphényl)-1-[4-[N-méthyl-N-(3-diméthylaminopropyl)carbamoyl]-2-isopropylphényl]pyrazol-3-yl-carbonylamino] adamantane-2-carboxylique.

A une solution de 0,08 g d'H₂SO₄ dans 5 ml de MeOH on ajoute à TA 0,5 g du composé obtenu à l'EXEMPLE 1', verse cette solution sur 150 ml d'éther refroidi à 5°C et essore le précipité formé. On obtient 0,54 g du produit attendu. Après recristallisation dans l'eau, F = 212°C (déc). Après recristallisation dans l'isopropan-2-ol, F = 263°C.

RMN (DMSO + TFA) : 1,1 : d : 6H ; 1,5-2,2 : m : 14H ; 2,5 : se : 2H ; 2,6-3 : m : 10H ; 3,1 : mt : 2H ; 3,5 : mt : 2H ; 3,65 : s : 6H ; 6,6 : d : 2H ; 6,75 : s : 1H ; 7,1-7,5 : m : 4H.

On peut aussi préparer le produit de l'EXEMPLE 37 selon le mode opératoire ci-dessous :

A une suspension de 3,4 g de sel interne de l'EXEMPLE 1' dans 34 ml d'eau on ajoute lentement avec agitation, 22 ml d'H₂SO₄ concentré et chauffe à 40° jusqu'à l'obtention d'un changement d'aspect de la suspension. On laisse refroidir à TA pendant 4 heures sous agitation, filtre et sèche pour obtenir 3,8 g d'hydrogénosulfate attendu.

### EXEMPLE 38

Benzènesulfonate de l'acide 2-[5-(2,6-diméthoxyphényl)-1-[4-[N-méthyl-N-(3-diméthylaminopropyl)carbamoyl]-2-isopropylphényl]pyrazol-3-yl-carbonylamino) adamantane-2-carboxylique.

On verse le mélange de 0,5 g du composé obtenu à l'EXEMPLE 1' et 0,16 g d'acide benzènesulfonique dans 5 ml de MeOH sur 75 ml d'éther refroidi à 5°C et essore le précipité formé. On obtient 0,06 g du produit attendu, F = 170°C (déc).

RMN (DMSO + TFA) : 1 : d : 6H ; 1,4-2,2 : m : 14H ; 2,45 : se : 2H ; 2,5-3,2 : m : 12 H ; 3,4 : mt : 2H ; 3,55 : s : 6H ; 6,5 : d : 2H ; 6,65 : s : 1H ; 7-7,4 : m : 7H ; 7,5 : mt : 2H.

### EXEMPLE 39

Citrate de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

A une solution de 0,3 g du composé obtenu à l'EXEMPLE 1' dans 5 ml d'EtOH et 3 ml de DCM, on ajoute à TA 0,084 g d'acide citrique et laisse 2 heures sous agitation à TA. On concentre sous vide et recristallise le résidu dans le propan-2-ol. On obtient 0,26 g du produit attendu, F = 168°C.

RMN (DMSO + TFA) : 1,05 : d : 6H ; 1,5-2,3 : m : 14H ; 2,5 : se : 2H ; 2,6-3,3 : m : 16H ; 3,3-3,8 : s + m : 8H ; 6,6 : d : 2H ; 6,75 : s : 1H ; 7,1-7,5 : m : 4H.

### EXEMPLE 40

Maléate de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On dissout à chaud 0,1 g du composé obtenu à l'EXEMPLE 1' et 0,017 g d'acide maléique dans 2,3 ml de propan-2-ol et concentre sous vide. On dissout le résidu dans 0,3 ml d'EtOH, verse cette solution sur 30 ml d'éther et essore le précipité formé. On obtient 0,04 g du produit attendu, F = 260°C (déc).

RMN (DMSO + TFA) : 1,05 : d : 6H ; 1,55-2,2 : m : 14H ; 2,5 : se : 2H ; 2,6-3 : m : 10H ; 3,5 : mt : 2H ; 3,65 : s : 6H ; 6,3 : s : 2H ; 6,6 : d : 2H ; 6,75 : s : 1H ; 7,15-7,45 : m : 4H.

### EXEMPLE 41

Sel de (S)-(+)-arginine de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On dissout à chaud 0,1 g du composé obtenu à l'EXEMPLE 1' et 0,03 g de (S)-(+)-arginine dans 4 ml de MeOH, concentre cette solution à 1 ml et verse sur 10 ml d'éther refroidi à 5°C. On obtient 0,055 g du produit attendu après essorage et séchage sur P₂O₅, F = 176°C (déc).

RMN (DMSO + TFA) : 1,05 : d : 6H ; 1,5-2,2 : m : 20H ; 2,55 : se : 2H ; 2,6-3,55 : m : 16H ; 3,65 : s : 6H ; 3,95 : t : 1H ; 6,6 : d : 2H ; 6,75 : s : 1H ; 7,15-7,4 : m : 4H.

### EXEMPLE 42

Edisylate de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

### A) Acide 1,2-éthanedisulfonique.

On introduit lentement une solution de 3 g de sel disodique de l'acide 1,2-éthanedisulfonique dans 10 ml d'eau sur 200 ml de résine Dowex® 50W X 8 puis élue avec 200 ml d'eau déminéralisée. On dilue l'éluat par ajout d'EtOH et concentre sous vide. On obtient 3,35 g du produit attendu sous forme d'huile qui cristallise à TA.
B) Edisylate de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On dissout à chaud 0,05 g du composé obtenu à l'EXEMPLE 1' et 0,04 g du composé obtenu à l'étape A dans 2 ml de propan-2-ol et concentre sous vide. On dissout le résidu dans 0,3 ml d'eau et 8 gouttes de dioxane et laisse en cristallisation à TA. On essore le produit cristallisé formé, le lave à l'eau et sèche à 90°C sous vide. On obtient 0,042 g du produit attendu, F = 266°C (déc).

RMN (DMSO + TFA) : 1,05 : d : 6H ; 1,5-2,2 : m : 14H ; 2,5 : se : 2H ; 2,6-3 : m + s : 14H ; 3,1 : mt : 2H ; 3,5 : mt : 2H ; 3,65 : s : 6H ; 6,6 : d : 2H ; 6,75 : s : 1H ; 7,15-7,45 : m : 4H.

### EXEMPLE 43

Sel de sodium de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On dissout 0,206 g du composé obtenu à l'EXEMPLE 1' et 0,026 g de méthylate de sodium dans 1 ml de MeOH et quelques gouttes de DCM puis verse cette solution sur 50 ml d'éther refroidi à 5°C. On essore le précipité gélatineux formé et le sèche sur P₂O₅ et sous vide. On obtient 0,15 g du produit attendu, F = 191°C.

On peut également obtenir ce composé en suivant le mode opératoire décrit ci-dessous.

A une solution de 0,1 g du composé obtenu à l'EXEMPLE 1' dans 5 ml de MeOH et 4 ml de DCM on ajoute 0,7 ml d'une solution de 0,104 g de NaOH dans 10 ml de MeOH et concentre sous vide. On dissout le résidu dans 1 ml de propan-2-ol, verse cette solution sur 75 ml d'éther refroidi à 5°C et essore le précipité formé. On obtient 0,005 g du produit attendu.

RMN : 1 : d : 6H ; 1,4-2,3 : m : 20H ; 2,55 : se : 2H ; 2,6 : mt : 1H ; 2,85 : d : 3H ; 3,1 et 3,4 : 2mt : 4H ; 3,6 : s : 6H ; 6,55 : s : 1H ; 6,6 : s : 2H ; 6,95 : s : 1H ; 7-7,35 : m : 4H.

Le spectre RMN enregistré en présence de DMSO + TFA est légèrement différent.

RMN (DMSO + TFA) : 1,05 : d : 6H ; 1,5-2,3 : m : 14H ; 2,5 : se : 2H ; 2,6-3 : m : 10H ; 3,1 : mt : 2H ; 3,5 : mt : 6H ; 6,6 : d : 2H ; 6,75 : s : 1H ; 7,1-7,4 : m : 4H.

### EXEMPLE 44

Fumarate de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On dissout 0,1 g du composé obtenu à l'EXEMPLE 1' et 0,017 g d'acide fumarique dans 1,5 ml d'EtOH, 1,5 ml de DCM et 4 ml de MeOH et laisse 10 minutes sous agitation à TA. On concentre partiellement sous vide et laisse cristalliser. On obtient 0,025 g du produit attendu après essorage et lavage à l'EtOH, F = 243°C.

RMN (DMSO + TFA) : 1 : d : 6H ; 1,5-2,3 : m : 14H ; 2,5 : se : 2H ; 2,6-3 : m : 10H ; 3,1 : mt : 2H ; 3,4 : mt : 2H ; 3,6 : s : 6H ; 6,5-6,7 : d + s : 3H ; 6,75 : s : 1H ; 7,1-7,4 : m : 5H.

### EXEMPLE 45

Sel de N-méthyl-(D)-glucamine de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On chauffe à reflux une solution de 0,07 g du composé obtenu à l'EXEMPLE 1' dans 5 ml d'EtOH et 1 ml de DCM, ajoute 0,02 g de N-méthyl-(D)-glucamine et laisse 1 heure 30 minutes sous agitation à TA. On concentre partiellement sous vide, verse sur 15 ml d'éther et essore le précipité formé. On obtient 0,032 g du produit attendu, F = 90°C (gomme).

RMN (DMSO + TFA) : 1 : d : 6H ; 1,5-2,2 : m : 14H ; 2,5-2,6 : mt : 5H ; 2,6-3,2 : m : 14H ; 3,2-3,7 : m : 13H ; 3,85 : mt : 1H ; 6,6 : d : 2H ; 6,7 : s : 1H ; 7,1-7,4 : m : 4H.

### EXEMPLE 46

Sel de diéthanolamine de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On dissout 0,1 g du composé obtenu à l'EXEMPLE 1' dans 1,5 ml d'EtOH et 1,5 ml de DCM, ajoute 0,015 g de diéthanolamine, laisse 30 minutes sous agitation à TA et abandonne une nuit à 5°C. On essore le produit cristallisé formé. On obtient 0,03 g du produit attendu, F = 200°C (déc).

RMN (DMSO + TFA) : 1,05 : d : 6H ; 1,5-2,2 : m : 14H ; 2,5 : se : 2H ; 2,6-3,25 : m : 16H ; 3,3-3,8 : m + s : 12H ; 6,6 : d : 2H ; 6,7 : s : 1H ; 7,1-7,4 : m : 4H.

### EXEMPLE 47

(L)(+)-Tartrate de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On chauffe à reflux un mélange de 0,1 g du composé obtenu à l'EXEMPLE 1' et 0,022 g d'acide (L)(+)-tartrique dans 1,5 ml d'EtOH et 1,5 ml de DCM puis on ajoute 8 ml d'EtOH et poursuit le reflux pendant 5 minutes. Après refroidissement à TA, on concentre partiellement sous vide, verse sur 10 ml d'éther et essore le précipité formé. On obtient 0,07 g du produit attendu après séchage sur P₂O₅, F = 154°C (déc).

RMN (DMSO + TFA) : 1,05 : d : 6H ; 1,5-2,4 : m : 14H ; 2,55 : se : 2H ; 2,6-3 : m : 10H ; 3,1 : mt : 2H ; 3,5 : mt : 2H ; 3,65 : s : 6H ; 4,35 : s : 2H ; 6,6 : d : 2H ; 6,75 : s : 1H ; 7,15-7,5 : m : 4H.

### EXEMPLE 48

Sel de choline de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On laisse 15 minutes sous agitation à 35°C une solution de 1 ml de DCM contenant de 0,05 g du composé obtenu à l'EXEMPLE 1' et 0,025 ml d'une solution à 45 % d'hydroxyde de choline dans le MeOH, puis concentre sous vide. On triture le résidu dans 5 ml d'éther et essore le précipité formé. On obtient 0,03 g du produit attendu, F = 150°C.

RMN (DMSO + TFA) : 1,05 : d : 6H ; 1,4-2,2 : m : 14H ; 2,5 : se : 2H ; 2,4-3 : m : 10H ; 3,1 : se : 11H ; 3,4 : mt : 2H ; 3,5 : mt : 2H ; 3,6 : s : 6H ; 3,8 : mt : 2H ; 6,6 : d : 2H ; 6,7 : s : 1H ; 7,1-7,4 : m : 4H.

### EXEMPLE 49

Iséthionate de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On chauffe à reflux un mélange de 0,1 g du composé obtenu à l'EXEMPLE 1' dans 3 ml de propan-2-ol, ajoute 0,022 g d'acide iséthionique à 83 % (obtenu par élution d'iséthionate de sodium sur résine DOWEX® 50W X 8 sous forme H⁺) et laisse une nuit en cristallisation. On essore le produit cristallisé formé. On obtient 0,055 g du produit attendu, F = 230°C.

RMN (DMSO + TFA) : 1,05 : d : 6H ; 1,55 : m : 14H ; 2,55 : se : 2H ; 2,6-3,05 : m : 12H ; 3,1 : mt : 2H ; 3,5 : mt : 2H ; 3,6-3,7 : s + mt : 8H ; 6,6 : d : 2H ; 6,75 : s : 1H ; 7,15-7,45 : m : 4H.

### EXEMPLE 50

Sel de potassium de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On laisse une nuit à TA une solution de 0,15 g du composé obtenu à l'EXEMPLE 1' et 0,03 g de *tert*-butylate de potassium dans 6,5 ml de propan-2-ol puis concentre sous vide. On dissout le résidu dans 0,5 ml de MeOH et verse cette solution sur 25 ml d'éther isopropylique refroidi à -20°C. On essore le précipité formé et le sèche sur P₂O₅ à 80°C. On obtient 0,09 g du produit attendu, F = 222°C.

On peut également obtenir ce composé en suivant le mode opératoire décrit ci-dessous.

A une solution de 0,1 g du composé obtenu à l'EXEMPLE 1' dans 4 ml de DCM, on ajoute 1 ml d'une solution de 0,129 g de KOH dans 10 ml de MeOH puis concentre sous vide. On dissout le résidu dans 0,5 ml de propan-2-ol, verse cette solution sur 75 ml d'éther refroidi à 5°C et essore le précipité formé. On obtient 0,015 g du produit attendu.

RMN (DMSO + TFA) : 1,05 : d : 6H ; 1,5-2,3 : m : 14H ; 2,5 : se : 2H ; 2,6-3 : m : 10H ; 3,1 : t : 2H ; 3,5 : t : 2H ; 3,6 : s : 6H ; 6,6 : d : 2H ; 6,7 : s : 1H ; 7,1-7,5 : m : 4H.

### EXEMPLE 51

Dihydrogénophosphate de l'acide 2-[5-(2,6-diméthoxyphényl-1-[4-[N-méthyl-N-(3-diméthylaminopropyl)carbamoyl]-2-isopropylphényl]pyrazol-3-yl-carbonylamino] adamantane-2-carboxylique.

On laisse 1 heure sous agitation à TA un mélange de 0,1 g du composé obtenu à l'EXEMPLE 1' et 0,017 g d'acide orthophosphorique à 85 % dans 2 ml de DCM et 3 ml d'EtOH. On concentre partiellement sous vide, verse sur 10 ml d'éther refroidi à 5°C et essore le précipité formé. On obtient 0,04 g du produit attendu après séchage à 60°C.

RMN (DMSO + TFA) : 1,05 : d : 6H ; 1,5-2,2 : m : 14H ; 2,5 : se : 2H ; 2,6-3,3 : m : 12 H ; 3,5 : mt : 2H ; 3,6 : s : 6H ; 6,6 : d : 2H ; 6,7 : s : 1H ; 7,1-7,4 : m : 4H.

### EXEMPLE 52

2-Naphtalènesulfonate de l'acide 2-[5-(2,6-diméthoxyphényl)-1-[4-[N-méthyl-N-(3-diméthylaminopropyl)carbamoyl]-2-isopropylphényl]pyrazol-3-yl-carbonylamino] adamantane-2-carboxylique.

On précipite une solution de 0,5 g du composé obtenu à l'EXEMPLE 1' et 0,16 g d'acide 2-naphtalènesulfonique dans 5 ml de MeOH, sur 25 ml d'éther refroidi à 5°C, essore le précipité formé et conserve le filtrat. On dissout le précipité dans 2 ml de MeOH, verse cette solution sur 50 ml d'éther refroidi à 5°C, essore le précipité formé et obtient 0,2 g du produit attendu. On précipite le premier filtrat sur 50 ml d'éther refroidi à 5°C, essore le précipité formé et obtient 0,27 g de second jet du produit attendu.

RMN (DMSO + TFA) : 1,05 : d : 6H ; 1,5-2,2 : m : 14H ; 2,5 : se : 2H ; 2,6-3 : m : 10H ; 3,1 : mt : 2H ; 3,5 : mt : 2H ; 3,65 : s : 6H ; 6,6 : d : 2H ; 6,7 : s : 1H ; 7,1-7,6 : m : 7H ; 7,7 : d : 1H ; 7,8-8,1 : m : 2H ; 8,2 : s : 1H.

### EXEMPLE 53

Acide 2-[5-(2,6-diméthoxyphényl)-1-[4-[N-(2-diisopropylaminoéthyl) carbamoyl]-2-isopropylphényl]pyrazol-3-ylcarbonylamino]adamantane-2-carboxylique.

(I : R₁ = 4-CONH(CH₂)₂N(iPr)₂ ; R₂ = 2-iPr ; R₃ = H ; R₄ = CH₃ ; AA(OH) = 2-carboxyadamant-2-yl).

On laisse 4 heures sous agitation à TA un mélange de 0,53 g du composé obtenu à la Préparation 4.30 et 2 ml de SOCl₂, On concentre sous vide, reprend le résidu au DCM et évapore sous vide, reprend le résidu au DCM et évapore sous vide le solvant. Le chlorure d'acide ainsi obtenu est utilisé tel quel. D'autre part on chauffe à reflux, sous atmosphère d'azote, pendant 35 minutes un mélange de 0,19 g du composé B et 0,49 ml de bis(triméthylsilyl)acétamide dans 2 ml d'acétonitrile. Après refroidissement à TA, on ajoute une solution du chlorure d'acide préparé ci-dessus dans 8 ml d'acétonitrile et laisse une nuit sous agitation à TA. On évapore sous vide, agite le résidu avec 8,8 ml de MeOH, ajoute 8,8 ml d'eau et évapore sous vide. On traite le résidu par une solution d'HCl 1,2N et filtre le précipité formé. On traite le précipité par 10 ml d'eau, alcalinise jusqu'à pH = 8 par ajout d'une solution de NaOH 1,3N, essore le précipité et le lave à l'eau. On obtient 0,475 g du produit attendu après cristallisation à chaud dans 40 ml d'acétonitrile, F = 196-198°C.

RMN (DMSO + TFA) : 1,1 : d : 6H ; 1,3 : d : 12H ; 1,6-2,2 : m : 12H ; 2,55 : m : 2H ; 2,7 : mt : 1H ; 3,2 : m: 2H ; 3,5-3,8 : m + s : 10H ; 6,6 : d : 2H ; 6,75 : s : 1H ; 7,2-7,4 : mt : 2H ; 7,65 : d : 1H ; 7,85 : s : 1H.

### EXEMPLE 54

Acide 2-[5-(2,6-diméthoxyphényl)-1-[4-[N,N-bis(2-diéthylaminoéthyl) carbamoyl]-2-isopropylphényl]pyrazol-3-ylcarbonylamino]adamantane-2-carboxylique.

(I : R₁ = 4-CON(CH₂CH₂NEt₂)₂ ; R₂ = 2-iPr ; R₃ = H ; R₄ = CH₃ ; AA(OH) = 2-carboxyadamant-2-yl).

On laisse 24 heures sous agitation à TA un mélange de 0,4 g du composé obtenu à la Préparation 4.34 et 2,5 ml de SOCl₂. On concentre sous vide, reprend le résidu au toluène et évapore sous vide le solvant. On utilise tel quel le chlorure d'acide ainsi obtenu. D'autre part on chauffe à reflux pendant 45 minutes un mélange de 0,18 g du composé B et 0,5 ml de bis(triméthylsilyl)acétamide dans 4 ml d'acétonitrile. Après refroidissement à TA, on ajoute une solution du chlorure d'acide préparé ci-dessus dans 3 ml d'acétonitrile et laisse 72 heures sous agitation à TA. On ajoute 3 ml de MeOH et concentre sous vide le mélange réactionnel. On dissout le résidu dans 3 ml d'HCl 1,2N, lave trois fois à l'AcOEt, neutralise la phase aqueuse à pH = 6 par ajout d'une solution concentrée de NaOH et décante le produit gommeux formé. On chromatographie le produit gommeux sur silice H en éluant par le mélange DCM/MeOH/NH₄OH (75/25/1,2 ; v/v/v). On obtient 0,4 g du produit attendu après trituration dans l'éther, F = 169°C.

### EXEMPLE 55

Chlorhydrate de l'acide 2-[5-(2,6-diméthoxyphényl)-1-[4-[N-(pipérid-4-yl) carbamoyl]-2-isopropylphényl]pyrazol-3-ylcarbonylamino]adamantane-2-carboxylique. 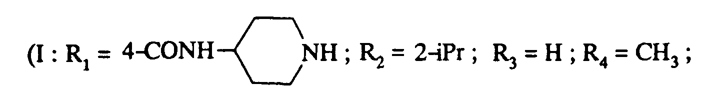 AA(OH) = 2-carboxyadamant-2-yl).

On hydrogène pendant 5 jours à TA puis 4 jours à 50°C, à pression atmosphérique, un mélange de 0,3 g du composé obtenu à l'EXEMPLE 12, 0,05 g de palladium sur charbon à 10 % et 0,033 ml d'HCl concentré dans 10 ml de MeOH et 4 ml de DMF. On filtre le catalyseur sur Célite® et évapore sous vide le filtrat. On reprend le résidu à l'éther et essore le produit cristallisé formé. On obtient 0,121 g du produit attendu après séchage sur P₂O₅ à 70°C sous vide, F = 252°C.

RMN (DMSO + TFA) : 1,1 : d : 6H ; 1,5-2,2 : m : 16H ; 2,4-3,5 : 3mt + se : 7H ; 3,6 : s : 6H ; 3,9-4,15 : m : 1H ; 6,6 : d : 2H ; 6,7 : s : 1H ; 7,1-7,4 : mt : 2H ; 7,6 : d : 1H ; 7,8 : s : 1H.

### EXEMPLE 56

Acide 2-[5-(2,6-diméthoxyphényl)-1-[4-[N-(1-éthylpyrrolidin-2-yl)méthyl] carbamoyl]-2-isopropylphényl]pyrazol-3-ylcarbonylamino]adamantane-2-carboxylique. 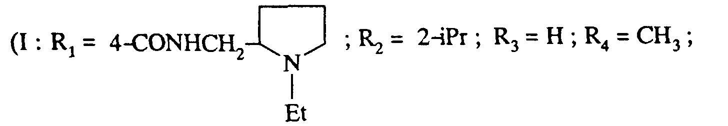 AA(OH) = 2-carboxyadamant-2-yl).

On prépare ce composé selon le mode opératoire décrit à l'EXEMPLE 53 à partir de 0,48 g du composé obtenu à la Préparation 4.35 et 2 ml de SOCl₂ puis 0,18 g de composé B et 0,46 ml de bis(triméthylsilyl)acétamide dans 2 ml d'acétonitrile. On obtient 0,2 g du produit attendu après recristallisation dans le propan-2-ol, F = 212°C (déc).

RMN (DMSO + TFA) : 0;9 : se : 6H ; 1,05 : m : 3H ; 1,3-2,1 : m : 14H ; 2,3 : se : 2H ; 2,5 : m : 1H ; 2,9 : m : 2H ; 3,2-3,6 : m + s : 11H ; 6,4 : d : 2H ; 6,5 : se : 1H ; 7-7,3 : m : 2H ; 7,45 : d : 1H ; 7,65 : se : 1H.

### EXEMPLE 57

Acide 2-[5-(2,6-diméthoxyphényl)-1-[4-[N-(2,2,6,6-tétraméthylpipérid-4-yl) carbamoyl]-2-isopropylphényl]pyrazol-3-ylcarbonylamino]adamantane-2-carboxylique. 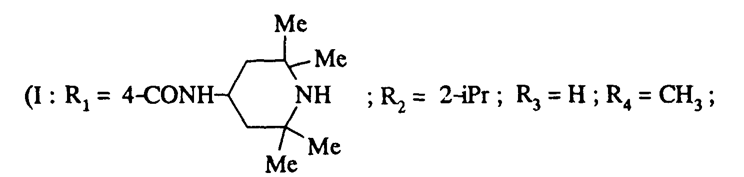 AA(OH) = 2-carboxyadamant-2-yl).

On prépare ce composé selon le mode opératoire décrit à l'EXEMPLE 53 à partir de 0,43 g du composé obtenu à la Préparation 4.36 et 2 ml de SOCl₂ puis 0,15 g du composé B et 0,39 ml de bis(triméthylsilyl)acétamide dans 2 ml d'acétonitrile. Après agitation du mélange réactionnel une nuit à TA, on essore le précipité formé et la lave à l'acétonitrile. On reprend le précipité dans 4 ml de MeOH, ajoute progressivement 4 ml d'eau et concentre sous vide. On reprend le résidu par une solution d'HCl 1,2N et essore le précipité après trituration. On reprend le précipité dans 3 ml d'eau, alcalinise jusqu'à pH = 9 par ajout d'une solution de NaOH 1,3N, essore le précipité formé et le lave à l'eau. On obtient 0,24 g du produit attendu après séchage sur P₂O₅, F = 270-272°C.

RMN (DMSO + TFA) :1,5 : d : 6H ; 1,3 : s : 6H ; 1,4 : s : 6H ; 1,5-2,2 : 2m : 16H ; 2,65 : mt : 1H ; 3,6 : s : 6H ; 4,2-4,4 : m : 1H ; 6,55 : d : 2H ; 6,7 : s : 1H ; 7,1-7,4 : m : 2H ; 7,6 : d : 1H ; 7,8 : s : 1H.

### EXEMPLE 58

Acide 2-[5-(2,6-diméthoxyphényl)-1-[4-[[3-(diéthylamino)pyrrolidin-1-yl] carbonyl]-2-isopropylphényl]pyrazol-3-ylcarbonylamino]adamantane-2-carboxylique. 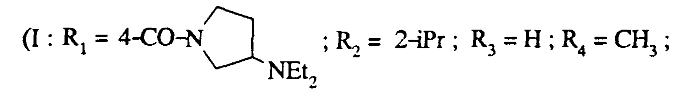 AA(OH) = 2-carboxyadamant-2-yl).

On prépare ce composé selon le mode opératoire décrit à l'EXEMPLE 53 à partir de 0,39 g du composé obtenu à la Préparation 4.41 et 2 ml de SOCl₂ puis 0,13 g du composé B et 0,34 ml de bis(triméthylsilyl)acétamide dans 2 ml d'acétonitrile. Après agitation du mélange réactionnel une nuit à TA, on filtre un insoluble et concentre sous vide le filtrat. On reprend le résidu dans 5 ml de MeOH, ajoute 5 ml d'eau et concentre sous vide. On reprend le résidu par une solution d'HCl 1,2N et essore les cristaux formés. On dissout les cristaux dans l'eau, alcalinise jusqu'à pH = 9 par ajout de NaOH 1,3N et essore le précipité formé. On obtient 0,07 g du produit attendu après recristallisation dans l'acétonitrile, F = 175°C (déc).

RMN (DMSO + TFA) :1,0 : d : 6H ; 1,1-1,3 : m : 6H ; 1,5-2,8 : 4m : 17H ; 2,8-4,2 : 3m + 1s : 15H ; 6,6 : d : 2H ; 6,7 : s : 1H ; 7,2-7,4 : m : 3H ; 7,5 : se : 1H.

### EXEMPLE 59

Acide 2-[5-(2,6-diméthoxyphényl)-1-[4-[[4-(diméthylamino)pipérid-1-yl] carbonyl]-2-isopropylphényl]pyrazol-3-ylcarbonylamino]adamantane-2-carboxylique. 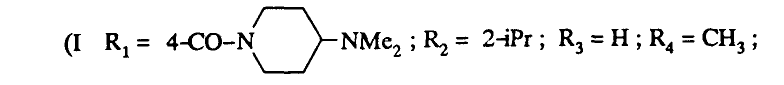 AA(OH) = 2-carboxyadamant-2-yl).

On prépare ce composé selon le mode opératoire décrit à l'EXEMPLE 53 à partir de 0,45 g du composé obtenu à la Préparation 4.37 et 2 ml de SOCl₂ puis 0,17 g du composé B et 0,43 ml de bis(triméthylsilyl)acétamide dans 2 ml d'acétonitrile. On obtient 0,26 g du produit attendu après cristallisation à chaud dans l'acétone puis dans le MeOH, F = 200°C (déc).

RMN (DMSO + TFA) :1,05 : d : 6H ; 1,4-2,3 : 2m : 16H ; 2,5 : se : 2H ; 2,7 : s + mt : 7H ; 2,8-3,8 : 2m + s : 10H ; 4,4-4,8 : m : 1H ; 6,6 : d : 2H ; 6,7 : s : 1H ; 7,1-7,4 : m : 4H.

### EXEMPLE 60

Acide 2-[5-(2,6-diméthoxyphényl)-1-[4-[N-méthyl-N-(2-cyanoéthyl) carbamoyl]-2-isopropylphényl]pyrazol-3-ylcarbonylamino]adamantane-2-carboxylique.

(I : R₁ = 4-CONMe(CH₂)₂CN ; R₂ = 2-iPr ; R₃ = H ; R₄ = CH₃ ; AA(OH) = 2-carboxyadamant-2-yl).

On prépare ce composé selon le mode opératoire décrit à l'EXEMPLE 53 à partir de 3,48 g du composé obtenu à la Préparation 4.38 et 20 ml de SOCl₂ puis 1,43 g du composé B et 3,6 ml de bis(triméthylsilyl)acétamide dans 25 ml d'acétonitrile. Après agitation du mélange réactionnel une nuit à TA, on concentre sous vide, reprend le résidu dans 64 ml de MeOH, ajoute 64 ml d'eau et concentre sous vide. On reprend le résidu dans HCl 1,2N, essore le précipité formé et le lave avec HCI 1,2N. On reprend le précipité dans 5 ml de MeOH, chauffe à reflux, laisse refroidir à TA et essore le précipité. On obtient 3,78 g du produit attendu après séchage sur P₂O₅, F = 249°C.

RMN (DMSO + TFA) :1,05 : d : 6H ; 1,5-2,2 : m : 12H ; 2,42-3,0 : m : 8H ; 3,3-3,75 : m : 8H ; 6,58 : d : 2H ; 6,73 : s : 1H ; 7,1-7,42 : m : 4H.

### EXEMPLE 61

Acide 2-[5-(2,6-diméthoxyphényl)-1-[4-[N-méthyl-N-(3-aminopropyl) carbamoyl]-2-isopropylphényl]pyrazol-3-ylcarbonylamino]adamantane-2-carboxylique.

(I : R₁ = 4-CONMe(CH₂)₃NH₂ ; R₂ = 2-iPr ; R₃ = H ; R₄ = CH₃ ;

AA(OH) = 2-carboxyadamant-2-yl).

On hydrogène pendant 4 heures à TA et sous pression atmosphérique un mélange de 1 g du composé obtenu à l'EXEMPLE 60, 10 ml d'une solution à 20 % d'ammoniaque et 0,1 g de nickel de Raney® dans 20 ml d'EtOH. On filtre le catalyseur sur Célite® , lave à l'EtOH puis au MeOH et concentre partiellement le filtrat. On essore le produit cristallisé formé et concentre sous vide le filtrat. On reprend le produit cristallisé et le résidu de concentration dans HCl 1,2N, essore le précipité et le lave avec HCI 1,2N. On dissout le précipité dans l'eau, neutralise à pH = 7 la phase aqueuse par ajout de NaOH 1,3N, essore le précipité formé, le lave à l'eau et sèche sur P₂O₅. On reprend le précipité dans le propan-2-ol, chauffe à reflux, laisse refroidir à TA et essore le précipité. On obtient 0,54 g du produit attendu après séchage, F = 239-241°C.

RMN (DMSO + TFA) :1,05 : d : 6H ; 1,5-2,2 : m : 14H ; 2,4-3,8 : m : 8H ; 3,5 : mt : 2H ; 3,64 : s : 6H ; 6,6 : d : 2H ; 6,72 : s : 1H ; 7,1-7,45 : m : 4H.

### EXEMPLE 62

Acide 2-[5-(2,6-diméthoxyphényl)-1- [4-[N-méthyl-N-(2-carbamoyléthyl) carbamoyl]-2-isopropylphényl]pyrazol-3-ylcarbonylamino]adamantane-2-carboxylique.

(I : R₁ = 4-CONMe(CH₂)₂CONH₂ ; R₂ = 2-iPr ; R₃ = H ; R₄ = CH₃ ;

AA(OH) = 2-carboxyadamant-2-yl).

On laisse 3 heures 30 minutes sous agitation à TA un mélange de 0,2 g du composé obtenu à l'EXEMPLE 60 0,12 ml d'une solution à 30 % de peroxyde d'hydrogène dans l'eau et 0,18 ml de NaOH 6N dans 10 ml d'EtOH 95. Puis on ajoute 0,06 ml de la solution à 30 % de peroxyde d'hydrogène et 0,06 ml de NaOH 6N et poursuit l'agitation à TA pendant 1 heure 30 minutes. On filtre un insoluble, ajoute de l'eau au filtrat, lave deux fois la phase aqueuse au DCM, acidifie à pH = 3 par ajout d'HCl 1,2N, extrait au DCM, sèche la phase organique sur MgSO₄ et évapore sous vide le solvant. On chromatographie le résidu sur silice H en éluant par le mélange toluène/MeOH (90/10 , v/v). On obtient 0,018 g du produit attendu après trituration dans l'éther, F = 164-166°C.

### EXEMPLE 63

Acide 2-[5-(2,6-diméthoxyphényl)-1-[4-[N-méthyl-N-(2-carboxyéthyl) carbamoyl]-2-isopropylphényl]pyrazol-3-ylcarbonylamino]adamantane-2-carboxylique.

(I : R₁ = 4-CONMe(CH₂)₂CO₂H ; R₂ = 2-iPr ; R₃ = H ; R₄ = CH₃ ;

AA(OH) = 2-carboxyadamant-2-yl).

On refroidit à 0°C un mélange de 0,4 g du composé obtenu à l'EXEMPLE 60 et 0,042 ml de l'alcool 4-méthoxybenzylique dans 3 ml de DCM, fait barbotter pendant 30 minutes un courant d'HCl gaz, dilue le mélange réactionnel par ajout de 17 ml de DCM et laisse 2 heures sous agitation à 0°C. On concentre sous vide, reprend l'imidate intermédiaire obtenu dans 9 ml d'acétone, ajoute 2 ml d'HCl 1,2N et laisse 5 jours sous agitation à TA. On ajoute 6 ml de DMF et 1 ml d'HCl 1,2N, chauffe à reflux pendant 3 jours et laisse 72 heures sous agitation à TA. On concentre sous vide, reprend le résidu au DCM, extrait la phase organique par une solution saturée de NaHCO₃, lave la phase aqueuse au DCM, acidifie à pH = 1 par ajout d'une solution concentrée d'HCl, extrait au DCM, sèche la phase organique sur MgSO₄ et évapore sous vide le solvant. On obtient 0,03 g du produit attendu après trituration dans l'éther puis séchage à 60°C sur P₂O₅, F = 166-168°C.

RMN (DMSO + TFA) : 1 : d : 6H ; 1,5-1,9 : m : 12H ; 1,9-2,8 : m : 5H ; 2,8-3,0 : mt : 3H ; 3,2-3,6 : mt : 2H ; 3,65 : s : 6H ; 6,6 : d : 2H ; 6,7 : s : 1H ; 7,0-7,5 : m : 4H.

### EXEMPLE 64

Acide 2-[5-(2,6-diméthoxyphényl)-1-[4-[N-(propèn-2-yl)carbamoyl]-2-isopropylphényl]pyrazol-3-ylcarbonylamino]adamantane-2-carboxylique.

(I : R₁ = 4-CONHCH₂CH = CH₂ ; R₂ = 2-iPr ; R₃ = H ; R₄ = CH₃ ;

AA(OH) = 2-carboxyadamant-2-yl).

On prépare ce composé selon le mode opératoire décrit à l'EXEMPLE 53 à partir de 1,49 g du composé obtenu à la Préparation 4.42 et 25 ml de SOCl₂ puis 0,65 g du composé B et 1,6 ml de bis(triméthylsilyl)acétamide dans 10 ml d'acétonitrile. Après agitation une nuit à TA, on filtre un insoluble et concentre sous vide le filtrat. On reprend le résidu dans 10 ml de MeOH, ajoute 10 ml d'eau, essore le produit solide et le lave au MeOH. On reprend le produit solide dans l'acétonitrile, chauffe à reflux, laisse refroidir à TA, essore le précipité et sèche sur P₂O₅. On obtient 1,6 g du produit attendu, F = 304°C.

RMN :1,1 : d : 6H ; 1,5-2,2 : m : 12H ; 2,5 : se : 2H ; 2,65 : qt : 1H ; 3,65 : s : 6H ; 3,9 ; t : 2H ; 5,0-5,2 : m : 2H ; 5,8-6,0 : m : 1H ; 6,6 : d : 2H ; 6,7 : s : 1H ; 7.1-7,4 : m : 3H ; 7,6 : d : 1H ; 7,85 : s : 1H ; 8,7 : t : 1H.

### EXEMPLE 65

Iodure de l'acide 2-[5-(2,6-diméthoxyphényl)-1-[4-[N-méthyl-N-(3-triméthyl ammoniopropyl)carbamoyl]-2-isopropylphényl]pyrazol-3-ylcarbonylamino] adamantane-2-carboxylique. 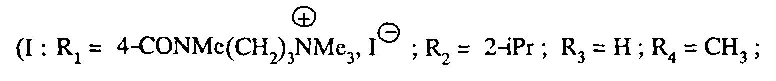 AA(OH) = 2-carboxyadamant-2-yl).

On laisse 24 heures sous agitation à TA un mélange de 0,1 g du composé obtenu à l'EXEMPLE 1' et 0,04 g d'iodure de méthyle dans 6 ml de DCM. On concentre sous vide, triture le résidu dans l'éther et essore le précipité formé. On obtient 0,12 g du produit attendu, F = 222°C.

RMN (DMSO + TFA) : 1,05 : d : 6H ; 1,5-2,3 : m : 14H ; 2,5 : se : 2H ; 2,7 : qt : 1H ; 2,75-3,7 : m : 22H ; 6,6 : d : 2H ; 6,7 : s : 1H ; 7,1-7,5 : m : 4H.

### EXEMPLE 66

Acide 2-[5-(2,6-diméthoxyphényl)-1-[4-[N-méthyl-N-[3-[N'-méthyl-N'-(*tert*-butoxycarbonyl)amino]propyl]carbamoyl]-2-isopropylphényl]pyrazol-3-ylcarbonylamino]adamantane-2-carboxylique.

(I : R₁ = 4-CONMe(CH₂)₃N(Me)COOtBu ; R₂ = 2-iPr ; R₃ = H ; R₄ = CH₃ ; AA(OH) = 2-carboxyadamant-2-yl).

On refroidit à -10°C une solution de 1,17 g du composé obtenu à la Préparation 4.43 et 0,3 ml de triéthylamine dans 3 ml de DMF, ajoute sous atmosphère d'azote 0,21 ml de chloroformiate d'éthyle et laisse 15 minutes sous agitation à -10°C. D'autre part on chauffe à 80°C pendant 45 minutes un mélange de 0,77 g du composé B et 2 ml de bis(triméthylsilyl)acétamide dans 3 ml de DMF. Après refroidissement à TA, on ajoute cette solution sur la solution d'anhydride mixte préparée ci-dessus et laisse 3 jours sous agitation à TA. On filtre un insoluble et concentre sous vide le filtrat. On reprend le résidu avec 32 ml de MeOH, ajoute progressivement 32 ml d'eau et concentre sous vide. On reprend le résidu à l'eau, essore le produit cristallisé formé, le lave à l'eau et le sèche. On reprend les cristaux au DCM, filtre un insoluble et chromatographie le filtrat sur silice en éluant par le mélange DCM/MeOH de (100/0,5 ; v/v) à (100/2,5 ; v/v). On obtient 1 g du produit attendu après trituration dans le pentane, F = 118-120°C.

### EXEMPLE 67

Chlorhydrate de l'acide 2-[5-(2,6-diméthoxyphényl)-1-[4-[N-méthyl-N-(3-méthylaminopropyl)carbamoyl]-2-isopropylphényl]pyrazol-3-ylcarbonylamino] adamantane-2-carboxylique.

(I : R₁ = 4-CONMe(CH₂)₃NHMe ; R₂ = 2-iPr ; R₃ = H ; R₄ = Me ; AA(OH) = 2-carboxyadamant-2-yl).

On laisse 20 minutes sous agitation à TA un mélange de 0,6 g du composé obtenu à l'EXEMPLE 66 et 4,2 ml d'une solution concentrée d'HCl dans 2,7 ml de MeOH et 1,8 ml d'eau. On ajoute de l'EtOH et concentre sous vide le mélange réactionnel. On reprend le résidu à l'EtOH et évapore sous vide le solvant. On reprend le résidu à l'éther, essore le précipité formé et le lave à l'éther. On obtient 0,51 g du produit attendu après séchage sous vide à 60°C, F = 240°C.

RMN (DMSO + TFA) : 1,1 : d : 6H ; 1,5-2,4 : m : 14H ; 2,6 : d : 3H ; 2,7 : mt : 1H ; 2,8-3,6 : m + s : 7H ; 3,65 : s : 6H ; 6,6 : d : 2H ; 6,7 : s : 1H ; 7,1-7,45 : m : 4H.

### EXEMPLE 68

Acide 2-[5-(2,6-diméthoxyphényl)-1-[4-(4-méthylphénylsulfonylamino)-2-isopropylphényl]pyrazol-3-ylcarbonylamino]adamantane-2-carboxylique. 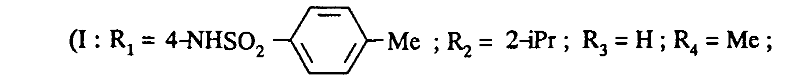 AA(OH) = 2-carboxyadamant-2-yl).

On chauffe 1 heure à 40°C un mélange de 0,92 g du composé obtenu à la Préparation 4.44 et 7 ml de SOCl₂ dans 7 ml de DCM. On concentre sous vide et le chlorure d'acide ainsi obtenu est utilisé tel quel. D'autre part on chauffe 1 heure à reflux un mélange de 0,54 g du composé B et 1,35 ml de bis(triméthylsilyl)acétamide dans 5 ml d'acétonitrile. Après refroidissement à TA, on ajoute cette solution sur le chlorure d'acide préparé ci-dessus, ajoute 0,25 ml de triéthylamine et laisse 2 heures sous agitation à TA. On concentre sous vide, reprend le résidu par une solution à 10 % d'HCl, extrait au DCM, sèche la phase organique sur Na₂SO₄ et évapore sous vide le solvant. On chromatographie le résidu sur silice H en éluant par le mélange DCM/MeOH/H₂O (100/3/0,5 ; v/v/v). On obtient 0,9 g du produit attendu.

RMN (DMSO + TFA) : 0,85 : d : 6H ; 1,52-2,25 : m : 12H ; 2,34 : s : 3H ; 2,45-2,06 : m : 3H ; 3,55 : s : 6H ; 6,55 : d : 2H ; 6,65 : s : 1H ; 6,84 : dd : 1H ; 6,95-7,05 : m : 2H ; 7,23-7,36 : m : 3H ; 7,58 : d : 2H.

### EXEMPLE 69

Acide 2-[5-(2,6-diméthoxyphényl)-1-[4-[3-(diéthylamino)propanoylamino]-2-isopropylphényl]pyrazol-3-ylcarbonylamino]adamantane-2-carboxylique.

(I : R₁ = 4-NHCO(CH₂)₂NEt₂ ; R₂ = 2-iPr ; R₃ = H ; R₄ = Me ;

AA(OH) = 2-carboxyadamant-2-yl).

On prépare ce composé selon le mode opératoire décrit à l'EXEMPLE 68 à partir de 0,27 g du composé obtenu à la Préparation 4.46 dans 5 ml de SOCl₂ et 5 ml de DCM d'une part et d'autre part 0,155 g du composé B et 0,39 ml de bis(triméthylsilyl)acétamide dans 2 ml d'acétonitrile et 0,14 ml de triéthylamine. On obtient 0,13 g du produit attendu, F = 180°C.

RMN (DMSO + TFA) : 1,05 : d : 6H ; 1,25 : t : 6H ; 1,55-2,22 : m : 12H ; 2,5-2,72 : m : 3H ; 2,85 : t : 2H ; 3,2 : qd : 4H ; 3,4 : mt : 2H ; 3,68 : s : 6H ; 6,6 : d : 2H ; 6,72 : s : 1H ; 7,15 : d : 1H ; 7,25-7,5 : m : 2H ; 7,58 : d : 1H.

### EXEMPLE 70

Acide 2-[5-(2,6-diméthoxyphényl)-1-[4-[N-acétyl-N-(3-diéthylaminopropyl) amino]-2-isopropylphényl]pyrazol-3-ylcarbonylamino]adamantane-2-carboxylique.

(I : R₁ = 4-N(COMe)(CH₂)₃NEt₂ ; R₂ = 2-iPr ; R₃ = H ; R₄ = Me ;

AA(OH) = 2-carboxyadamant-2-yl).

On prépare ce composé selon le mode opératoire décrit à l'EXEMPLE 68 à partir de 0,38 g du composé obtenu à la Préparation 4.48 et 3 ml de SOCl₂ dans 3 ml de DCM, puis 0,164 g du composé B et 0,36 ml de bis(triméthylsilyl)acétamide dans 2 ml d'acétonitrile et 0,075 ml de triéthylamine. On obtient 0,24 g du produit attendu, F = 220°C.

RMN (DMSO + TFA) : 1,0 : d : 6H ; 1,5 : t : 6H ; 1,45-2,2 : m : 17H ; 2,5-2,72 : m : 3H ; 2,9-3,15 : m : 6H ; 3,6 : s : 6H ; 3,7 : t : 2H ; 6,59 : d : 2H ; 6,74 : s : 1H ; 7,15-7,42: m : 5H.

### EXEMPLE 71

Acide 2-[5-(2,6-diméthoxyphényl)-1-[4-[(3-diéthylaminopropyl)amino]-2-isopropylphényl]pyrazol-3-ylcarbonylamino]adamantane-2-carboxylique.

(I : R₁ = 4-NH(CH₂)₃NEt₂ ; R₂ = 2-iPr ; R₃ = H ; R₄ = Me ;

AA(OH) = 2-carboxyadamant-2-yl).

On chauffe 16 heures à reflux un mélange de 0,2 g du composé obtenu à l'EXEMPLE 70 et 1 ml d'HCl concentré dans 5 ml d'eau et 5 ml d'EtOH. On ajoute de l'eau, ajuste le pH à 5 par ajout de NaOH 10 % et essore le précipité. On obtient 0,145 g du produit attendu, F = 180°C.

RMN (DMSO + TFA) : 1,05 : d : 6H ; 1,19 : t : 6H ; 1,4-2,2 : m : 14H ; 2,4-2,63 : m : 3H ; 2,98-3,3 : m : 8H ; 3,62 : s : 6H ; 6,5-6,85 : m : 5H ; 7,05 : d : 1H ; 7,3 : t : 1H.

### EXEMPLE 72

Chlorhydrate de l'acide 2-[5-[2-(cyclopropylméthyloxy)-6-méthoxyphényl]-1-[4-[N-méthyl-N-(3-diméthylaminopropyl)carbamoyl]-2-isopropylphényl]pyrazol-3-ylcarbonylamino]adamantane-2-carboxylique. 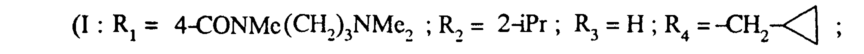 AA(OH) = 2-carboxyadamant-2-yl).

On chauffe à 60°C pendant 8 heures un mélange de 3,87 g du composé obtenu à la Préparation 4.49 et 2,4 ml de SOCl₂ dans 50 ml de DCM. On concentre sous vide, reprend le résidu au toluène et évapore sous vide le solvant. Le chlorure d'acide ainsi obtenu est utilisé tel quel. D'autre part on chauffe à 80°C pendant 3 heures un mélange de 1,27 g du composé B et 2,65 g de bis(triméthylsilyl)acétamide dans 80 ml d'acétonitrile. Puis on ajoute une solution du chlorure d'acide préparé ci-dessus dans 80 ml d'acétonitrile et chauffe à 60°C pendant 3 heures. On filtre un insoluble et concentre sous vide le filtrat. On reprend le résidu dans 16 ml de MeOH, ajoute 16 ml d'eau et concentre sous vide. On reprend le résidu à l'eau, extrait au DCM, sèche la phase organique sur Na₂SO₄ et évapore sous vide le solvant. On chromatographie le résidu sur silice H en éluant par le mélange DCM/MeOH/H₂O (100/5/0,5 ; v/v/v). On obtient 2,1 g du produit attendu.

### EXEMPLE 73

Chlorhydrate de l'acide 2-[5-(2-hydroxy-6-méthoxyphényl)-1-[4-[N-méthyl-N-(3-diméthylaminopropyl)carbamoyl]-2-isopropylphényl]pyrazol-3-ylcarbonylamino] adamantane-2-carboxylique.

(I : R₁ = 4-CONMe(CH₂)₃NMe₂ ; R₂ = 2-iPr ; R₃ = H ; R₄ = H ;

AA(OH) = 2-carboxyadamant-2-yl).

On chauffe à 60°C pendant 5 heures un mélange de 1 g du composé obtenu à l'EXEMPLE 72 et 20 ml de MeOH et 20 ml d'HCl. On concentre sous vide, reprend le résidu au toluène et concentre sous vide. On chromatographie le résidu sur silice H en éluant par le mélange DCM/MeOH (90/10 ; v/v) puis par le mélange DCM/MeOH/NH₄OH (80/20/2 ; v/v/v). On obtient 0,6 g du produit attendu, F > 250°C.

### EXEMPLE 74

Acide 2-[5-(2,6-diméthoxphényl)-1- [4- [3-(diéthylaminopropanoyl)amino]-2-méthylphényl]pyrazol-3-ylcarbonylamino]adamantane-2-carboxylique.

(I : R₁ = 4-NHCO(CH₂)₂NEt₂ ; R₂ = 2-Me ; R₃ = H ; R₄ = Me ;

AA(OH) = 2-carboxyadamant-2-yl).
A) Acide 2-[5-(2,6-diméthoxyphényl)-1-(2-méthyl-4-nitrophényl)pyrazol-3-ylcarbonylamino]adamantane-2-carboxylique.

On prépare ce composé selon le mode opératoire décrit à l'EXEMPLE 68 à partir de 3,2 g du composé obtenu à la Préparation 4.50 et 20 ml de SOCl₂ dans 40 ml de DCM puis 2,4 g du composé B et 6 ml de bis(triméthylsilyl)acétamide dans 15 ml d'acétonitrile et ensuite 1,1 ml de triéthylamine. Après une nuit sous agitation à TA, on concentre sous vide, reprend le résidu dans un mélange acétone/eau, essore le précipité formé et le sèche. On chromatographie le précipité sur silice H en éluant par le mélange DCM/MeOH/H₂O (100/3/0,2 ; v/v/v). On obtient 4,3 g du produit attendu, F=150°C.

RMN : 1,6-2,2 : m : 12H ; 2,25 : s : 3H ; 2,62 : mt : 2H ; 3,63 : s : 6H ; 6,68 : d : 2H ; 6,88 : s : 1H ; 7,03-7,43 : m : 2H ; 7,58 : s : 1H ; 8,05 : dd : 1H ; 8,28 : d : 1H ; 12,4 : se : 1H.
B) Acide 2-[5-(2,6-diméthoxyphényl)-1-(4-amino-2-méthylphényl)pyrazol-3-ylcarbonylamino]adamantane-2-carboxylique.

On hydrogène pendant 4 heures à TA et à pression atmosphérique un mélange de 4,2 g du composé obtenu à l'étape précédente et 0,5 g de nickel de Raney® dans 40 ml de MeOH et 2 ml de DMF. On filtre le catalyseur et concentre sous vide le filtrat. On reprend le résidu à l'éther et essore le précipité formé. On obtient 3,37 g du produit attendu, F = 205°C.

RMN : 1,42-2,1 : m : 15H ; 2,52 : mt : 2H ; 3,57 : s : 6H ; 5,1 : se : 2H ; 6,1 : dd : 1H ; 6,22 : d : 1H ; 6,42-6,68 : m : 4H ; 7,17-7,25 : m : 2H.
C) Acide 2-[5-(2,6-diméthoxyphényl)-1-[4-[3-(diéthylaminopropanoyl)amino]-2-méthylphényl]pyrazol-3-ylcarbonylamino]adamantane-2-carboxylique.

On chauffe à 35°C pendant 45 minutes un mélange de 0,3 ml de chlorhydrate de l'acide 3-diéthylaminopropanoïque et 3 ml de SOCl₂ dans 6 ml de DCM puis concentre sous vide. On ajoute le chlorure d'acide ainsi obtenu sur une solution de 0,87 g du composé obtenu à l'étape précédente et 0,157 ml de triéthylamine dans 5 ml de DCM. On concentre sous vide et chromatographie le résidu sur silice H en éluant par le mélange DCM/MeOH/H₂O (100/5/0,5 ; v/v/v). On obtient 0,5 g du produit attendu, F =190°C.

RMN : 1,28 : t : 6H ; 1,6-2,22 : m : 15H ; 2,5-3,2 : m : 10H ; 3,6 : s : 6H ; 6,63 : d : 2H ; 6,75 : s : 1H ; 7,05 : d : 1H ; 7,28-7,48 : m : 3H ; 7,55 : d : 1H ; 10,18 : s : 1H.

### EXEMPLE 75

Acide 2-[5-(2,6-diméthoxyphényl)-1-[4-[[3-(pipérid-1-yl)propanoyl]amino]-2-méthylphényl]pyrazol-3-ylcarbonylamino]adamantane-2-carboxylique. 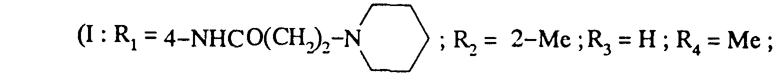 AA(OH) = 2-carboxyadamant-2-yl).

On prépare ce composé selon le mode opératoire décrit à l'EXEMPLE 74 étape C à partir de 0,1 g d'acide 3-(pipérid-1-yl)propanoïque et 1 ml de SOCl₂ dans 2 ml de DCM puis 0,337 g du composé obtenu à l'étape B de l'EXEMPLE 74 et 0,17 ml de triéthylamine dans 5 ml de DCM. On obtient 0,2 g du produit attendu, F = 240°C.

RMN : 1,22-2,1 : m : 21H ; 2,22-2,38 : m : 10H ; 3,58 : s : 6H ; 6,5 : d : 2H ; 6,6 : s : 1H ; 6,9 : d : 1H ; 7,18-7,3 : m : 3H ; 7,38 : d : 1H ; 10,1 : s : 1H.

### EXEMPLE 76

Acide 2-[5-(2,6-diméthoxyphényl)-1-[4-[3-(diéthylamino)propanoylamino]-2-isobutylphényl]pyrazol-3-ylcarbonylamino]adamantane-2-carboxylique.

(I : R₁ = 4-NHCO(CH₂)₂NEt₂ ; R₂ = 2-iBu ; R₃ = H ; R₄ = Me ;

AA(OH) = 2-carboxyadamant-2-yl).

On prépare ce composé selon le mode opératoire décrit à l'EXEMPLE 68 à partir d'une part de 0,15 g du composé obtenu à la Préparation 4.52 et 2 ml de SOCl₂ dans 2 ml de DCM et d'autre part de 0,084 g du composé B et 0,21 ml de bis(triméthylsilyl)acétamide dans 2 ml d'acétonitrile et 0,79 ml de triéthylamine. On obtient 0,014 g du produit attendu, F = 180-200°C.

RMN : 0,75 : d : 6H ; 1,15 ; t : 6H ; 1,4-2,25 : m : 15H ; 2,5 : s : 2H ; 2,8 : t : 2H ; 3,1 : qd : 4H ; 3,3 : t : 2H ; 3,55 : s : 6H ; 6,5 : d : 2H ; 6,6 : s : 1H ; 6,8-7,6 : m : 5H ; 10,3 : s : 1H.

### EXEMPLE 77

Acide 2-[5-(2,6-diméthoxyphényl)-1-[4-[3-(diéthylamino)propanoylamino]-2-cyclopentylphényl]pyrazol-3-ylcarbonylamino]adamantane-2-carboxylique. 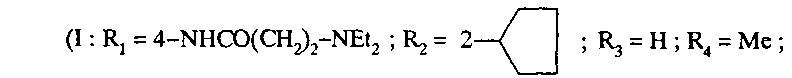 AA(OH) = 2-carboxyadamant-2-yl).

On prépare ce composé selon le mode opératoire décrit à l'EXEMPLE 68 à partir d'une part de 0,32 g du composé obtenu à la Préparation 4.54 et 2 ml de SOCl₂ dans 5 ml de DCM et d'autre part de 0,17 g du composé B, et 0,42 ml de bis(triméthylsilyl)acétamide dans 2 ml d'acétonitrile et 0,154 ml de triéthylamine. On obtient 0,035 g du produit attendu, F = 175-185°C.

RMN (DMSO + TFA) : 1,1-2,55 : m : 26H ; 2,5-2,75 : m : 5H ; 3,15 : mt : 4H ; 3,35 : mt : 2H ; 3,62 : s : 6H ; 6,55 : d : 2H ; 6,65 : s : 1H ; 7,03 : d : 1H ; 7,25 : t : 1H ; 7,35 : dd : 1H ; 7,55 : d : 1H.

### EXEMPLE 78

Chlorhydrate de l'acide 2-[5-(2,6-diméthoxyphényl)-1-[4-[N-(2-diéthylamino éthyl)carbamoyl]-3-isopropylphényl]pyrazol-3-yl-carbonylamino]adamantane-2-carboxylique.

(I : R₁ = 4-CONH(CH₂)₂NEt₂ ; R₂ = 3-iPr ; R₃ = H ; R₄ = Me ;

AA(OH) = 2-carboxyadamant-2-yl).

On laisse une nuit sous agitation à TA un mélange de 0,36 g du composé obtenu à la Préparation 4.55 et 5 ml de SOCl₂ dans 15 ml de chloroforme. On concentre sous vide, reprend le résidu au toluène et concentre sous vide. On utilise tel quel le chlorure d'acide ainsi obtenu. D'autre part on chauffe à reflux pendant 30 minutes un mélange de 0,123 g du composé B et 0,315 ml de bis(triméthylsilyl)acétamide dans 10 ml d'acétonitrile. On ajoute cette solution à une solution du chlorure d'acide préparé ci-dessus dans 15 ml d'acétonitrile et chauffe à reflux pendant 3 heures. On concentre sous vide, on reprend le résidu dans 15 ml de MeOH et 5 ml d'eau et laisse 2 heures sous agitation à TA. On concentre sous vide, extrait le résidu au chloroforme, lave à l'eau, sèche sur Na₂SO₄ et évapore sous vide le solvant. On obtient 0,35 g du produit attendu après cristallisation dans le chloroforme, F = 210°C (déc) (le produit cristallise avec 1 mole de chloroforme).

### EXEMPLE 79

Chlorhydrate de l'acide 2-[5-(2,6-diméthoxyphényl)-1-[4-(2-aminoacétyl amino)-5,6,7,8-tétrahydronapht-1-yl]pyrazol-3-ylcarbonylamino]adamantane-2-carboxylique.

(I : R₁ = 4-NHCOCH₂NH₂ ; R₂, R₃ = -(CH₂)₄- ; R₄ = Me ;

AA(OH) = 2-carboxyadamant-2-yl).
A) Acide 2-[5-(2,6-diméthoxyphényl)-1-[4-nitro-5,6,7,8-tétrahydronapht-1-yl]pyrazol-3-ylcarbonylamino]adamantane-2-carboxylique.

On prépare ce composé selon le mode opératoire décrit à l'EXEMPLE 15 à partir de 4 g du composé obtenu à la Préparation 4.56 et 20 ml de SOCl₂ dans 20 ml de DCM puis 2,74 g du composé B et 6,86 ml de bis(triméthylsilyl)acétamide dans 20 ml d'acétonitrile et 0,8 ml de triéthylamine. Après concentration sous vide, on reprend le résidu dans l'EtOH et essore le précipité formé. On reprend le précipité dans le MeOH, essore le précipité et le lave à l'éther. On obtient 5,3 g du produit attendu.

RMN (DMSO + TFA) : 1,5-2,25 : m : 16H ; 2,42-2,65 : m : 4H ; 2,8 : mt : 2H ; 3,6 : s : 6H ; 6,61 : d : 2H ; 6,75 : s : 1H ; 7,06 : d : 1H ; 7,3 : t : 1H ; 7,4 : s : 1H ; 7,65 : d : 1H.
B) Acide 2-[5-(2,6-diméthoxyphényl)-1-(4-amino-5,6,7,8-tétrahydronapht-1-yl)pyrazol-3-ylcarbonylamino]adamantane-2-carboxylique.

On hydrogène à TA et sous pression atmosphérique un mélange de 3 g du composé obtenu à l'étape précédente et 0,5 g de nickel de Raney® dans 200 ml de DMF.On filtre le catalyseur et concentre sous vide le filtrat. On reprend le résidu à l'eau et essore le précipité formé. On obtient 2,16 g du produit attendu après séchage.

RMN (DMSO + TFA) : 1,42-2,2 : m : 16H ; 2,3-2,8 : m : 6H ; 3,6 : s : 6H ; 6,55 : d : 2H ; 6,7 : s : 1H ; 6,98 : d : 1H ; 7,12 : d : 1H ; 7,25 : t : 1H.
C) Acide 2-[5-(2,6-diméthoxyphényl)-1-[4-[2-(*tert*-butoxycarbonylamino) acétylamino]-5,6,7,8-tétrahydronapht-1-yl]pyrazol-3-ylcarbonylamino] adamantane-2-carboxylique.

On chauffe 1 heure à 60°C un mélange de 0,3 g du composé obtenu à l'étape précédente et 0,258 ml de bis(triméthylsilyl)acétamide dans 2 ml de toluène. Après refroidissement à TA, on ajoute 0,64 ml de Boc-glycine-N-carboxyanhydride, 0,006 ml de N-méthylmorpholine et laisse une nuit sous agitation à TA. On ajoute une solution tampon pH = 4, extrait à l'AcOEt, sèche la phase organique sur Na₂SO₄ et évapore sous vide le solvant. On chromatographie le résidu sur silice H en éluant par le mélange DCM/MeOH de (100/1 ; v/v) à (100/5 ; v/v). On obtient 0,14 g du produit attendu.

RMN (DMSO + TFA) : 1,32 : s : 9H ; 1,45-2,12 : m : 16H ; 2,35-2,6 : m : 6H ; 3,55 : s : 6H ; 3,65 : s : 2H ; 6,5 : d : 2H ; 6,62 : s : 1H ; 6,83 : d : 1H ; 7,13-7,3 : m : 3H.
D) Chlorhydrate de l'acide 2-[5-(2,6-diméthoxyphényl)-1-[4-(2-aminoacétyl amino)-5,6,7,8-tétrahydronapht-1-yl]pyrazol-3-ylcarbonylamino]adamantane-2-carboxylique.

On laisse 30 minutes sous agitation à TA un mélange de 0,14 g du composé obtenu à l'étape précédente et 5 ml d'HCl concentré dans 5 ml de MeOH. On ajoute de l'eau, essore le précipité formé et le sèche. On obtient 0,06 g du produit attendu, F = 220°C.

RMN : 1,59-2,5 : m : 16H ; 2,42-2,75 : m : 6H ; 3,7 : s : 6H ; 3,88 : mt : 2H ; 6,68 : d : 2H ; 6,75 : s : 1H ; 6,95 : d : 1H ; 7,2-7,48 : m : 3H ; 8,2 : mt : 1H ; 9,85 : s : 1H ; 12,4 : se : 1H.

### EXEMPLE 80

Chlorhydrate de l'acide 2-[5-(2,6-diméthoxyphényl)-1-[4-[(3-diéthylamino propanoyl)amino]-5,6,7,8-tétrahydronapht-1-yl]pyrazol-3-ylcarbonylamino] adamantane-2-carboxylique.

(I : R₁ = 4-NHCO(CH₂)₂NEt₂ ; R₂, R₃ = -(CH₂)₄- ; R₄ = Me ;

AA(OH) = 2-carboxyadamant-2-yl).

On chauffe à 40°C pendant 1 heure un mélange de 0,16 g de chlorhydrate de l'acide 3-diéthylaminopropanoïque et 2 ml de SOCl₂ dans 2 ml de DCM. On concentre sous vide, reprend le résidu au DCM et ajoute à TA sur une solution de 0,5 g du composé obtenu à l'étape B de l'EXEMPLE 79 et 0,124 ml de triéthylamine dans 3 ml de DCM. Après agitation une nuit à TA, on ajoute de l'eau, extrait au DCM, sèche les phases organiques sur MgSO₄ et évapore sous vide le solvant. On chromatographie le résidu sur silice H en éluant par le mélange DCM/MeOH (100/3 ; v/v). On obtient 0,11 g du produit attendu.

RMN (DMSO + TFA) : 1,21 : t : 6H ; 1,41-2,2 : m : 16H ; 2,35-2,7 : m : 6H ; 2,84 : t : 2H ; 3,01-3,12 : m : 4H ; 3,35 : t : 2H ; 3,6 : s : 6H ; 6,55 : d : 2H ; 6,7 : s : 1H ; 6,9 : d : 1H ; 7,12-7,3 : m : 2H.

### EXEMPLE 81

Chlorhydrate de l'acide 2-[5-(2,6-diméthoxyphényl)-1-[4-(2-aminoéthyl sulfonylamino)-5,6,7,8-tétrahydronapht-1-yl]pyrazol-3-ylcarbonylamino] adamantane-2-carboxylique.

(I : R₁ = 4-NHSO₂(CH₂)₂NH₂ ; R₂, R₃ = -(CH₂)₄- ; R₄ = Me ;

AA(OH) = 2-carboxyadamant-2-yl).
A) Sel de potassium de l'acide 2-phtalimidoéthanesulfonique.

Ce composé et celui de l'étape B sont préparés selon J. Am. Chem. Soc., 1947, 69, 1393-1401.

On porte à reflux pendant 10 minutes un mélange contenant 30 g de taurine, 25 g d'acétate de potassium et 90 ml d'acide acétique puis on ajoute 37,8 g d'anhydride phtalique. On chauffe à reflux pendant 2 heures et demie puis on filtre, lave par AcOH puis par le propan-2-ol ; on rince à l'éther puis sèche sous vide pour obtenir 59,14 g du produit attendu.
B) Chlorure de l'acide 2-phtalimidoéthanesulfonique.

On chauffe à reflux pendant 1 heure 60 g du composé obtenu à l'étape A dans 300 ml de toluène en présence de 30,7 g du pentachlorure de phosphore. On ajoute à nouveau 30,7 g de pentachlorure de phosphore et on maintient le reflux pendant 90 minutes. On ajoute 280 g de glace au milieu réactionnel, on agite, filtre l'insoluble puis lave à l'eau glacée. Le résidu est séché sur P₂O₅ puis on recristallise dans le dichloroéthane pour obtenir 32 g du produit attendu, F = 160°C.
C) Acide 2-[5-(2,6-diméthoxyphényl)-1-[4-(2-phtalimidoéthanesulfonyl)-5,6,7,8-tétrahydronapht-1-yl]pyrazol-3-ylcarbonylamino]adamantane-2-carboxylique.

On laisse sous agitation à 70°C pendant 1 heure un mélange contenant 0,5 g du composé obtenu à l'EXEMPLE 79, étape B, 0,43 ml de bis(triméthylsilyl)acétamide et 5 ml d'acétonitrile. On laisse revenir à TA puis on ajoute 0,63 g du composé obtenu à l'étape B et 0,30 ml de triéthylamine. Après 2 heures sous agitation à TA, on acidifie avec une solution d'HCl à 10 %. On filtre puis sèche sur P₂O₅ pour obtenir 0,9 g du produit attendu sous forme brute. On recristallise dans EtOH 100 et décolore sur du noir animal dans le DCM. Le produit obtenu est chromatographié sur silice H en éluant par un mélange DCM/MeOH/H₂O (100/2/0,2 ; v/v/v) pour obtenir 0,28 g du produit attendu.

RMN (DMSO + TFA) : 1,45-2,15 : m : 16H ; 2,4-2,6 : m : 4H ; 2,75 : mt : 2H ; 3,48 : s : 6H ; 3,95-4,15 : m : 4H ; 6,46 : d : 1H ; 6,75 : s : 1H ; 6,9 : d : 1H ; 7,1-7,3 : m : 2H ; 7,7-7,85 : m : 4H.
D) Chlorhydrate de l'acide 2-[5-(2,6-diméthoxyphényl)-1-[4-(2-aminoéthylsulfonylamino)-5,6,7,8-tétrahydronapht-1-yl]pyrazol-3-ylcarbonylamino]adamantane-2-carboxylique.

On chauffe à reflux pendant 2 heures un mélange contenant 0,24 g du composé obtenu à l'étape précédente, 2 ml d'EtOH 95 et 23 µl d'hydrate d'hydrazine. On dilue le milieu réactionnel avec du MeOH puis filtre les cristaux que l'on chauffe à reflux dans de l'eau, et filtre à chaud. On sèche sur P₂O₅ les cristaux obtenus. On les redissout dans du MeOH, on ajoute de l'éther chlorhydrique, évapore à sec puis on reprend par de l'éther et du pentane. On filtre pour obtenir 60 mg du produit attendu.

RMN (DMSO + TFA) : 1,48-2,18 : m : 16H ; 2,18-2,62 : m : 4H ; 2,7 : mt : 2H ; 3,19 : mt : 2H ; 3,41 : mt : 2H ; 3,62 : s : 6H ; 6,58 : d : 1H ; 6,7 : s : 1H ; 6,82 : d : 1H ; 7,08 : d : 1H ; 7,28 : t : 1H ; 7,39 : s : 1H.

### EXEMPLE 82

Acide (R) 2-cyclohexane-2-[5-(2,6-diméthoxyphényl)-1-[4-[N-méthyl-N-(3-diméthylaminopropyl)carbamoyl]-2-isopropylphényl]pyrazol-3-ylcarbonylamino] acétique.

(I : R₁ = 4-CON(Me)(CH₂)₃NMe₂; R₂ = 2-iPr ; R₃ = H ; R₄ = CH₃ ;

AA(OH) = (R)(α-carboxy)cyclohexylméthyl.

On mélange 1,2 g de soude dans 20,2 ml d'eau et 1,62 g de trifluoroacétate de (D) cyclohexylglycine. On ajoute goutte à goutte, 1,58 g de chlorure d'acide préparé à l'EXEMPLE 1, étape A, dans 40 ml de THF anhydre et on laisse sous agitation 48 heures à TA. On concentre le milieu, ajoute de la glace et ajuste à pH = 7 par addition d'HCl concentré. On filtre, lave à l'eau puis au pentane et on sèche sous vide. Le produit est broyé puis agité dans un mélange eau-DCM. On filtre puis on extrait la phase aqueuse au DCM et sèche sur Na₂SO₄. Le résidu est concentré, agité dans du pentane et filtré à nouveau. On obtient 380 mg du produit attendu, F 160°C.

### EXEMPLE 83

Chlorhydrate de l'acide (S) 2-cyclohexyl-2-[5-(2,6-diméthoxyphényl)-1-[4-[N-méthyl-N-(3-diméthylaminopropyl)carbamoyl]-2-isopropylphényl]pyrazol-3-ylcarbonylamino] acétique.

(I : R₁ = 4-CON(Me)(CH₂)₃NMe₂ ; R₂ = 2-iPr ; R₃ = H ; R₄ = CH₃ ;

AA(OH) = (S)(α-carboxy)cyclohexylméthyl.

On chauffe à 80°C pendant 3 heures un mélange contenant 0,57 g de (S) cyclohexylglycine et 1,49 g de bis(triméthylsilyl)acétamide dans 39 ml d'acétonitrile et on ajoute goutte à goutte une solution de 1,93 g du chlorure d'acide préparé à l'EXEMPLE 1, étape A dans 39 ml d'acétonitrile. Après 3 heures à 60°C, on laisse revenir à TA puis on filtre et concentre le filtrat. Au résidu, on ajoute 8 ml de MeOH, 3 ml d'eau et laisse 30 minutes sous agitation. On ajoute 5 ml d'eau et concentre. L'huile formée est reprise dans le DCM, on lave la phase organique par une solution de NaCl saturée, sèche sur Na₂SO₄ et concentre. Le résidu est repris dans de l'éther isopropylique et filtré pour obtenir 1,12 g du composé attendu, F = 160°C.

### EXEMPLE 84

Acide 9-[5-(2,6-diméthoxyphényl)-1-[4-[N-méthyl-N-(3-diméthylamino propyl) carbamoyl]-2-isopropylphényl]pyrazol-3-ylcarbonylamino]bicyclo[3.3.1] nonane-9-carboxylique.

(I : R₁ = 4-CON(Me)(CH₂)₃NMe₂ ; R₂ = 2-iPr ; R₃ = H ; R₄ = CH₃ ;

AA(OH) = 9-carboxybicyclo[3.3.1]nonane-9-yl.

On mélange 585 mg d'acide 9-amino[3.3.1]bicyclononane-9-carboxylique et 1,5 ml de bis(triméthylsilyl)acétamide dans 39 ml d'acétonitrile et on chauffe à 80°C pendant 3 heures. On ajoute goutte à goutte 1 équivalent du chlorure d'acide préparé à l'EXEMPLE 1, étape A dans 39 ml d'acétonitrile et on chauffe à 60°C pendant 3 heures. Après 12 heures à TA, on filtre l'insoluble puis on concentre le filtrat, on agite ensuite le résidu avec 8 ml de McOH et 8 ml d'eau. On concentre à nouveau puis on extrait au DCM pour obtenir 900 mg du produit attendu, F = 160°C.

### EXEMPLE 85

Acide 2-[5-(2,6-diméthoxyphényl)-1-[5-[(3-diéthylaminopropanoyl)amino]-2-isopropylphényl]pyrazol-3-ylcarbonylamino]adamantane-2-carboxylique.

(I : R₁ = 5-NHCO(CH₂)₂NEt₂ ; R₂ = 2-iPr ; R₃ = H ; R₄ = CH₃ ;

AA(OH) = 2-carboxyadamant-2-yl).

On prépare le chlorure d'acide à partir de 0,95 g du composé de la Préparation 4.57 dans 5 ml de chlorure de thionyle et 15 ml de DCM par chauffage à reflux pendant 1 heure puis évaporation.

On chauffe à reflux pendant 1 heure un mélange de 0,55 g de composé B, 1,37 ml de bis(triméthylsilyl)acétamide dans 5 ml d'acétonitrile et le chlorure d'acide en solution dans 5 ml de DCM et 0,5 ml de triéthylamine. Après 2 heures d'agitation à TA, on évapore à sec puis on agite le résidu avec 10 ml d'eau, extrait au DCM, sèche la phase organique sur MgSO₄, évapore à sec et cristallise dans l'acétone pour obtenir 0,55 g du produit attendu.

RMN (DMSO + TFA) : 0,8-1,35 : m : 12H ; 1,5-2,4 : m : 12H ; 2,4-2,6 : m : 3H ; 2,8 : t : 2H ; 3,15 : qd : 4H ; 3,3 : t : 2H ; 3,5 : s : 6H ; 6,55 : d : 2H ; 6,65 : s : 1H ; 7,15-7,4 : m : 3H ; 7,75 : d : 1H.

### EXEMPLE 86

Sel interne de l'acide 2-[5-(2,6-diméthoxyphényl)-1-[4-[N-méthyl-N-(3-diméthyl aminopropyl)carbamoyl]-2-isopropylphényl]pyrazol-3-ylcarbonylamino] adamantane-2-carboxylique.

Le composé peut également être préparé à partir du composé de l'EXEMPLE 61 selon le mode opératoire suivant.

On chauffe à 100°C pendant 30 minutes un mélange contenant 0,2 g du composé de l'EXEMPLE 61, 0,33 ml d'acide formique et 0,11 ml de formaldéhyde. Après 2 heures à TA, on ajoute 1 ml d'HCl 2N puis on ajoute du DCM et du méthanol pour dissoudre la gomme formée. On évapore les solvants, reprend par de l'eau puis neutralise avec de la soude 1,3N jusqu'à pH = 7, en refroidissant le milieu dans de la glace. On filtre, rince à l'eau puis sèche sur P₂O₅ pour obtenir 0,13 g du produit attendu.

### EXEMPLE 87

Chlorhydrate de l'acide 2-[5-(2,6-diméthoxyphényl)-1-[4-[N-méthyl-N-(3-diméthylaminopropyl)carbamoyl]-2-isopropylphényl]pyrazol-3-yl-carbonylamino] adamantane-2-carboxylique.

Un autre mode de préparation du composé de l'EXEMPLE 1 est décrit ci-après.
A) Acide 2-(benzyloxycarbonylamino)adamantane-2-carboxylique.

On chauffe à reflux pendant 1 heure et demie 1,015 g d'acide 2-aminoadamantane-2-carboxylique et 6 ml de bis(triméthylsilyl)acétamide dans 10 ml de DCM. On ajoute 0,75 ml de chlorure de benzyloxycarbonyle et on chauffe à 50°C pendant 15 minutes. Le milieu réactionnel est refroidi à -70°C puis on décompose par addition de glace et extrait par AcOEt. On lave à l'eau (2 fois), à la saumure, sèche la phase organique sur MgSO₄ et évapore sous vide. Le produit cristallise dans l'hexane, on obtient 1,164 g.

RMN (DMSO + TFA) ; 1,5 : d : 2H ; 1,8 : m : 6H ; 2 : t : 4H ; 2,4-2,5 : m : 2H ; 5 : s : 2H ; 7,3 : se : 5H.
B) Ester *tert*-butylique de l'acide 2-(benzyloxycarbonyl)aminoadamantane-2-carboxylique.

1,164 g du composé de l'étape précédente est dissous dans 15 ml de DCM, on ajoute 100 mg d'acide paratoluènesulfonique hydraté puis le mélange est refroidi à -78°C et on ajoute une solution d'isobutylène dans 15 ml de DCM. On laisse revenir à TA et agite pendant 24 heures.

On ajoute 50 µl d'acide sulfurique concentré pour dissoudre le solide, après 5 heures le milieu est refroidi puis on ajoute une solution saturée de NaHCO₃, sèche sur MgSO₄ et évapore sous vide. Le résidu est chromatographié sur silice en éluant par un mélange hexane/AcOEt (80/20 ; v/v) et on obtient 612 mg du composé attendu.

RMN (CDCl₃) : 1,4 : s : 9H ; 1,5-1,9 : m : 8H ; 2 : t : 4H ; 2,5 : s : 2H ; 4,9 : s : 1H ; 5,1 : s : 2H ; 7,2-7,4 : m : 5H.
C) Chlorhydrate de l'ester *tert*-butylique de l'acide 2-aminoadamantane-2-carboxylique.

600 mg du produit de l'étape précédente sont dissous dans 40 ml d'EtOH, on ajoute 150 µl d'HCl concentré puis 80 mg de Pd/C puis le milieu est hydrogéné. Après 1 heure, on filtre le catalyseur et on évapore le solvant pour obtenir 503 mg du produit attendu.

RMN (CD₃OD) : 1,6 : s : 9H ; 1,8-2 : m : 8H ; 2-2,2 : m : 4H ; 2,4 : s : 2H.
D) Ester méthylique de l'acide 5-(2,6-diméthoxyphényl)-1-[4-[N-méthyl-N-[3-[N'-méthyl-N'-(benzyloxycarbonyl)amino]propyl]carbamoyl]-2-isopropylphényl]pyrazole-3-carboxylique.

On dissout 0,33 g du composé de la Préparation 3,57 dans 20 ml de MeOH chlorhydrique. Après 72 heures sous agitation, on évapore les solvants. Le chlorhydrate obtenu est dissous dans 5 ml de DCM puis on ajoute 0,5 ml de triéthylamine et 150 µl de chlorure de benzyloxycarbonyle. Après une heure, le milieu réactionnel est concentré sous vide puis on chromatographie sur silice en éluant par un mélange toluène-acétone (80/20 à 70/30 ; v/v). On obtient 252 mg du produit attendu.
E) Acide 5-(2,6-diméthoxyphényl)-1-[4-[N-méthyl-N-[3-[N'-méthyl-N'-(benzyloxycarbonyl)amino]propyl]carbamoyl]-2-isopropylphényl]pyrazole-3-carboxylique.

On dissout le composé obtenu à l'étape précédente (252 mg) dans 2,5 ml de dioxane et 90 µl de solution aqueuse de potasse (1 g/ml). Après 24 heures d'agitation, le milieu est acidifié par 1 ml d'HCl concentré. On extrait par AcOEt puis on sèche la phase organique sur MgSO₄ pour obtenir 236 mg du composé attendu.
F) Ester *tert*-butylique de l'acide 5-(2,6-diméthoxyphényl)-1-[4-[N-méthyl-N-[3-[N'-méthyl-N'-(benzyloxycarbonyl)amino]propyl]carbamoyl]-2-isopropylphényl] pyrazol-3-ylcarbonylamino]adamantane-2-carboxylique.

L'acide obtenu à l'étape précédente est dissous dans 2 ml d'acétonitrile, on ajoute 0,5 ml de tétrachlorure de carbone et 158 mg de triphénylphosphine et on laisse 2 heures sous agitation.

Au chlorure d'acide ainsi formé, on ajoute 110 mg du composé préparé à l'étape C et 100 µl de triéthylamine. Le chlorhydrate de triéthylamine précipite et on laisse sous agitation 15 minutes. On ajoute de l'eau puis on extrait au DCM ; la phase organique est séchée sur MgSO₄ et concentrée sous vide. Le résidu est chromatographié sur silice en éluant par un mélange toluène/acétone (80/20 ; v/v) pour obtenir 323 mg du produit attendu.
G) Chlorhydrate de l'ester *tert*-butylique de l'acide 5-(2,6-diméthoxyphényl)-1-[4-[N-méthyl-N-[3-[N'-méthylamino]propyl]carbamoyl]-2-isopropylphényl]pyrazol-3-ylcarbonylamino]adamantane-2-carboxylique.

On agite pendant 24 heures sous atmosphère d'hydrogène un mélange contenant le composé de l'étape précédente (323 mg), 2 mg de Pd/C et 40 µl d'HCl concentré dans 15 ml d'éthanol. On filtre le catalyseur et on évapore sous vide. Le milieu est repris par de l'éther et agité. Le précipité blanc formé est filtré pour donner 190 mg du produit attendu.

RMN (CD₃OD) : 1,1 : d : 6H ; 1,5 : s : 9H ; 1,7-1,9 : m : 8H ; 2,2-2,3 : m : 6H ; 2,6 : s : 2H ; 2,7-2,9 : q + s : 4H ; 3 : s : 3H ; 3,1 : t : 2H ; 3,7 : s + mt : 8H ; 6,6 : d : 2H ; 6,8 : s : 1H ; 7,2-7,6 : m : 5H.
H) Chlorhydrate de l'acide 2-[5-(2,6-diméthoxyphényl)-1-[4-[N-méthyl-N-(3-diméthylaminopropyl)carbamoyl]-2-isopropylphényl]pyrazol-3-yl-carbonylamino] adamantane-2-carboxylique.

Le composé de l'étape précédente (190 mg) est mis en suspension dans 50 µl d'acétonitrile, on ajoute 0,5 ml d'une solution d'iodure de méthyle dans le toluène (89 µ 1 d'iodure de méthyle dans 100 ml de toluène) et 7,6 mg de carbonate d'argent. On filtre l'insoluble puis on évapore le solvant sous vide. Le milieu est repris par 2 ml d'acide formique et 0,2 ml d'HCl concentré et agité pendant une nuit. Après évaporation sous vide et trituration dans l'éther on obtient 90 mg du produit attendu.

### EXEMPLE 88

Oxazolone du composé de l'EXEMPLE 1' :

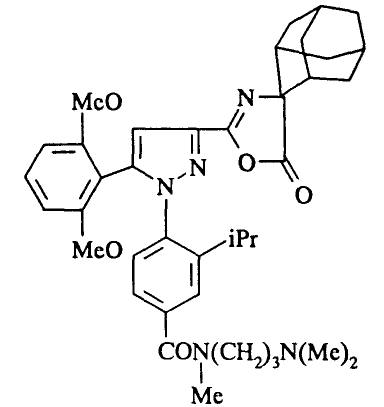

On agite pendant 4 heures 30 minutes une solution de 0,23 g du composé de l'EXEMPLE 1' dans 2 ml de DCM et 0,5 ml d'anhydride acétique. On évapore sous vide, triture le résidu dans le pentane, filtre et sèche pour obtenir 230 mg d'oxazolone attendue, F = 129°C (déc). IR (KBr) : 1800 cm⁻¹. Spectre de masse : M : 667,9.

RMN : 1 : d : 6H ; 1,5-1,9 : m : 8H : 2 : se : 8H ; 2,1-2,5 : m : 6H ; 2,65 : qt : 1H ; 2,9 et 3 : 2s : 3H ; 3,1 : mt : 2H ; 3,4 : mt : 2H ; 3,65 : s : 6H ; 6,6 : d : 2H ; 6,9 : s : 1H ; 7,1-7,4 : m : 4H.

## Revendications

1. Composé de formule : 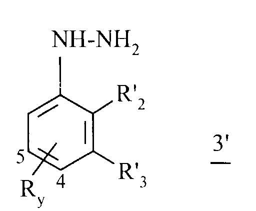 dans laquelle :
- R'₂ et R'₃ représentent chacun indépendamment un hydrogène, un (C₁-C₆)alkyle, un (C₃-C₈)cycloalkyle, un (C₃-C₈) cycloalkylméthyle ;
- ou R'₂ et R'₃ ensemble constituent un groupe triméthylène , tétraméthylène ou pentaméthylène ,
- R_{y} est en position 4 ou en position 5 et représente un groupe choisi parmi : cyano, carboxy, (C₁-C₄) alcoxycarbonyle, benzyloxycarbonyle, sulfo, (C₁-C₄) alkyl sulfonylamino, (C₁-C₄) alkylphénylsulfonylamino, carbamoyle, (C₁-C₄) alkyl carboxamido ;
- à la condition que R'₂ et R'₃ ne représente pas simultanément l'hydrogène et à la condition que R'₂ soit autre que méthyle lorsque R_{y} est le groupe sulfo ;
et ses sels.
